# EUROPEAN PATENT SPECIFICATION

(11) **EP 1 709 165 B1**
(45) Date of publication and mention of the grant of the patent: **23.04.2014**
(21) Application number: 05706772.0
(22) Date of filing: 06.01.2005
(51) Int. Cl.: C12N 9/52

(54) **POLYPEPTIDES OF ALICYCLOBACILLUS**
POLYPEPTIDE VON ALICYCLOBACILLUS
POLYPEPTIDES DE L'ESPECE ALICYCLOBACILLUS

(30) Priority: 06.01.2004 DK 200400010; 04.02.2004 DK 200400165; 23.02.2004 US 784592; 25.02.2004 DK 200400298
(43) Date of publication of application: 11.10.2006
(73) Proprietor: Novozymes A/S, 2880 Bagsvaerd (DK)
(72) Inventor: WILTING, Reinhard, DK-3520 Farum (DK); LASSEN, Søren, Flensted, DK-3520 Farum (DK); ØSTERGAARD, Peter, Rahbek, DK-2830 Virum (DK)
(86) International application number: PCT/DK2005/000004
(87) International publication number: WO 2005/066339

(56) References cited:
- WO-A-02/12463
- ECKERT KELVIN ET AL: "A thermoacidophilic endoglucanase (CelB) from Alicyclobacillus acidocaldarius displays high sequence similarity to arabinofuranosidases belonging to family 51 of glycoside hydrolases." EUROPEAN JOURNAL OF BIOCHEMISTRY / FEBS. SEP 2003, vol. 270, no. 17, September 2003 (2003-09), pages 3593-3602, XP002297030 ISSN: 0014-2956 -& DATABASE EMBL SEQ ID AAC551527 29 March 2003 (2003-03-29), XP002297031 Database accession no. AJ551527
- JARA P ET AL: "Cloning and characterization of the eapB and eapC genes of Cryphonectria parasitica encoding two new acid proteinases, and disruption of eapC." MOLECULAR & GENERAL GENETICS : MGG. 15 JAN 1996, vol. 250, no. 1, 15 January 1996 (1996-01-15), pages 97-105, XP002347647 ISSN: 0026-8925
- POUSSEREAU N ET AL: "Regulation of acp1, encoding a non-aspartyl acid protease expressed during pathogenesis of Sclerotinia sclerotiorum." MICROBIOLOGY (READING, ENGLAND) MAR 2001, vol. 147, no. Pt 3, March 2001 (2001-03), pages 717-726, XP002347648 ISSN: 1350-0872
- FUJINAGA MASAO ET AL: "The molecular structure and catalytic mechanism of a novel carboxyl peptidase from Scytalidium lignicolum." PROCEEDINGS OF THE NATIONAL ACADEMY OF SCIENCES OF THE UNITED STATES OF AMERICA. 9 MAR 2004, vol. 101, no. 10, 9 March 2004 (2004-03-09), pages 3364-3369, XP002347649 ISSN: 0027-8424 cited in the application
- SIMS A H ET AL: "Glutamic protease distribution is limited to filamentous fungi" FEMS MICROBIOLOGY LETTERS, AMSTERDAM, NL, vol. 239, no. 1, 1 October 2004 (2004-10-01), pages 95-101, XP004579958 ISSN: 0378-1097
- ANONYMOUS: "Summary for family G1" INTERNET ARTICLE, [Online] 26 July 2005 (2005-07-26), XP002348371 Retrieved from the Internet: URL:http://merops.sanger.ac.uk/famcards/su mmary/g1.htm> [retrieved on 2005-10-10]
- ANONYMOUS: "Summary for G01.001" INTERNET ARTICLE, [Online] 26 July 2005 (2005-07-26), XP002348374 Retrieved from the Internet: URL:http://merops.sanger.ac.uk/pepcards/su mmary/g01p001.htm> [retrieved on 2005-10-10]

### SEQUENCE LISTING

<110> Novozymes A/S
<120> POLYPEPTIDES OF ALICYCLOBACILLUS SP.
<130> NZ 10406
<160> 60
<170> PatentIn version 3.2
<210> 1
   <211> 2877
   <212> DNA
   <213> Alicyclobacillus sp.
<220>
   <221> misc_feature
   <222> (1)..(2877)
   <223> CDS
<220>
   <221> misc_feature
   <222> (1)..(72)
   <223> sig_peptide
<220>
   <221> misc_feature
   <222> (73)..(2877)
   <223> mat_peptide
<400> 1
   ttgaagactc gatggtcagg tgcgctggct gtgctcatcg ccctcggaac gggtgcctcg      60
   cccgcttggg ccagtgtcca cagcgcggcc acgcacgcaa aggcgcacgt cggcgtgcgc     120
   gctgcggata tggccgcagc gtccatgtcg gccgagattc agattctgca cgacgcgctc     180
   acggcttccg agctgtcgtc cgtccaggcc gcggcacagg ccgccgccaa cctgcctgcc     240
   tccacgtggg tgagctggct gtatccgagc gcctcctcgc cgagcgccgc acagacgcag     300
   acggcgcagg ccctgggcgc gctcctcacc ttggtcacgt atggcgccgt cgcagacgat     360
   ggccaaaaca tcgcacagaa tttgcaaacc cttcagtcga cttcgccgct cttatcgccc     420
   gcggccgtct cgatgttcta tcaaaacttc ttcgtgctcg tcggccaatc gtccaaatcc     480
   gtgctttcgg gccaggcaac cacctccacc gccggccacg ccctcgccca agcggccgcg     540
   ctgacgccac agctcgccgc gtacctgcgc caatccggtc tttcgccgga cgatctcgcc     600
   cgcgcctacg tgagctttgc ctccgccgtg gattcgcagg gcgcggcgca aacggctctc     660
   ctgacgcgca tctgcaccaa catcctgggc tttggcgcgc cgacctccac ggcgaccatc     720
   accgtcaacg ccgcggcgaa ccttggacag gtgccgacca ccgcgtttgg cctgaacgcg     780
   gccgtgtggg acagcggtct caactcccag accgtcatct ccgaggtgca agcgctccac     840
   cccgccctca tccgctggcc cggaggctcc atctcggacg tgtacaattg ggagaccaac     900
   acgcggaacg acggcggcta cgtgaatccc gacgacacgt ttgatcactt catgcagttt     960
   gtgaatgccg tcggctccac gcctatcatc acggtcaact acggcaccgg cacgccacag    1020
   ctcgccgccg actgggtgaa gtacgccgac gtgacccacc acgacaacgt catgtattgg 1080
   gaaattggca acgagattta cggcaacggt tactacaacg gcaacgggtg ggaggcggac 1140
   gatcacgccg tggccggcca gccgcaaaaa ggcaaccctg gtttaagccc gcaggcgtac 1200
   gcgcaaaacg ccctgcagtt catcaaggcg atgcgcgccg aggacccgtc catcaagatt 1260
   ggggccgtgc tcacgatgcc gtacaactgg ccgtggggcg cgaccgtgaa cggcaacgac 1320
   gactggaata ccgtcgtcct gaaggcgctc gggccctaca tcgattttgt ggacgtgcac 1380
   tggtaccccg agacgcccgg gcaggagacc gacgccggcc tgctcgccga cacagatcaa 1440
   atccccgcca tggtggcgga gctcaagcgc gaagtgaaca cctacgccgg atcgaacgcg 1500
   aagaacatcc aaatctttgt gaccgagacc aacagcgtat cgtacaaccc cggcgagcag 1560
   tcgaccaacc tgcctgaagc gctcttcttg gcggacgatc tcaccgggtt catccaggcc 1620
   ggcgcggcca acgtcgactg gtgggatctg ttcaacggcg ccgaggacaa ctacacaagc 1680
   ccgagcctct acggccagaa cctgtttggc gattatggac tcttgtcctc cggccagacc 1740
   acgcaaaacg gttggcagga gccgcccgcc aacacgccgc ttccgcccta caatggcttc 1800
   cagctggtct cggatttcgc gcagcccggc gacacgatgc tcggctccac cacgtcgcag 1860
   agcgccatcg acgtgcacgc cgtgcgcaag ccgaatggcg acatttcgct catgctcgtc 1920
   aatcgcagcc catccgccat ctacagcgcc aacctgaacg tgctcgggtt cgggccgttt 1980
   gtcgtgacac atgcgctcgc gtacggtgaa ggctcgagcc gcgtggcgcc catgccggtt 2040
   cttcccgtcc ccggcgcgcc catcaagctc atgccctaca gcgggatcga tctcaccctg 2100
   cacccgctca ttccggcgcc acacgccgcc gcgcaggtga ccgatacgct cacgctgtct 2160
   tcgcccacgg tgacggccgg cggtgcggag acgctctccg cctcgttcca ggcggatcga 2220
   ccggttcatc acgccacggt ggagctcgag ctgtatgact cgacgaacga tctcgtcgcc 2280
   acccacaccg tctcggatgt cgatcttcag ccgggatcgg ccacaagcga gacgtggagt 2340
   ttcaccgcac cggccgcgaa cggcaattac cgcgttgagg cgtttgtgtt tgacccggtg 2400
   acgggcgcga cgtacgacgc ggacacgcag ggcgcggttc tgaccgtcaa ccagccgcct 2460
   caggcgacct acggcgacat cgtgacgaaa gacacggtca tcacggtgaa cgggacgacg 2520
   tacgacgttc cggcacctga tgcgggcggg cactatccgt cggggacgaa tatttcggtg 2580
   gcacccgggg acacggtgac cgtgcagacg acgtttgtca acgtctcatc gacggacgcg 2640
   ctgcagaacg ggctcatcga catggaagtg gacggatcga acggggccat cctgcagaaa 2700
   tactggccga gcacgactct tttgcctggc caatcggaga cggtgacggc gacgtggcaa 2760
   gtgcctgcga atgtggcggc cggaacgtac ccgctcaact tccaggcctt caacacgagc 2820
   agctggacgg gaaactgtta cttcacaaac ggtggcgtgg tcaacttcgt gatcagc 2877
<210> 2
   <211> 816
   <212> DNA
   <213> Alicyclobacillus sp.
<220>
   <221> misc_feature
   <222> (1)..(816)
<220>
   <221> misc_feature
   <222> (1)..(96)
   <223> sig_peptide
<220>
   <221> misc_feature
   <222> (97)..(816)
   <223> mat_peptide
<400> 2
   atgaacggca cctcagtctg gaaagcgtca ggcatcgcag ccgcctcgtg cctgacagcc      60
   gcggcacttc tcgcctggcc ccacgccaca tccacgttgg acgcgtcgcc cgccatcttc     120
   cacgcgccgc ggcacgcgct ctcgcccaac accagcccga aaccgaacag cgtccaggca     180
   cagaactttg gttggtcggc gtcgaactgg tcgggatatg ccgtgaccgg cagcacgtac     240
   aacgacatca caggcagttg gattgtgcct gcggtgagcc catccaagag aagcacgtac     300
   tcttcgagct ggatcggcat cgacgggttc aacaacagcg atctcattca aaccggcacg     360
   gagcaggact atgtcaacgg tcacgcgcag tacgacgcct ggtgggaaat cctccccgcc     420
   cccgagacgg tcatctcgaa catgaccatc gccccgggcg accggatgag cgcgcacatc     480
   cacaacaacg gcaacggaac ctggacgatt acgttgacgg acgtgacccg caacgagacg     540
   ttctccacca cgcagtcgta ctcgggccct ggctcgtcgg ccgagtggat ccaggaggcg     600
   ccggagatcg gcggccggat cgccacgctc gccaactacg gcgagaccac gttcgatccc     660
   ggcaccgtaa acggcggcaa cccaggtttt accctgtccg acgcgggcta catggtgcag     720
   aacaacgcgg tcgtgtctgt gccgtccgca cccgactcgg ataccgacgg cttcaacgtg     780
   gcctacggct ccaaccagcc gagcccaccg gcctcc                                816
<210> 3
   <211> 945
   <212> DNA
   <213> Alicyclobacillus sp.
<220>
   <221> misc_feature
   <222> (1)..(945)
   <223> CDS
<220>
   <221> misc_feature
   <222> (1)..(75)
   <223> sig_peptide
<220>
   <221> misc_feature
   <222> (76)..(945)
   <223> mat_peptide
<400> 3
   atgagaagac gcatgtcagg ctttgcgacg ggccttggca tcgcggcggg gctcgccctc      60
   agttccgccc tcgccgcgcc gttcttccac gccgggaacg cgtccgcggc gtcgacgatg     120
   tcgatggcgc cgacgagcac catgggcgcc ctgcccgcgc ccgaaggcgt gccggacgca     180
   ggcccgctgt cgatcacgcc cgaggtcatt cgccaacaac aggctgacgc tgtccgggtc     240
   atggacgaag aaggcctgaa gccacagatc ctctccggcg acatcaagcg attcaccctc     300
   accgcgagcc aggtgaactg gtatttgtac cccggcaaag cggtcgtcgc gtgcggctac     360
   aacggccaag tgcctggccc ggtcctccgc gtgcgcgtgg gcgatcgcgt ccaaatcctc     420
   ctgagaaacg agctgaacga gcccaccacg ctgcacatcc agggcctcga tctgccggcg     480
   tcgcagttgg gaatcggaga cgtcaccgaa tcccccatcc ctccgggcgg cgaacgcctg     540
   tacagcttca ccgtgacgcc acagatggtg ggcacccacc tgtacgagag cggcacggat     600
   atggccagcg agatcgaccc aaggactgca cggggtgctg ctcgtcgatc cggcccgggg     660
   atccctttat ccccaggcga aggtggacgc gctcttcgag atcgacgcgt ggatggtgga     720
   cggatcgacc accgaaaacg cgtttggcct ggacggcaag ccgtatcccg acgcgcccga     780
   actgacggtg ccgtacggca gccgcgtggt gctgcgcatc gtcaacgcga gcgggatgtg     840
   ctaccacgcc atgcacctgc acgagacgac gttttggctg ctggcggaag acgggcaccc     900
   cctcgccaag ccgcggccga tgaacgtgct cgccatcgcg ccagg                     945
<210> 4
   <211> 1878
   <212> DNA
   <213> Alicyclobacillus sp.
<220>
   <221> misc_feature
   <222> (1)..(1878)
   <223> CDS
<220>
   <221> misc_feature
   <222> (1)..(66)
   <223> sig_peptide
<220>
   <221> misc_feature
   <222> (67)..(567)
   <223> propeptid
<220>
   <221> misc_feature
   <222> (568)..(1878)
   <223> mat_peptide
<400> 4
   atgggcttgt ggaaacggct ggcgctcggc gtgcctgcgg cacttagcat gctcgcggtt 60
   ggggtgcctg tgatgagcgc ggacaccgtg gaggctgcgc cgcttgcgaa tccttcaacg 120
   gaaaatgcgc aggatatggg gccggcgagt gggagccaga cggtgacggc atccatcatt 180
   ttgcgtgtgc agaatccgac ggcgctgcag aactacattc aagagacgga gacaccgggc 240
   agtccgctgt accataagtt cttgacgacg gcgcagttcg ctcagcagta cgcgccttcg 300
   gcggcgaccc ttcagcagat tgagcaggag cttcagggct atgggctcca ggtcgtgaat 360
   gtcgacgcgg atcacctgga catgcaggtt cagggcacag ttcagcagtt tgacaacgcg 420
   ttcaacaccg tgatcgacct gtttaaggca aacgggcaca tcttccgcgc gccgaagaag 480
   ccgccgcaga tcccggtggc gcttctcacc aacgtgctcg ccgtggtggg actcgatacg 540
   gcacaggcgg cgcagtcgct cacggtgaag acgccgaacg tcgcgggtgt gccttcgccc 600
   aaggtggtgc ttccgcaggg aggcagcacg gcgacgggca cgccagggag ctacacggtt 660
   ggggatacgg cgaatcgcta cgacatcaac ccactctatc agaagggtat cacgggcaag 720
   ggcgagacca tcggcattgt gacgctgtcg agctttaatc cgcaggatgc ctacacctac 780
   tggcagggca ttgggctgaa ggtggctcca aaccgcatcc agatggtgaa tgtggacggc 840
   ggtggccaga tggatgatgg atcggtcgag acgacgctgg acgtggaaca gtcgggcggt 900
   ttggcgccgg acgccaacgt cgtggtgtac gacgcgccga atacggatca gggcttcatc 960
   gatgcgttct accaggcggt ctcggacaac caggcggatt ccctctcggt gagctgggga 1020
   cagcctgaaa tcgattacct gccgcagatg aaccaaggcc agtcgtatgt ggatgagctc 1080
   ctcgccttca cccaggcgtt catggaggcg gcggctcagg gcatttccat gtacgcggcc 1140
   gcgggggatt caggcgccta cgacacggcg cgcgacttcc cgccctccga tggcttcacc 1200
   acgccgctca gcgtggactt tcccgcctcc gacccgtaca tcacggctgc gggaggcacg 1260
   acggtaccgt tcaccgcaaa gttctcgctc ggcacggtca acatcacgca ggagcagccc 1320
   tggtcgtggc aataccttca aaacctcggc taccaagggc tcttctccgt gggcacaggc 1380
   ggtggcgtga gcgtcatctt cccgcgcccg tggtatcagc tcggcgtggg cggcatgcaa 1440
   aatagcgcgg ccaatcaggc cttcaccgac tcgcagggcg ttttgtacgg atcgcccttc 1500
   acgtacaacc tgccgagcaa ttacgcgggc cggaatctgc cggacatctc catggatgct 1560
   gatccggaga cgggctatct ggtctactgg agcgcgggcg gtggctggat tgcgggctac 1620
   ggcgggacga gcttcgtggc gccgcagttg aacggtatca cggcgctcat tgatcaggag 1680
   gtccatgggc gagtgggctt cctcaatccg ctgctgtaca ccctgttgac gcaaggggtc 1740
   caaggtgggg cgcagccgtt ccacgacatc acgacgggga acaactggta ttggaatgcg 1800
   gtgcctggtt acgatccggc ctcgggcgtg ggcacgccgg acgtcgcgaa cttggcgcag 1860
   gacatcgcat cgctgaga 1878
<210> 5
   <211> 1599
   <212> DNA
   <213> Alicyclobacillus sp.
<220>
   <221> misc_feature
   <222> (1)..(1599)
   <223> CDS
<220>
   <221> misc_feature
   <222> (1)..(72)
   <223> sig_peptide
<220>
   <221> misc_feature
   <222> (73)..(1599)
   <223> mat_peptide
<400> 5
   atgcgagcgc tcgcacattt ggccattggc gccatcgcgt ccggcgtttt cgctgcacct      60
   gtcgcttttg cctcgccggt tcaggaacgc gtggtggtgg cctcgcccga tccgcggacg     120
   cgtagcgttc acgcggatgg cgaaatttca ccgtcgcagc cgatgcactt ggtcattacg     180
   cttcgcctgc gccacgaggc gcagctcgag cagctgattc gagacctgta cacgccggga     240
   tcgcccgatg caggtcactt cttgacgccc gcggcgttta acgcggcgta tgcaccgacg     300
   gctgaggacg tgcaggccgt ggtccagggg ctgcgcgcat acggcctccg cgttgagccg     360
   acggtaaatc ccatggtgct gaccgtgagt ggacgggccc gcgacgtcga gcgagcgttt     420
   ggcgtgcatg agctccaatt tgggcgcgga gctggcgcat ggtacgcccc ggatggtgcg     480
   gccacgctgc ctgcaccgct cgccgcgcgc gtgtcggccg tggtaggcct gacgagcgac     540
   gcgatggagc gccacctcgt cctggcgcac gtcgcgccgg caggaggtgg ctacacgccc     600
   gcgcaaattc agcgcgccta cgactatacg ccgctctaca gccaatacat ggggcgcgga     660
   caggtcattg cggtggtgac ttccggctcg gtgctccgct ccgacctgct cgcgttcgat     720
   cgcgccttcg ggcttccgaa tccggtggtg cgccagcggg tgatcgacgg atcgtccacg     780
   tctcccgacg acgagaccac cctcgactgc gagtgggcgc atgccatcgc gccgacggca     840
   tcgctcgccg tgtacgaggc cgctcaaccg gacgcgcagt cgttcatcga tgcgtttgcc     900
   caggtggcgg cggacgatgg cgcgcatgtg gtcacgacga gttggggagc gcccgagtcg     960
   gagaccgacg cggcgaccat gcaggcggag caccagatct tcatgcagat ggccgcccag    1020
   gggcagagcg tgttcgccgc ggcgggcgac agcggatcgt cggacggaac aagcgggacg    1080
   gacgtcgact atccgtcgtc ggatccgtat gtcaccgcgt gtggcgggac gaggctcgtt    1140
   cttggtgcgg gtgcaaagcg gctgcaggag acggcgtggg ccgacacggg cggcggcgcg    1200
   agctcggtgt acggagagcc gtggtggcaa tatggcccgg gcgtgccgca gacgggctat    1260
   cggcagacgt gcgacgtcgc cctgaacgcc gatccggcca cgggctacga tttcatctat    1320
   gagggtcagt gggaggtggc cggggggacg agctttgtcg cgccgatgat ggccgcgacg    1380
   tttgcgctca tcgaccaagc gcgtgccctc gaaggtaagc cacccgttgg gctcgcagac    1440
   gtcggcatct atgcgatggc gcgcaacgcg tcctacgcgc cgtacgcatt ccacgacatc    1500
   acggccggat cgaacggcgc gtacagcgcg ggcccgggat gggatcatcc aaccggcttt    1560
   ggttccatcg acgcgtacta ctttttgcac gggctcgac                           1599
<210> 6
   <211> 1233
   <212> DNA
   <213> Alicyclobacillus sp.
<220>
   <221> misc_feature
   <222> (1)..(1233)
   <223> CDS
<220>
   <221> misc_feature
   <222> (1)..(123)
   <223> sig_peptide
<220>
   <221> misc_feature
   <222> (124)..(1233)
   <223> mat_peptide
<400> 6
   atgcggcgtc gacgttggga ttacgaggac tggccgagtg agaacaggcg tgtcggcgtg     60
   tggctcgcga gcgggaccgc gctgcttgcc atctgctaca tcctcggcat ctggacgggt    120
   gcggcgctca cgcgcggtca ttcccagacg accgtggaat acgttcctcc ccagacgggc    180
   aacaccgcga gcacgtccgg atcgctcacg ccgatcccgg gcgtcgagga cacgaccata    240
   gtgacgcaga tttataaccg agtgaaaaat agcatcttta ccattacggc cgtctccgga    300
   ggcaagccga cgtcgagcga cgcagaagaa gatatcggca cggggttcct gatcgatcac    360
   aacggcgatc tcttgaccaa cgcgcatgtc gtcggatcgg ccacaacggt ccaggtgtcc    420
   ggggacaacc gccaattcgt cggccgcgtg attgacgccg accagctgga cgatctcgcc    480
   atcgttcgca tcccggcgcc caaatcgctg gaaccgctgc cgttgggatc ggtgaagtcg    540
   cttcagccgg gcagcctggt catcgccatc ggcaacccgt ttgagctgac ctcgagcgtc    600
   agctcgggca tcgtgagcgg actcaaccgg tcgatgtccg agtcgaacgg gcacgtgatg    660
   aacggcatga tccagacgga cgcgccgctc aaccctggaa attcgggagg cccgctgctc    720
   aacgcggcag gacaggtcgt cggcatcaac acgctgatcg aaagccctat cgaggggtcc    780
   atcggcattg gctttgccat tcctatcgac cggtttatcc agctcgagcc agaattgctc    840
   gccggcaaac ccgtcgcgca cgcctggctc ggcatcgagg gaatggacat cgacaacctg    900
   atgcgtcaag cgctgcactt gcctgtggcc tcgggcgtct atgtgaccga agtgaccccg    960
   ggcggccccg ccgcgaaagc ggggctgcgc ggagattcga acgcggccaa gttgaacagt   1020
   ctaagccagt cggccaatcc gtacgcgctg ctcaagggga acggggacat catcgtcggg   1080
   attgacggca agcaggtctc cagcatcgaa cagttgacgc aggatatcaa ccaagatcaa   1140
   ccgggtcaga cggtggtgct caccgtgttg cgcgcaggca aaaccctgca cgtgcgcgtc   1200
   acgctcggga cctggccatc cagccaaaat ccg                                1233
<210> 7
   <211> 633
   <212> DNA
   <213> Alicyclobacillus sp.
<220>
   <221> misc_feature
   <222> (1)..(633)
   <223> CDS
<220>
   <221> misc_feature
   <222> (1)..(90)
   <223> sig_peptide
<220>
   <221> misc_feature
   <222> (91)..(633)
   <223> mat_peptide
<400> 7
   atgcgcaggt cttggagcgt gctcatggcc gtttgcatgt cttggttggc ggtggggtgt      60
   ggcacgcctg caaactcgtt gtcacaagcg accgctgcgt ctggaaggca cgcgccgcac     120
   cccctcgtgt ttcagaacct cacaggtgcc atgaacgagg ggcaggatcc ccggtgggac     180
   ccgaaagcgg ctcccacggg tgtctacgac gacgtgaccg tggtcacagc gagtggccga     240
   caggaggtgc tctccgttcg ggatgcgccg ctcctgttcg cagcgtactg gtgccctcac     300
   tgccagcgca cactgcagct tctcacgtcg attgaatcac gcctgaagca aaagcccatt     360
   cttgtgaacg tcggctatcc tccgggcacg acactgcaga ccgcggcgcg catcgcgcgc     420
   gaggagtctc aagttcttca cttggcgccg ttccaagagg tctttatctt gaatcctgat     480
   gcaggggatc gatacgcccc gctagggtac ccaacactcg ctttttatcg cgccgggcga     540
   gattggacgc tgtacggtga acatcgagcg tctatttggg aaaaggccct gtccgaatcg     600
   acatcaaaag cgtacaatgg cagcgaggaa tca                                  633
<210> 8
   <211> 798
   <212> DNA
   <213> Alicyclobacillus sp.
<220>
   <221> misc_feature
   <222> (1)..(798)
   <223> CDS
<220>
   <221> misc_feature
   <222> (1)..(87)
   <223> sig_peptide
<220>
   <221> misc_feature
   <222> (88)..(798)
   <223> mat_peptide
<400> 8
   atggatgaga tgaacattcg atcttggtgt gtcgctgctt gtaccgtagc cttgacaagc      60
   gccgtgggcg cgacgaccgc gttcgcgcag acggtgaccg tacaacccgg acaatcgctc     120
   tggaccatcg cacgcgcaca cgggatgccc gttcagttgg tggcgtccgc caatccgcag     180
   tacaatccgc tgaatctccc tgttggtgcg accgtcacac ttcccagtct caaggacgtg     240
   gctgtgcagc cgggcgactc cctgtttctg atcggcaggc aatatggcgt gtcgctcgcc     300
   gagatgttgg ccgcaaaccc gaacgtggat ccattgaatc tgcaagtggg ttcaagtgtg     360
   cgtgttcccc ttgcatcatc ttcgaccaag agctccacag tttctgccca tgttgccgca     420
   tccacgcccg aaaactccaa caacctgtac tggttggagc gcgtcattca cgcggaggcc     480
   ggcggagaat cgctgcaggc acaaatcgcc gtggccgacg tcattctcca tcgcatggcc     540
   gcgggtggat acgggagcac ggtgcaacaa gtggtcttcc aagtgagcga cgggcactac     600
   caattcgaga gtgtcgcaaa cggttcgatt tacggtcagc cagacgcaca aaacgtgcag     660
   gctgctctcg acgcgttgaa cggagacgat gtcgtcccag gcgcgttggt cttctacaac     720
   cccgcgcaga cgccttccgg aagttgggtt tggcaacaac ctgtggtcgc tcatatcggt     780
   catctcgtgt ttgcgaag                                                   798
<210> 9
   <211> 2304
   <212> DNA
   <213> Alicyclobacillus sp.
<220>
   <221> misc_feature
   <222> (1)..(2304)
   <223> CDS
<220>
   <221> misc_feature
   <222> (1)..(78)
   <223> sig_peptide
<220>
   <221> misc_feature
   <222> (79)..(2304)
   <223> mat_peptide
<400> 9
   gtgaagacgc atcgcctgct cgcggtcgcg gcactgcctg caacagtgct gttgacaacg 60
   ccggcgcccg cgctggctga gacctcgagc tcgcagagcg cttcggcgcc gtcgctgaac 120
   gtgccggtcg ctgccctgac cctcgcgggt gttcaatcgt atcccatgct gagctacgga 180
   tccacgggcg tgtacgtgga aattttgcag aacgccctga atgccctggg ctatgacgtg 240
   ggacaagcca gcgggctgtt cgacgccacc acgcaggccg aagtgaaggc ttttcagcag 300
   gcgatgggcc tgcagacgga cggcattgtg ggtcccctga cctggggggc tttggcgaag 360
   gcggtggccg attatcgcca ggtgatgacc gtactctcca gtcgcagctc gctggttcag 420
   caagtcgaat ggaagcgcat cgtatggaac ggcaggttga tttcgaagcc catcggcttc 480
   acgtaccagg ggacagcgta catgcccatt tggtacgtca tgcaggcgct tagcaaggcg 540
   ggcattgcga gcacgtggca gggaggggtt tggacgctca cgccgcccgg aggtcagacc 600
   gtgaattacg gaaagatctc gtacgggccg ggcagtgcgg ccatcgccat cggccagacc 660
   gtggtcgcca atgtgcccgc ggtggtgtac cctgatccgg catccggaaa gctcacgacc 720
   ttcatgcctg tttggtacgt catgaacgcg ttgcagcggc tgggcatcgg ttcgacgtgg     780
   cagggaaccg agtgggacat gaagccagct cccgtcgtga tcgagacggg cgatccgtcg     840
   aacaacacca ccgggtcaga tcccgcgaac agcacgggca acggcaccgg gaactcgacg     900
   ggcaacgcca cgggcgccgt gccaggcggc aataccgtga cgaacgtcac cacgggctcg     960
   tccaacgtca ccggcaactc gacgggcaac agtttgggga actcgacggg caacagcttg    1020
   ggcaacagca cgtcgaacgc gacgggcaat gccaccggca acaccaccgg gaatgcgacc    1080
   ggcaattcca cgggcacgag cagcgggtcg ttcacgaatg tcgacctgcg ctatccggcg    1140
   ccgtccaaca tcaatgcgca gagcatcaac cagtttctgc tgcagaacag ctcgccgctc    1200
   aatgggctgg gcaattcgtt catggacgcc cagaacctgt acagcgtcga cgccaactac    1260
   cttgtctcgc acgccatcct cgagagtgcg tgggggcaaa gccaaattgc ccttcagaag    1320
   aacaatctgt ttggctacgg cgcttacgat tcgaaccccg gacaggatgc gggcgtattc    1380
   ccgagcgacg actacgccat ccgattcgag gcgtggaccg tgcgcatgaa ctacctcacg    1440
   ccgggcgcga gcttgtacgt gacgccgacg ctcagcggaa tgaacgtgaa ctacgccaca    1500
   gccaagacct gggcaagcgg cattgcggcc atcatgacgc agtttgcgag ctccgtcgga    1560
   tcgaacgtga atgcgtacgt gcagtacacg ccgtccaaca atccgcccgc tccgagatcg    1620
   acagcggaac cggtgtacta catgaacggc gcgcaagggg taacgcagca ggatccgtat    1680
   tacccgaatg gcggcgttcc gtactacccg accatcgcgc agggtgagaa tcagcagttc    1740
   tttggccagc taagtgtcgg cagcttcggt caacccgtgg tggaggttca gcagttcctg    1800
   aaccggacca tcaacgcggg gctgaccgtg gacgggcagt ttggcccgct gacgcaggcc    1860
   gcggtcgaga agttccagtc gcaggtcatg cacatgtcga acccgaacgg catttggacg    1920
   ttcagcatgt gggtccagta catccagcct tctcagtcga acgccaatct catcccggct    1980
   gggaccaccg tgaaaattga ccaggtcgcc gagggcatgg cgggcccgta cgtcgtgcct    2040
   tggtaccacg tggtgggcta tggctgggtc gactcgcagt atatcaagtt gaccaacgtg    2100
   tatcgcgtca ttgtgcagaa cccggccgga acggccacca ccattcccgt ctaccaggtg    2160
   ggcaacctgt cttcggtatt gctcaatctg cacagcggag actgggtggt tgccaactca    2220
   gcgcagccct cgggcggcgt gtacaccatt cagattgcgg ctcaggatcc accgtgtcga    2280
   acggctacgc cgccgggacg ctct                                           2304
<210> 10
   <211> 1791
   <212> DNA
   <213> Alicyclobacillus sp.
<220>
   <221> misc_feature
   <222> (1)..(1791)
   <223> CDS
<220>
   <221> misc_feature
   <222> (1)..(147)
   <223> sig_peptide
<220>
   <221> misc_feature
   <222> (148)..(1791)
   <223> mat_peptide
<400> 10
   atgatggccc acgatagatt ggacaggcga gtgaatgaga ggaggcaagc catgcgacgc     60
   gcggcaaaat gggcaatcgc ccttggcacg acggcagtgg tggctggtgt cagcagcgtg    120
   ttcgcacttc gcagtgtgcg agaagcaaac ctgaatccca acgcccctct cgcgaacgtg    180
   cccgggcctc agggcgccta tacgcccatc agcgcgcttc agcccgtcgt tccgaaaaac    240
   gcgcggatcg accactacac gctgacggcg gaatcccgca cactgaccgt cggcggccat    300
   gccctgcaag ccatgacgtt caacggcacc gcgccagggc cgttgcttgt ggcccatcaa    360
   ggcgacgtcg tgaaggtcac ggtgcacaac cgcctctccg tccctctgac cattcactgg    420
   cacggcatcg cggtgcccgg cgcggaagac ggcgtccctg gtgtcacgca aaacccaatt    480
   ccgcctggcg ggagctacac gtacgagttt caggttaacc agcccggaac gtactggtac    540
   cactcgcacg aggcgagctt tgaagaggtg ggcctcgggt tgtacggcgc cttcgtcgtt    600
   ctgcccaaac gggcggtcca tccggccgat cgcgactaca cgctcgtcct gcacgagtgg    660
   ccgaccgcat ccaccgcgca gacgatgatg gcgaacctca aggctgggaa cttgggattc    720
   tcagcgaaag gcgaatccgc aggcatgggc ggcatgggca tgcaacaaaa cggggacatg    780
   aacggcatgg gcatgatggg cgcggcggac ggcacgggtc agggaggaaa tagcgcgagc    840
   gacatcgcgc acgtgttgcc tggccccccg cttcaactga acggtttttc gccgaccgca    900
   aacgattggg ctgcgcttga cgaaatggcg ggcatgtatg acgccttcac ggtgaatcag    960
   aacgcgagcg gtacaacgct cttgccagcc aagccgggac agctcgttcg gcttcgcatc   1020
   gtgaacagcg gcaacatgac acacctgttc acgctggtcg gcgcaccgtt tcgcgtcgtg   1080
   gcgctcgacg gccacgacat tgccaacccc ggttggatcc gcggcgtctt gcttcccgtc   1140
   ggcgctgcag agcgatacga catcgaattt cgcgtgccaa agtccggggc cgcattcctt   1200
   gtgtgcgccg atcccgacac gactgcacag cgcgagcttc gcgccgccat cggtctgccc   1260
   gacgcctggt cacaattcaa ggagacggat gcagcgagcc ttgaacgagc gccgtggttc   1320
   gactttacac actatggcag cggcaggctg cccggcgaag ccgtgttccg cctgcatcag   1380
   gcgtatcagg tacgctacaa catgaagctc accgtcggca tgtcgatgaa cggcatggtg   1440
   tacgccatca acggcaaggt ctttccgaac atcccgccca tcgtcgtgcg aaagggcgac   1500
   gccgtcctgg tccacatcgt gaacgacagc ccctacattc acccgatgca tctgcacgga   1560
   cacgactttc aagtgctgac gcgcgatggg aaacctgtct ccggaagccc catcttcctg   1620
   gacaccttgg acgtgttccc cggcgagagc tacgacatcg cgtttcgcgc cgacaacccg   1680
   ggtttatgga tgtttcactg tcacgatctc gaacacgccg cggccggtat ggacgtcatg   1740
   gtccagtacg cgggcatccg cgatccctac ccgatgagcg agatgtcgga g            1791
<210> 11
   <211> 735
   <212> DNA
   <213> Alicyclobacillus sp.
<220>
   <221> misc_feature
   <222> (1)..(735)
   <223> CDS
<220>
   <221> misc_feature
   <222> (1)..(87)
   <223> sig_peptide
<220>
   <221> misc_feature
   <222> (88)..(735)
   <223> mat_peptide
<400> 11
   atgaaacgtc ggaccttgct tgcgggcatc acgctggcgg cgctcgtcgc ggtggcgggc    60
   tgtggcacgc cggccggtaa caccgcctcg ccggacaaca cagcgaactt gtcgaacacg   120
   aacgcgccgg acacgctgtc caatgaaacc ggccagacgc tcgatacggc caacccgccg   180
   tacctgcaca cgtcgaccga gcagtggaag agcatgccga agatgttcat caacccgaac   240
   aagacctatg acgccattgt ccacaccaat tacgggacgt tcaccatcca gctgttcgcc   300
   aaagacgcgc ccatcacggt gaacaacttc gtgttcctgg cagagcacaa cttctaccac   360
   gattgcacgt tcttccgcat cgtgaagaac ttcgtgattc aaacgggcga tcctcgcaac   420
   gacggtaccg gcggcccggg ctacaccatc ccagatgaac tcagccatca ggtgccattc   480
   acgaagggca ttgtcgcgat ggccaacacg ggccagccgc acacgggcgg aagccagttt   540
   ttcatctgca cggccaatga cacgcaggtc ttccagccgc ccaacaatcg ctatacggaa   600
   ttcggccgcg tgatctccgg aatggacgtg atcgacaaga ttgccgccat cccggtgacc   660
   gaaaacccca tgacgcagga agacagctat cctctgaaga ctgcgtacat cgagtcgatt   720
   caaattcaag aatcg                                                    735
<210> 12
   <211> 1824
   <212> DNA
   <213> Alicyclobacillus sp.
<220>
   <221> misc_feature
   <222> (1)..(1824)
   <223> CDS
<220>
   <221> misc_feature
   <222> (1)..(81)
   <223> sig_peptide
<220>
   <221> misc_feature
   <222> (82)..(1824)
   <223> mat_peptide
<400> 12
   gtgaagaagg gaaagagatg gtccgccgcg ctcgcgacgt ccgtggccct gtttgccacc      60
   ctgtcgcccc aagcgctcgc cagcgacacc gtggttccgc aagtgaacac gctcacgccc     120
   attcatcacc tcgtcgtcat cttcgacgag aacgtctcct ttgatcacta tttcgccacc     180
   tatccgaacg ccgccaatcc agccggcgag ccgccctttt acgccgcgcc gggcaccccg     240
   agcgtcaatg gcctgtccgg aagccttctc acgcacaatc ccaacggcgt gaatccgcag     300
   cgcctcgacc gttcccaagc cgtgacgccg gacatgaacc acaactacac gccggagcag     360
   caggccgtgg acgggggccg catggataac tttatcaata cggtcggccg cggaaatccc     420
   atcgatctcg actactacga cggaaacacg gtcaccgcgc tctggtatta cgcgcaacac     480
   ttcgccttga acgacaacgc gtactgcacg cagtacggcc cgtctacgcc tggcgccatc     540
   aacctgattt cgggcgacac cgcgggagcg acggtttatt cttcaagtga gaccagcggc     600
   gccgcacaag tcgtgccacc cggcagcaaa aactttccga atgccgtgac gccaaacggc     660
   gtcgacatcg gcgacatcga tccctactac gacagcgcct ccaaaggcat gaccatggcg     720
   atggccggca aaaacatcgg cgacctgtta aacgcgaagg gggtcacctg gggctggttc     780
   cagggcggct ttgcaaatcc gaacgccaag gacaacaata tcgccggcac agatgaaacc     840
   accgattaca gcgcacacca tgagccgttc cagtattatg cgtctacggc aaatccgaat     900
   catctgccgc ctacgagcgt ggcgatgatc gggcgcacgg atcaggcaaa ccaccagtac     960
   gacatcacga atttcttcca agcattgcaa aacggaaaca tgcccgccgt gagtttcctg    1020
   aaagctcccg aatacgaaga cggtcacgcc ggctattccg atcccctcga cgaacagcgc    1080
   tggctggtcc agaccatcaa tcaaatcgag gcgtcgcccg attggtcctc caccgccatc    1140
   atcatcacct atgacgactc ggatggttgg tacgatcacg tcatgcctcc gctcgtgaac    1200
   ggatcgagcg acaaggccgt ggacgtgctc ggtggcacgc cggttctgca aaacgggacc    1260
   gacagggcgg gctatggacc gcgggtgccg ttcctcgtca tctcgcccta cgccaaacac    1320
   aattttgtcg ataacacgct catcgaccag acttccgttc tgcggttcat cgaggagaac    1380
   tggggcctcg gctcgttggg cccagcgtcg tacgactcgc tcgccggatc gatcatgaac    1440
   atgtttgact ggaacacgca gaacccgcct gtgtttctcg atccgacgac cggtgaaccc    1500
   gtgtccccag atatgcagcc ggaggtcatt cgcggcacca cgtatctcag cctgaatcac    1560
   tacgctcaaa acctcgatgt cgtgctgcaa acctctcggg ggatggcgcg gttctcctac    1620
   gaggggcacg aggtcgagat cgacgagcgt tccgggcttg tccgggtcga tggcgaagcg    1680
   gtccatctca aggcgcctct tgtgcgggtg gacggcgtat ggatggtgcc cgtagaggaa    1740
   atggattcgc tcattggggc cacgctgcac acctacaccg acggtcatct cacctactat    1800
   ctcttttctc cgcaagacgc ccat                                           1824
<210> 13
   <211> 750
   <212> DNA
   <213> Alicyclobacillus sp.
<220>
   <221> misc_feature
   <222> (1)..(750)
   <223> CDS
<220>
   <221> misc_feature
   <222> (1)..(75)
   <223> sig_peptide
<220>
   <221> misc_feature
   <222> (76)..(750)
   <223> mat_peptide
<400> 13
   atgctgagct tgtggaagcg aatccgaacg ggaacactct cacttctggc tgcatgcgcg      60
   tgcgcgctgt cggcgatggg cgctggggca ggatgggtgc atgcggctga gtcccaagcg     120
   caagccccaa gggccattta caaggtggac acgaaggaaa aggtggtcgc tctcacgttc     180
   gacatctcat gggggcaccg cacgcccgaa ccggttctcg agacactcaa gaagtgcggc     240
   gtgaccaagg cgacgttttt cctgagcggt ccttggacca tgcaccacgc ggacatcgca     300
   aagaaaatca aggcgatggg ctacgaaatt ggcagccatg ggtacctgca caaggactat     360
   tccaattacc cggactcttg gattcgagaa caggcgatgc tcgcagacaa ggccattcaa     420
   caggtcactg gggtcaagcc gaagctgttc aggacgccaa atggcgactt gaatccgcgc     480
   gtcatccgct gcctgacgag catgggctac acggtggtcc aatggaacac cgattcgctt     540
   gactggaaaa acccaggcgt cgacgcgatc gtcaaccgcg tcacgaagcg cgtggtgcct     600
   ggcgatatca tcctgatgca cgcgagcgac tcgtccaaac agattgtgga ggccctgccg     660
   cgcatcattg aatcgcttcg gcagcagggc taccggttcg tcaccgtctc cgagctgttg     720
   gcgggcgcca gcgttcaatc caaggtccag                                      750
<210> 14
   <211> 972
   <212> DNA
   <213> Alicyclobacillus sp.
<220>
   <221> misc_feature
   <222> (1)..(972)
   <223> CDS
<220>
   <221> misc_feature
   <222> (1)..(63)
   <223> sig_peptide
<220>
   <221> misc_feature
   <222> (64)..(972)
   <223> mat_peptide
<400> 14
   atgcggaaga cggctgcagg cgcgtgcgcc ctggcgctga tgggggtctt gggcggttgg      60
   gcgggcgcgg ccggcacggc ggtgaacgcg cacgcgccgg cggcgtcggc gccaagtgtt     120
   tcggcacatg tgtgggaaga agtcagccgc acgtggggaa cgcttcccgt cgatgcccgc     180
   cacgacggcg tgtggcacaa catccccggt ttgtcaggct ttgcgctcga cacggcggcg     240
   agcgagcgcg agaccgcgcg gcgccatgac ggcgcgctcc acctggtatg gcgaaccctt     300
   ccgccgaagc gaagactcgg agacctttcg cccgacgtga tttaccgcgg ccccgcgcag     360
   gagaagtcgg tggcgctgat ggtgaatgtg tcctggggcg atgcgtacgt gcccaggatg     420
   cttgaggtgc tgcgcagcgc gcacgtgaag gccacgtttt tcgtggacgg cgcgtttgcg     480
   aagaagttcc ccgatctcgt ccgcgcgatg gcgcgagacg ggcacgcggt cgagtcccac     540
   ggctttggac acccagactt tcgccggctg agcgacgcga agctcgccgc ccagcttgac     600
   gagacgaatc gagtgctcgc cggcatcacg ggcaaggttc cacggctcat cgcgcctccg     660
   gccggatcgt atgatgcgcg cctggctccg ctggcgcatt cgcggcgcat gtacgccatc     720
   ctgtggaccg cggataccgt ggactggaaa aacccgcctg cggatgtcat cgtccaacgc     780
   gttcagcgcg gtgcggaacc cggcgcgttg atcctgatgc atcccacggc gcccacggcg     840
   gaggccctgc ctgatgtgat ccgctggctc gaggggcacg gttatcggct gaaaacggtg     900
   gaggacgtga tcgacgaacg cccagcggtc acccctccga cgacgctggc gaacgagacg     960
   ttccacagcg cg                                                         972
<210> 15
   <211> 642
   <212> DNA
   <213> Alicyclobacillus sp.
<220>
   <221> misc_feature
   <222> (1)..(642)
   <223> CDS
<220>
   <221> misc_feature
   <222> (1)..(87)
   <223> sig_peptide
<220>
   <221> misc_feature
   <222> (88)..(642)
   <223> mat_peptide
<400> 15
   atgatgcgtt ggaattggaa ggttgctgtg ggatcgttgg cgttggccgc actgggcgca     60
   ggggcggcgg tgtcgccggt gtttgcggcg gcgaagtcgt cgaaggccgc gcagtcccac    120
   gcagaggcga gcgcggcagt cgtgatggct gggaagctgt acggcaacat tccgaacgtc    180
   accattcgcg gcgtggaagc tgggaaggcg ccgtgggtcg tggacggatc gtaccagctg    240
   aagagcaacc tgttcacggc gagtgggaag tggctcatca ttccgaagca gggctatatg    300
   gagaacggtc agccggttcc ggccaaaatt ggcggcacga cgaacaacat tccggccgtc    360
   ggggccgaaa tcacgtttgc aaacgcggcg cccattgtgt tgccgccggt caagctgtcg    420
   agccaaggtg acttctcgtt ccacgacgcc atccagtggc cgaagggtgc cgcgcagccg    480
   gtcatcctga ttgggcccga gaagaacggt cagctcgtcg cgtggtttgc ggcgtcggac    540
   ttcctcgccg actacggcca ggcgacgggc atgggcggcg gatgggtgaa cgcggcgcat    600
   ccagagactc ccgtgcggca cacccacctc gcttcgaaga ag                       642
<210> 16
   <211> 771
   <212> DNA
   <213> Alicyclobacillus sp.
<220>
   <221> misc_feature
   <222> (1)..(771)
   <223> CDS
<220>
   <221> misc_feature
   <222> (1)..(63)
   <223> sig_peptide
<220>
   <221> misc_feature
   <222> (64)..(771)
   <223> mat_peptide
<400> 16
   atgaactggg cgcgtgtcgg cgcgtgggta tccacctggc tggtggctac ggcgcttgga      60
   gctggctgtg ggacggcttc gcaagagcat ccgtccaaca cctccacgtc agatcaccgc     120
   gttgcgcccg cggcgccagg cggctccgcc tcgatgcaaa accggcatat tctgcaggag     180
   ccgctgccgc gtggcgtgaa aacggaaacg gatttgtaca actggctttt atggcagaga     240
   ctcgccgaga tcaacaatcc ggcgcagggt gaaatctgcc tggacgccgc atgcaagatt     300
   gcggccaccg tcttttctgg cccggccaag gccgcggccg gcacgcctgt cactctggtg     360
   gcgttttcgc cgcgggcggg ttggcaggtg ctcgtgggtc cgctgcccca gtcggacaac     420
   cctccgcgtc aagcacaatc catcacaggc cagtctgcgc gactacccgc gcaaagaggg     480
   cgtatgcgtc gttcaaaccc acgaaatcga ctggtactgg attcaggacg gacacctgca     540
   gctgatgcgt cagccgcgcg catgacgcgt cagctaaggc gatccgccag ctcgacgaac     600
   gcgtcgagat cgcgcagggc aaagtcgatg gcgcgctgcc aaaagtcagg ttgcgtgaga     660
   tccgcaccga tgtgtttttg ggccagatcc tcgacccgca tgcgaccggt gtcgcgaagc     720
   aacgccacat acttgtccgc aaatcccgtg ccttccgctg aggccatggc a              771
<210> 17
   <211> 3390
   <212> DNA
   <213> Alicyclobacillus sp.
<220>
   <221> misc_feature
   <222> (1)..(3390)
   <223> CDS
<220>
   <221> misc_feature
   <222> (1)..(72)
   <223> sig_peptide
<220>
   <221> misc_feature
   <222> (73)..(3390)
   <223> mat_peptide
<400> 17
   ttgaaacgca cactgagtgg cattgcttca gctgcaattg ttctgggtgc gattagcccg     60
   atggcgtttg cgcagacctc gtccagcggt ctcacgccgg ccggtcagtt gcctatcgtc    120
   gtcaatggac aggttctgtc gaacccgtat gagatggtgg gcatggactc cggcaacaag    180
   acgggcttct tcccgattta ctactttgac caggcgcttg aaaagattgg catcacggcg    240
   acctggaatg gtgcaaccca cacctgggcg ctgacggact ccaacgtcaa tgcttcgaac    300
   gtccaagtcg cgggtggtat gggcacgggg aacaccacgg tgaccctgaa cggcacgccg    360
   attaagatgt tctacaccca ggttgcgaag gacccggcgg gtggcccggt cacgacgtat    420
   atgccgattt actatatcaa caacatcctg agtgcgcttg ggatccatgg aacctttagc    480
   ggacagacgg gtctcaacat taccaccggg cagacgcttg ccggtagcct gagtgccatc    540
   acggtgacgg gggcgacgag cggtacgggg acctcttcga gcccggctgt ggcgttgaat    600
   aacggcaagg ttacgctctc gacgactctg acggattcga atggcaatcc gattggcaac    660
   gcggcggtca ccttcaactt ctctgaatat ggtgcgctgc cttcgaatgc gccgacggtc    720
   accaatgcgt cgggtgcgac aattccggcg accaccggct cgacggctta tcagtacacg    780
   gtctacacca actccagcgg tgtggcttcg atcacggtgt ctgggcccgt tggcttgacc    840
   tacgcatacc aggtgactgc gacggcgccg atcagcaatg gcagcaatca aatgattagc    900
   agccagccgg cgtatgtcga gtttgtcgcc aacaaccagg cgggtattgc gccgtacggc    960
   acggcttctc aaccgtactc ggcttcgctg ggtaccgcag ttcccatcac ggtgattttg   1020
   ccgccgggtg cgaacggtca gccgcaggcg aatgtgctcg tgaccctgtc gctgagcaac   1080
   ccgaatggtg gcaccaacta tgcatacttc accaactcgt cgggtgcgaa tctgggcacg   1140
   caaatccagg tgacgaccaa ctcgtcgggt gtggcgcaag cgtgggtcag cgacgcgaac   1200
   gcgcagcctg ttgtcgtgac ggccaatgtg tcgaatgcga ccaatgtcag caacacttcg   1260
   gtgagcacct acctgaactt tggtcaggca ggcgtgccag catcgatcgc caattacaac   1320
   gatccgtatt cggctttggt ggccaacggt cagcagccgc tcgccggtac gacggtgacg   1380
   attacgggta cgctcgtaga cgctgcaggc aacccggtgg ccaacggtca ggtgcttgta   1440
   accggctcgt cgtccagcgg cgacttcggc tatgtcacga cgtccaacgg caagagcacg   1500
   acgaccgact tcccgagcgt gggtacgttg cagcctggtc agcctgtgag ctccgcgctg 1560
   ggtgacgtca tcacggcgga tgcgaacggc aacttctcgt tgcaagtcac agacacgcag 1620
   aacgagcaag ccagcctgac gttctactcg gtgagcaacg gggtcattag cccggtgggg 1680
   gtcattaaga ccgacacgct gaaattcgca gtgaacaatc agctgtcgac cattgcgctg 1740
   ggtgcgacgg acgctcaagc ggacggcaac cagtacacga atctgacggg tctcacgggt 1800
   tcggacaatg cgccggtgcc ggtgtatgtg gatccgcaga atccgtcggg cacaatggtg 1860
   accaatcaga gcatcaccta tacgctcagc gtcagcagcg gcgacatcgt gggcattggc 1920
   tctggtgcgt atctggcgcc gaccaatgcg aacaacagca cgattccgat caacagcggc 1980
   aacggcctca gctccgtcca ggtcacggtc acggcattgg gcaacaacca ataccagatc 2040
   tcggtgcccg gtcagcaagg cgtgttgacg acctcgtcgc ctgactttac ggtgctggtg 2100
   aaaggctcga cgggttcgac gaagctgacg gtcagctccg gctcactctc gtcgacggca 2160
   accatcacct tcacgtcgag caacccgacg gtggtggcta gcctgacgcc agtttcctcg 2220
   gtgttggcgg ctggtcagaa cgagacggtc accttcaccg tggaagatgc agatggcaat 2280
   ccggtgagcg gtaatacgca ggttgccatc acggcgcatg acagcaatga tccgttgtgg 2340
   atcaccgcag tgaatggcac aaacttgagc gagtatgaga cgattaatgg tgctgcaacg 2400
   tctgtcagca cgccgattcc gctcggtacg agttcgtatg caacctctgg tggttctacg 2460
   ctctacccgg cttacacgaa cagcgggtac tttaagaatg gtgtgagcat cagcggtgtc 2520
   gtatcgtggg atggtacggt gggcgatcca atctacgtca ccaccaactc gcaaggccaa 2580
   gtcacgctga ccttgcaaaa cggcaacgtg acctattttg acggaaacaa caccacgttg 2640
   tcgaatggca tcagcgttgc cggtacgagc ggaagtgaag ggttctacac atattcgagc 2700
   gataccgcag cgacagcgtc ggatcttaca aatatgggcg tgttggtcat tggtcaagcc 2760
   aatggtgacg cttcaacgtc gctcggaacg atttacatcg gcagtggtgg tgctacgcag 2820
   acaccggccg ccttcaccta cgtggatgcc aataaccact cttacacgta ctcgaacacg 2880
   agcgatacat ttacggtatc tagcacccag agtgttagcg gtggcaacta tgcgatcaca 2940
   agcttcacgc cagttggagg tactgcaact tctacaatcc cgagtggcgt gagcgtaaat 3000
   agctcgacgg gtacggtttc ggtgtcccaa aacgctgcag tcggtacgta caccgtgagc 3060
   tattacctga acggcgtcac tgaatccact ggcacgttca aggtgtactc cggcagcggt 3120
   gtggctccta cagagatcac tggctcgtca gtgacggttc ctgctgcaac gtactcgggt 3180
   acgttgaaag tcacggtaag caacggtggt tcgccgctgt acgtgaacgt taccgctgga 3240
   gaatcggcca atgcggtggc tgcagctatt tacaacgcgc ttgtcaatgc caatatcagc 3300
   ggagatacct tctctgtttc gggttcgaca gtcagcgtga ccgctgcgag cggttcgccc 3360
   acgctcacag ttgtcgatgc gaccaatttc 3390
<210> 18
   <211> 744
   <212> DNA
   <213> Alicyclobacillus sp.
<220>
   <221> misc_feature
   <222> (1)..(744)
   <223> CDS
<220>
   <221> misc_feature
   <222> (1)..(123)
   <223> sig_peptide
<220>
   <221> misc_feature
   <222> (124)..(744)
   <223> mat_peptide
<400> 18
   gtgcgaatta tgaaagtttt gggatggatt ttggtaccgt atatcatgct gtttattcag      60
   tgggggcgaa tgaacagaat tctgcgtttt gccggttcat tgtgggcatt aattgtcttc     120
   gcgaacacgg tgtatatgat tcgaggaaac acaccgcgga acgcatcaac ggtaagcgct     180
   acaacttctt tggttaattc gacgaatagt tcacaggtag caaagcaaga gcaaaactcg     240
   agtacgtctc ccgctcataa gtctacgaac tcattgcaac atgcgcaaca tcaagctgct     300
   acgacttcat cttctcagtc gaagttacga tatatcccgt ttcacacata cgggaaggta     360
   ggagacttgg aaattagagt taactccctg cagcaagtta agagtgtggg gtacgacggg     420
   ataggtgaaa ccgcaaatgg tgcgttttgg gttatcaaca tcaccataag aaatgacgga     480
   tccactccta tggaggtcgt tgatggcata ttccatttgc agaacttaaa cgggaacgtt     540
   tatcagccgg attctactgc tgagatatat gcaaatacaa attcagggac tattccgacc     600
   gacctcaacc ctggtgtgtc catgacgaca aatctcgtat ttgatatgcc ggattttatg     660
   acatatggtc acgtcgggca gcattactca cttgtcgctt ccatgggttt cttcgggtca     720
   gatgaaacga cgtatgctct tccg                                            744
<210> 19
   <211> 516
   <212> DNA
   <213> Alicyclobacillus sp.
<220>
   <221> misc_feature
   <222> (1)..(516)
   <223> CDS
<220>
   <221> misc_feature
   <222> (1)..(75)
   <223> sig_peptide
<220>
   <221> misc_feature
   <222> (76)..(516)
   <223> mat_peptide
<400> 19
   atgaaccgca aatccatgtt gtctgtgttg ggtgtggcag ccgcagtagc cctgatggtg      60
   acgggctgtg gcacggccaa cagcacgaac aacacggcgt cgagcggtgc ggccagcaca     120
   gccgtcacgg tgaagcacga gcacaagggg gccaatgctt cgaagacaga gacgaagcag     180
   accgaagcga agtcgtcgaa caaggctgga gaaacggcga agtcgtcggt gaagctcacg     240
   gccccggtgg caggcgcgac ggtgacggcc ggcggcacgc tgaaggtgag cggccaagtg     300
   tcgtcgaacc tcgcgaagaa ggacgtgcaa attacgttga caaatagcgc gaagaaggtg     360
   ctcgtgcagc agatcgtcgg tacgaatagc accggcgcat tcgtggacac gctcaagctt     420
   ccaaagtacc ttgggaaagc cggaagcgac ctgacgctgt cggtgtccgt cgttggcgaa     480
   aatggagtcg taagcacctt gtcgctgcac gtgaag                               516
<210> 20
   <211> 726
   <212> DNA
   <213> Alicyclobacillus sp.
<220>
   <221> misc_feature
   <222> (1)..(726)
   <223> CDS
<220>
   <221> misc_feature
   <222> (1)..(90)
   <223> sig_peptide
<220>
   <221> misc_feature
   <222> (91)..(726)
   <223> mat_peptide
<400> 20
   atgaggcgcg cggttcgtat actagctgcg ctactgtttg ggctggcgac ggtaacagcc    60
   acattgatgt tcgtgcctca ggcaagagcg gccacggtga caggagcgtt ggcgcaatcg   120
   caagtggtgt ccattacggg cggctacaac acgacgacac agatgtatga gcagacgggt   180
   cagcaaaccg tcgttacgaa ttggaccttt tctcttcaac aaactgtcaa ccaaaacaac   240
   gagaatccgt cctacgctca atgcacagtc ttggcgggaa accagcaggt aacgtgcacg   300
   tcggacgcta cgaataacgg tgcaatttgc acatccccct atcctggagc tattgacaag   360
   caatgcacga acctgattgg gttcactgga aacatatcag tgagttcgca aaacggcaat   420
   ccaacgttca ctttttctct tccgagcatc gacccgagta ccatgaagcc agttgggatc   480
   tttgtgacgc ctgagacgat ctatggtcag atgggaacag ggtccgaaag ttatttaagc   540
   tcaggtcaat ctggaggatg gtcatttaac ttttccaacg tctcagatcc tcaagattgg   600
   tattttctcc ttgagttttt ggcgaatcca attgtcgcgg ccattgctgt gcccaccact   660
   caaacggttc cgatttatag ctgggtcacc accacggttt ggcaccccgt tcaaatttcc   720
   tacagc                                                              726
<210> 21
   <211> 540
   <212> DNA
   <213> Alicyclobacillus sp.
<220>
   <221> misc_feature
   <222> (1)..(540)
   <223> CDS
<220>
   <221> misc_feature
   <222> (1)..(72)
   <223> sig_peptide
<220>
   <221> misc_feature
   <222> (73)..(540)
   <223> mat_peptide
<400> 21
   gtggttcgga tgcgcaagcg gttgggactt gttctgagta tggtgacatc tgtgttggtt 60
   ggatgtggcg cttcacatcc gtctccattg aaccaagaca aatctttgtt gacgtggaac 120
   gctgctaaac acgaggtgcg gtggaaagtg gtcgccggcg acggacgcgc aaacggcggt 180
   atgaacttcg atggctatgc caatggcagt atgacactgg tcgtgccgat tgggtggcgc 240
   gtcgtgatcg actttgacaa tgccagtttg atgccgcaca gcgcgatggt ggtgccttac 300
   ggagatcgcg aacgctccaa cttcgacgca acgatggttg cgtttccagg cgcagaaacg 360
   cccaatccgt cacagggaga ccctcaaggg acgcatcggg atgtcatctt cactgctgcg 420
   aaggtgggaa cgtatgccct cgtctgcggg gtcccgggtc acgcgctggc gggaatgtgg 480
   gatcagcttg tggtgtccga tgaagcgaaa cacccgtccc ttcgcgtgca acgcgactca 540
<210> 22
   <211> 1431
   <212> DNA
   <213> Alicyclobacillus sp.
<220>
   <221> misc_feature
   <222> (1)..(1431)
   <223> CDS
<220>
   <221> misc_feature
   <222> (1)..(75)
   <223> sig_peptide
<220>
   <221> misc_feature
   <222> (76)..(1431)
   <223> mat_peptide
<400> 22
   atggcggttc gtagagcgtg gcttctggcg cccttgtgcg cgagcagtct ggtcgtcccg 60
   gcctcggtgc aggccggatt ggcccaggga catggcagct tttcgacggt tcgcgtgtcc 120
   gtggggacgt cgagttccct gtccgtcccc gcgctgattc agggaaacga aacgtacatt 180
   ccgctgtggg acctcatgca ggtgctccat cagctcggct tcaccgcgac gtgggcgaag 240
   ggccaattca gcgtttcggc cccgccatcg gtgccgatgg acgaggcgcc tgggccagcg 300
   ggcaaaggcg gggcgctcgt ggtgctcgac gggcaagtcg tggaacaggt gccgacggtc 360
   atcgccacgc caccgggggc ggccacccct gaggtgtttc tgccgctcac gaacgcggag 420
   gagatcctcg gtcggttggg cattcaggcc agcgcgaccg gcaatcaggt gaacctcgac 480
   gcgtcggctg tgccccaggc gcttcccaac cagcaggtgg ctgtgtggaa cgtgcttgcc 540
   gctgttgcgt ccgatctcgg cgtgtcgacc gcgccagccg ggccgagtcc ctacgccgac 600
   ttgccgacag cctcgccggc gtggggcgcg gtggaggcgg ccattcgtct gggctggtat 660
   tcgcccttat ccgcgtcgtc atccggcgcg tttcaaccca tcacgtgggc gcaaacggca 720
   tccattctgt ggaatgcgct cggcatttca cagcaggacg cggcgtacca gccaggcgga 780
   tcgccgacgg cgtgggcgag cgcccttggc cttgttccag aaaactggga tccagcgtcg 840
   tacatgaccg cgcaggaatt ggacaccttg gcgtcgaatt tgcacgaatg tctgcaagga 900
   gatgtcgaaa cgggcgccaa cacgtggcgg ctctggtatc cgccggctga cgaagtggag 960
   gctaccctcc agtcgggagg cgggcagtcg ctgttcacct cgaccgctga cgcgcaggcc 1020
   gccatctcgt cagcctacca attcttcaat cagcttgtgg tcacaagagt cggccaaggg 1080
   tatgtcgtca ccgttccctc tgtgcctgag ggatatgggt ttgccacctt ttctgcgctc 1140
   ggcggtgtgg cttaccagac gacacccggc ggtccgtgga cggtcgtgcc cgtgctggac 1200
   acgcgcgacg tctccatccc ggccaagggc cgtctcagtg tcaaggttcc cgcgcagggc 1260
   atcaccatca cgtggaatca gatgatgcca tcgctgggcg gaacggtggc catgggcgcg 1320
   ctccaggtgt cgcctggacc cagcgggcct tcggtcgagc gcttgaatat cgtcacaccg 1380
   aacttacctc cggtccttcc gtcgtccgtc acttctacgc aaccgcagtc a 1431
<210> 23
   <211> 1020
   <212> DNA
   <213> Alicyclobacillus sp.
<220>
   <221> misc_feature
   <222> (1)..(1020)
   <223> CDS
<220>
   <221> misc_feature
   <222> (1)..(57)
   <223> sig_peptide
<220>
   <221> misc_feature
   <222> (58)..(1020)
   <223> mat_peptide
<400> 23
   gtgaatcgac agtggaggct agcggtggcg acttctgccg tcgcggccag cctcgcgggg 60
   tgtggagcac cggacctcgc ggcgatgcgg ccgacggtcc aaaagtctgc ggtactcgtg 120
   gaggtcgtgg gcgcgccgcc gtttgcgccc tcagcttcac aactgggaac ggcaggggcc 180
   acctccgtcg aggtggttca cgttgccctt ggcgaatggc agtctgtcgc ggcccacgca 240
   ttggcgaagg ggcaattgac aggggtcatg gtcgtgtgcg acgacgcgaa cgccgtcgcg 300
   tctggcctca accaacttgc tgccgaccat cccgacgttc gctttctcgt ggtcagcaac 360
   tggccggctt cgcaaatcac ctccggaaac gtggaagacg tcgcacagga tcctgtggcc 420
   gtcgcttaca gcattggcgc gctgtgcgga gactggatcg cgagctcaac gtcgacgagc 480
   ggagcggtat acagcggcgt gcccagcatc gtctacgcgc cgcgcggtgc gaccgtggct 540
   gaacaaaaag ccttcttcac gggtctgtat caggcgaacc ccaatgtccg ggtcgtcgcg 600
   cttccgcagc ccgctgcgca gagcctgtcg agctatgggt acgcggtgga tttgggtgtg 660
   gtaggcgggt ctcctgcggc aggggaactg tcggcgcttc gcagtgccgc ccccgcctgg 720
   gctgcttttg gaacgtcgcc gatcgctggc tttgcgattt ctcctggcca tctgtcgtcg 780
   tcggaggccg tgcaagcatt ccaggcgctc gtgtcgccgg acgcgtggca ctcgggtgag 840
   catctcgtgc tcgacttgtc ttcggtggcc ttcgacgaca agcaggtgcc cgcgaccgtc 900
   atcgcggcgt gggccaagct ggaggtcaac gcgatcgcgg ctgcagcgca atcgaacgcg 960
   gccttcgcgt cactgccgcc gagcgtgcgc tcggacctcg ccaatgcgtt tcatttgtca 1020
<210> 24
   <211> 1023
   <212> DNA
   <213> Alicyclobacillus sp.
<220>
   <221> misc_feature
   <222> (1)..(1023)
   <223> CDS
<220>
   <221> misc_feature
   <222> (1)..(87)
   <223> sig_peptide
<220>
   <221> misc_feature
   <222> (88)..(1023)
   <223> mat_peptide
<400> 24
   atggtcatgc gcactcggtg gattcgatgg atggctttgg ctctcgcagt ctgtgtctgg 60
   ctcagcccgt ttcccttctc gtggggcgcg acgagcctcg acgctgatct tccacaaccc 120
   acgattccgc catccgcgtg gagcaacctc aatcaggact ggaaggacct tcagcgcttg 180
   gcgcaaaaca cagtgccgcc ctcgaaagag agcagccaga cccacgcgcc cacacacaag 240
   tcatcgcaac cgcctgccca agtcccgcaa gggccgctcg tcggggtcgg cgatacgggc 300
   gaagcggccc ggtggttaaa cgaagccttg gccgtgctcg gctatttgcc cgccgtcttc 360
   tctcccgcgg cgcagacgtc cacccgtcag gtgcggctcg cactcgcggc gagcgccgag 420
   catcagacgc tcgtgcccat cccaggctcg tttcaacttc tgtatcacgc gccaagctcg 480
   tgggtggcgc tctggtccgc cgacgaagac acgccgatca cggagggcgc cgtcatggcg 540
   tttgaagcac aacatcacct gggcgtggat ggcattgccg ggccggacgt cattcatgcg 600
   ctggcgcagg ccctcgccgg caatgagacg gcagaaaagg cgccctacag ctacatcctg 660
   gtgaccacgt cgttgcccga gacgctcgaa ctctgggtga atggccagct tgtcctcaaa 720
   tcgctgtgca acacaggcat cgcgcagtca cccacgccgt atggcacgta cggcgtctac 780
   gtgcagtaca cgtcgcagga aatgaagggc aaggatccgg acggcacgcc ctacgacgat 840
   cccggcgttc catgggtgag ctacttctac aaaggttgcg cggtccacgg tttcctgcgg 900
   gcaaagtacg gctttcccca gagcctcggt tgcgtggaac tgccgtatgc cgcggccaaa 960
   acggtgttct cctatacgca catcggcacg cttgtcaccg tcaccgcctc cccgctttcc 1020
   gcg 1023
<210> 25
   <211> 1197
   <212> DNA
   <213> Alicyclobacillus sp.
<220>
   <221> misc_feature
   <222> (1)..(1197)
   <223> CDS
<220>
   <221> misc_feature
   <222> (1)..(84)
   <223> sig_peptide
<220>
   <221> misc_feature
   <222> (85)..(1197)
   <223> mat_peptide
<400> 25
   atggataggc tgctgaacaa caaggtggcg cttcgcctga ccgcgctcgt cctcgcgtgc      60
   attctctggc tcgccgtgca cgcggagcag gggtcggggt cctccgcgtc cacgggagtg     120
   accgagtcgt tcgagctgcc ggtgcgggtg gaaacctcgg ccgacgaggt gttggtgtct     180
   caagttccga ccatcaccgc ccgggtgacg acgaacctgt tgagcctgcc gacgctggcc     240
   tcggatatga tgaaagccga gatcgtcgcg gacgccgaaa atctgggccc gggcacgtac     300
   acgttgcacg tggcggccgt caacatgcct gcaggggtgc gatcgtacac gctaacgcct     360
   tccaccatca cggtgacgtt ggagcccaaa gtgacggtgg agcgaacggt gcgggtgaac     420
   gtggtcggca cgccagggca gggatatgtc ctcggcaagc ccgagctcgg cgcgggggtc     480
   gtcgaggtct cgggcgccga atccagtgtg caggccgtgg ccgaggtggc gggcgtcgtg     540
   gacgcgagcg gcctgtcgca gacggcgacc aagctcgtcg agttgttgcc gcttgaccaa     600
   gcgggcaagg cggtgccggg tgtgacggtc acgccatccg cgatttcggt cacgctgccg     660
   atcacgtccg ccaatcaggc ggtgaagctg acgcctgcgg tcaccggcag ccctgcgcct     720
   ggatacgccg tcgcctcggt gcacctggag cccgcgagcg ctgtggaaca ggggctagcg     780
   gccagccagc ttccgcagcg cgggctcctc gtgcccatcg acgtcactgg attgaaccgg     840
   cccacgacgg tgtcggtccc ggtgccgctt ttgccgggga tgacgagcgt ttcgcccacg     900
   gcagtgacgg ccgtgatcga cgtggagccg tccgccgtct acaccgtttc gaacgtcccg     960
   gtggccatca cgggcgcgac gggtgtcaag ctggtgacgc ctcggaccgt gaatgtcacg    1020
   gtgacgggga tcgaggccga cgtgcgcgcg gtggagaggg atccggccgc ggtgcaggcg    1080
   tttgtggacg cgaccgggtt gacacatggc tcggcgacgc tgcccgattc aaattcgtct    1140
   gctgtcctgt ctcttgtgat ccggccacgg gaaaggcgta agcgaacaca tgtagtg       1197
<210> 26
   <211> 959
   <212> PRT
   <213> Alicyclobacillus sp.
<220>
   <221> SIGNAL
   <222> (1)..(24)
<220>
   <221> mat_peptide
   <222> (25)..(959)
   <223> acid endoglucanase or acid cellulase
<400> 26
   Met Lys Thr Arg Trp Ser Gly Ala Leu Ala Val Leu Ile Ala Leu Gly
                   -20                 -15                     -10
   Thr Gly Ala Ser Pro Ala Trp Ala Ser Val His Ser Ala Ala Thr His
               -5              -1  1               5
   Ala Lys Ala His Val Gly Val Arg Ala Ala Asp Met Ala Ala Ala Ser
       10                  15                  20
   Met Ser Ala Glu Ile Gln Ile Leu His Asp Ala Leu Thr Ala Ser Glu
   25                  30                  35                  40
   Leu Ser Ser Val Gln Ala Ala Ala Gln Ala Ala Ala Asn Leu Pro Ala
                   45                  50                  55
   Ser Thr Trp Val Ser Trp Leu Tyr Pro Ser Ala Ser Ser Pro Ser Ala
               60                  65                  70
   Ala Gln Thr Gln Thr Ala Gln Ala Leu Gly Ala Leu Leu Thr Leu Val
           75                  80                  85
   Thr Tyr Gly Ala Val Ala Asp Asp Gly Gln Asn Ile Ala Gln Asn Leu
       90                  95                  100
   Gln Thr Leu Gln Ser Thr Ser Pro Leu Leu Ser Pro Ala Ala Val Ser
   105 110 115 120
   Met Phe Tyr Gln Asn Phe Phe Val Leu Val Gly Gln Ser Ser Lys Ser
   125 130 135
   Val Leu Ser Gly Gln Ala Thr Thr Ser Thr Ala Gly His Ala Leu Ala
   140 145 150
   Gln Ala Ala Ala Leu Thr Pro Gln Leu Ala Ala Tyr Leu Arg Gln Ser
   155 160 165
   Gly Leu Ser Pro Asp Asp Leu Ala Arg Ala Tyr Val Ser Phe Ala Ser
   170 175 180
   Ala Val Asp Ser Gln Gly Ala Ala Gln Thr Ala Leu Leu Thr Arg Ile
   185 190 195 200
   Cys Thr Asn Ile Leu Gly Phe Gly Ala Pro Thr Ser Thr Ala Thr Ile
   205 210 215
   Thr Val Asn Ala Ala Ala Asn Leu Gly Gln Val Pro Thr Thr Ala Phe
   220 225 230
   Gly Leu Asn Ala Ala Val Trp Asp Ser Gly Leu Asn Ser Gln Thr Val
   235 240 245
   Ile Ser Glu Val Gln Ala Leu His Pro Ala Leu Ile Arg Trp Pro Gly
   250 255 260
   Gly Ser Ile Ser Asp Val Tyr Asn Trp Glu Thr Asn Thr Arg Asn Asp
   265 270 275 280
   Gly Gly Tyr Val Asn Pro Asp Asp Thr Phe Asp His Phe Met Gln Phe
   285 290 295
   Val Asn Ala Val Gly Ser Thr Pro Ile Ile Thr Val Asn Tyr Gly Thr
   300 305 310
   Gly Thr Pro Gln Leu Ala Ala Asp Trp Val Lys Tyr Ala Asp Val Thr
   315 320 325
   His His Asp Asn Val Met Tyr Trp Glu Ile Gly Asn Glu Ile Tyr Gly
   330 335 340
   Asn Gly Tyr Tyr Asn Gly Asn Gly Trp Glu Ala Asp Asp His Ala Val
   345 350 355 360
   Ala Gly Gln Pro Gln Lys Gly Asn Pro Gly Leu Ser Pro Gln Ala Tyr
   365 370 375
   Ala Gln Asn Ala Leu Gln Phe Ile Lys Ala Met Arg Ala Glu Asp Pro
   380 385 390
   Ser Ile Lys Ile Gly Ala Val Leu Thr Met Pro Tyr Asn Trp Pro Trp
   395 400 405
   Gly Ala Thr Val Asn Gly Asn Asp Asp Trp Asn Thr Val Val Leu Lys
   410 415 420
   Ala Leu Gly Pro Tyr Ile Asp Phe Val Asp Val His Trp Tyr Pro Glu
   425 430 435 440
   Thr Pro Gly Gln Glu Thr Asp Ala Gly Leu Leu Ala Asp Thr Asp Gln
   445 450 455
   Ile Pro Ala Met Val Ala Glu Leu Lys Arg Glu Val Asn Thr Tyr Ala
   460 465 470
   Gly Ser Asn Ala Lys Asn Ile Gln Ile Phe Val Thr Glu Thr Asn Ser
   475 480 485
   Val Ser Tyr Asn Pro Gly Glu Gln Ser Thr Asn Leu Pro Glu Ala Leu
   490 495 500
   Phe Leu Ala Asp Asp Leu Thr Gly Phe Ile Gln Ala Gly Ala Ala Asn
   505 510 515 520
   Val Asp Trp Trp Asp Leu Phe Asn Gly Ala Glu Asp Asn Tyr Thr Ser
   525 530 535
   Pro Ser Leu Tyr Gly Gln Asn Leu Phe Gly Asp Tyr Gly Leu Leu Ser
   540 545 550
   Ser Gly Gln Thr Thr Gln Asn Gly Trp Gln Glu Pro Pro Ala Asn Thr
   555 560 565
   Pro Leu Pro Pro Tyr Asn Gly Phe Gln Leu Val Ser Asp Phe Ala Gln
   570 575 580
   Pro Gly Asp Thr Met Leu Gly Ser Thr Thr Ser Gln Ser Ala Ile Asp
   585 590 595 600
   Val His Ala Val Arg Lys Pro Asn Gly Asp Ile Ser Leu Met Leu Val
   605 610 615
   Asn Arg Ser Pro Ser Ala Ile Tyr Ser Ala Asn Leu Asn Val Leu Gly
   620 625 630
   Phe Gly Pro Phe Val Val Thr His Ala Leu Ala Tyr Gly Glu Gly Ser
   635 640 645
   Ser Arg Val Ala Pro Met Pro Val Leu Pro Val Pro Gly Ala Pro Ile
   650 655 660
   Lys Leu Met Pro Tyr Ser Gly Ile Asp Leu Thr Leu His Pro Leu Ile
   665 670 675 680
   Pro Ala Pro His Ala Ala Ala Gln Val Thr Asp Thr Leu Thr Leu Ser
   685 690 695
   Ser Pro Thr Val Thr Ala Gly Gly Ala Glu Thr Leu Ser Ala Ser Phe
   700 705 710
   Gln Ala Asp Arg Pro Val His His Ala Thr Val Glu Leu Glu Leu Tyr
   715 720 725
   Asp Ser Thr Asn Asp Leu Val Ala Thr His Thr Val Ser Asp Val Asp
   730 735 740
   Leu Gln Pro Gly Ser Ala Thr Ser Glu Thr Trp Ser Phe Thr Ala Pro
   745 750 755 760
   Ala Ala Asn Gly Asn Tyr Arg Val Glu Ala Phe Val Phe Asp Pro Val
   765 770 775
   Thr Gly Ala Thr Tyr Asp Ala Asp Thr Gln Gly Ala Val Leu Thr Val
   780 785 790
   Asn Gln Pro Pro Gln Ala Thr Tyr Gly Asp Ile Val Thr Lys Asp Thr
   795 800 805
   Val Ile Thr Val Asn Gly Thr Thr Tyr Asp Val Pro Ala Pro Asp Ala
   810 815 820
   Gly Gly His Tyr Pro Ser Gly Thr Asn Ile Ser Val Ala Pro Gly Asp
   825 830 835 840
   Thr Val Thr Val Gln Thr Thr Phe Val Asn Val Ser Ser Thr Asp Ala
   845 850 855
   Leu Gln Asn Gly Leu Ile Asp Met Glu Val Asp Gly Ser Asn Gly Ala
   860 865 870
   Ile Leu Gln Lys Tyr Trp Pro Ser Thr Thr Leu Leu Pro Gly Gln Ser
   875 880 885
   Glu Thr Val Thr Ala Thr Trp Gln Val Pro Ala Asn Val Ala Ala Gly
   890 895 900
   Thr Tyr Pro Leu Asn Phe Gln Ala Phe Asn Thr Ser Ser Trp Thr Gly
   905 910 915 920
   Asn Cys Tyr Phe Thr Asn Gly Gly Val Val Asn Phe Val Ile Ser
   925 930 935
<210> 27
   <211> 272
   <212> PRT
   <213> Alicyclobacillus sp.
<220>
   <221> SIGNAL
   <222> (1)..(32)
<220>
   <221> mat_peptide
   <222> (33)..(272)
   <223> aspartyl protease
<400> 27
   Met Asn Gly Thr Ser Val Trp Lys Ala Ser Gly Ile Ala Ala Ala Ser
           -30                 -25                 -20
   Cys Leu Thr Ala Ala Ala Leu Leu Ala Trp Pro His Ala Thr Ser Thr
       -15                 -10                 -5                  -1
   Leu Asp Ala Ser Pro Ala Ile Phe His Ala Pro Arg His Ala Leu Ser
   1               5                   10                  15
   Pro Asn Thr Ser Pro Lys Pro Asn Ser Val Gln Ala Gln Asn Phe Gly
               20                  25                  30
   Trp Ser Ala Ser Asn Trp Ser Gly Tyr Ala Val Thr Gly Ser Thr Tyr
           35                  40                  45
   Asn Asp Ile Thr Gly Ser Trp Ile Val Pro Ala Val Ser Pro Ser Lys
       50                  55                  60
   Arg Ser Thr Tyr Ser Ser Ser Trp Ile Gly Ile Asp Gly Phe Asn Asn
   65                  70                  75                  80
   Ser Asp Leu Ile Gln Thr Gly Thr Glu Gln Asp Tyr Val Asn Gly His
                   85                  90                  95
   Ala Gln Tyr Asp Ala Trp Trp Glu Ile Leu Pro Ala Pro Glu Thr Val
               100                 105                 110
   Ile Ser Asn Met Thr Ile Ala Pro Gly Asp Arg Met Ser Ala His Ile
           115                 120                 125
   His Asn Asn Gly Asn Gly Thr Trp Thr Ile Thr Leu Thr Asp Val Thr
       130                 135                 140
   Arg Asn Glu Thr Phe Ser Thr Thr Gln Ser Tyr Ser Gly Pro Gly Ser
   145                 150                 155                 160
   Ser Ala Glu Trp Ile Gln Glu Ala Pro Glu Ile Gly Gly Arg Ile Ala
                   165                 170                 175
   Thr Leu Ala Asn Tyr Gly Glu Thr Thr Phe Asp Pro Gly Thr Val Asn
               180                 185                 190
   Gly Gly Asn Pro Gly Phe Thr Leu Ser Asp Ala Gly Tyr Met Val Gln
           195                 200                 205
   Asn Asn Ala Val Val Ser Val Pro Ser Ala Pro Asp Ser Asp Thr Asp
       210                 215                 220
   Gly Phe Asn Val Ala Tyr Gly Ser Asn Gln Pro Ser Pro Pro Ala Ser
   225                 230                 235                 240
<210> 28
   <211> 315
   <212> PRT
   <213> Alicyclobacillus sp.
<220>
   <221> SIGNAL
   <222> (1)..(25)
<220>
   <221> mat_peptide
   <222> (26)..(315)
   <223> multi copper oxidase
<400> 28
   Met Arg Arg Arg Met Ser Gly Phe Ala Thr Gly Leu Gly Ile Ala Ala
   -25                 -20                 -15                 -10
   Gly Leu Ala Leu Ser Ser Ala Leu Ala Ala Pro Phe Phe His Ala Gly
                   -5                  -1  1               5
   Asn Ala Ser Ala Ala Ser Thr Met Ser Met Ala Pro Thr Ser Thr Met
           10                  15                  20
   Gly Ala Leu Pro Ala Pro Glu Gly Val Pro Asp Ala Gly Pro Leu Ser
       25                  30                  35
   Ile Thr Pro Glu Val Ile Arg Gln Gln Gln Ala Asp Ala Val Arg Val
   40                  45                  50                  55
   Met Asp Glu Glu Gly Leu Lys Pro Gln Ile Leu Ser Gly Asp Ile Lys
                   60                  65                  70
   Arg Phe Thr Leu Thr Ala Ser Gln Val Asn Trp Tyr Leu Tyr Pro Gly
               75                  80                  85
   Lys Ala Val Val Ala Cys Gly Tyr Asn Gly Gln Val Pro Gly Pro Val
           90                  95                  100
   Leu Arg Val Arg Val Gly Asp Arg Val Gln Ile Leu Leu Arg Asn Glu
       105                 110                 115
   Leu Asn Glu Pro Thr Thr Leu His Ile Gln Gly Leu Asp Leu Pro Ala
   120                 125                 130                 135
   Ser Gln Leu Gly Ile Gly Asp Val Thr Glu Ser Pro Ile Pro Pro Gly
                   140                 145                 150
   Gly Glu Arg Leu Tyr Ser Phe Thr Val Thr Pro Gln Met Val Gly Thr
               155                 160                 165
   His Leu Tyr Glu Ser Gly Thr Asp Met Ala Ser Glu Ile Asp Pro Arg
           170                 175                 180
   Thr Ala Arg Gly Ala Ala Arg Arg Ser Gly Pro Gly Ile Pro Leu Ser
       185                 190                 195
   Pro Gly Glu Gly Gly Arg Ala Leu Arg Asp Arg Arg Val Asp Gly Gly
   200                 205                 210                 215
   Arg Ile Asp His Arg Lys Arg Val Trp Pro Gly Arg Gln Ala Val Ser
                   220                 225                 230
   Arg Arg Ala Arg Thr Asp Gly Ala Val Arg Gln Pro Arg Gly Ala Ala
               235                 240                 245
   His Arg Gln Arg Glu Arg Asp Val Leu Pro Arg His Ala Pro Ala Arg
           250                 255                 260
   Asp Asp Val Leu Ala Ala Gly Gly Arg Arg Ala Pro Pro Arg Gln Ala
       265                 270                 275
   Ala Ala Asp Glu Arg Ala Arg His Arg Ala Arg
   280                 285                 290
<210> 29
   <211> 626
   <212> PRT
   <213> Alicyclobacillus sp.
<220>
   <221> SIGNAL
   <222> (1)..(32)
<220>
   <221> PROPEP
   <222> (33)..(189)
<220>
   <221> mat_peptide
   <222> (190)..(626)
   <223> serine-carboxyl protease
<400> 29
   Met Gly Leu Trp Lys  Arg Leu Ala Leu Gly  Val Pro Ala Ala Leu
                   -185                 -180                 -175
   Ser Met Leu Ala Val  Gly Val Pro Val Met  Ser Ala Asp Thr Val
                   -170                 -165                 -160
   Glu Ala Ala Pro Leu  Ala Asn Pro Ser Thr  Glu Asn Ala Gln Asp
                   -155                 -150                 -145
   Met Gly Pro Ala Ser  Gly Ser Gln Thr Val  Thr Ala Ser Ile Ile
                   -140                 -135                 -130
   Leu Arg Val Gln Asn  Pro Thr Ala Leu Gln  Asn Tyr Ile Gln Glu
                   -125                 -120                 -115
   Thr Glu Thr Pro Gly  Ser Pro Leu Tyr His  Lys Phe Leu Thr Thr
                   -110                 -105                 -100
   Ala Gln Phe Ala Gln Gln Tyr Ala Pro Ser Ala Ala Thr Leu Gln Gln
                   -95                 -90                 -85
   Ile Glu Gln Glu Leu Gln Gly Tyr Gly Leu Gln Val Val Asn Val Asp
               -80                 -75                 -70
   Ala Asp His Leu Asp Met Gln Val Gln Gly Thr Val Gln Gln Phe Asp
           -65                 -60                 -55
   Asn Ala Phe Asn Thr Val Ile Asp Leu Phe Lys Ala Asn Gly His Ile
       -50                 -45                 -40
   Phe Arg Ala Pro Lys Lys Pro Pro Gln Ile Pro Val Ala Leu Leu Thr
   -35                 -30                 -25                 -20
   Asn Val Leu Ala Val Val Gly Leu Asp Thr Ala Gln Ala Ala Gln Ser
                   -15                 -10                 -5
   Leu Thr Val Lys Thr Pro Asn Val Ala Gly Val Pro Ser Pro Lys Val
           -1  1               5                   10
   Val Leu Pro Gln Gly Gly Ser Thr Ala Thr Gly Thr Pro Gly Ser Tyr
       15                  20                  25
   Thr Val Gly Asp Thr Ala Asn Arg Tyr Asp Ile Asn Pro Leu Tyr Gln
   30                  35                  40                  45
   Lys Gly Ile Thr Gly Lys Gly Glu Thr Ile Gly Ile Val Thr Leu Ser
                   50                  55                  60
   Ser Phe Asn Pro Gln Asp Ala Tyr Thr Tyr Trp Gln Gly Ile Gly Leu
   65 70 75
   Lys Val Ala Pro Asn Arg Ile Gln Met Val Asn Val Asp Gly Gly Gly
   80 85 90
   Gln Met Asp Asp Gly Ser Val Glu Thr Thr Leu Asp Val Glu Gln Ser
   95 100 105
   Gly Gly Leu Ala Pro Asp Ala Asn Val Val Val Tyr Asp Ala Pro Asn
   110 115 120 125
   Thr Asp Gln Gly Phe Ile Asp Ala Phe Tyr Gln Ala Val Ser Asp Asn
   130 135 140
   Gln Ala Asp Ser Leu Ser Val Ser Trp Gly Gln Pro Glu Ile Asp Tyr
   145 150 155
   Leu Pro Gln Met Asn Gln Gly Gln Ser Tyr Val Asp Glu Leu Leu Ala
   160 165 170
   Phe Thr Gln Ala Phe Met Glu Ala Ala Ala Gln Gly Ile Ser Met Tyr
   175 180 185
   Ala Ala Ala Gly Asp Ser Gly Ala Tyr Asp Thr Ala Arg Asp Phe Pro
   190 195 200 205
   Pro Ser Asp Gly Phe Thr Thr Pro Leu Ser Val Asp Phe Pro Ala Ser
   210 215 220
   Asp Pro Tyr Ile Thr Ala Ala Gly Gly Thr Thr Val Pro Phe Thr Ala
   225 230 235
   Lys Phe Ser Leu Gly Thr Val Asn Ile Thr Gln Glu Gln Pro Trp Ser
   240 245 250
   Trp Gln Tyr Leu Gln Asn Leu Gly Tyr Gln Gly Leu Phe Ser Val Gly
   255 260 265
   Thr Gly Gly Gly Val Ser Val Ile Phe Pro Arg Pro Trp Tyr Gln Leu
   270 275 280 285
   Gly Val Gly Gly Met Gln Asn Ser Ala Ala Asn Gln Ala Phe Thr Asp
   290 295 300
   Ser Gln Gly Val Leu Tyr Gly Ser Pro Phe Thr Tyr Asn Leu Pro Ser
   305 310 315
   Asn Tyr Ala Gly Arg Asn Leu Pro Asp Ile Ser Met Asp Ala Asp Pro
   320 325 330
   Glu Thr Gly Tyr Leu Val Tyr Trp Ser Ala Gly Gly Gly Trp Ile Ala
   335 340 345
   Gly Tyr Gly Gly Thr Ser Phe Val Ala Pro Gln Leu Asn Gly Ile Thr
   350 355 360 365
   Ala Leu Ile Asp Gln Glu Val His Gly Arg Val Gly Phe Leu Asn Pro
   370 375 380
   Leu Leu Tyr Thr Leu Leu Thr Gln Gly Val Gln Gly Gly Ala Gln Pro
   385 390 395
   Phe His Asp Ile Thr Thr Gly Asn Asn Trp Tyr Trp Asn Ala Val Pro
   400 405 410
   Gly Tyr Asp Pro Ala Ser Gly Val Gly Thr Pro Asp Val Ala Asn Leu
   415 420 425
   Ala Gln Asp Ile Ala Ser Leu Arg
   430 435
<210> 30
   <211> 533
   <212> PRT
   <213> Alicyclobacillus sp.
<220>
   <221> SIGNAL
   <222> (1)..(24)
<220>
   <221> mat_peptide
   <222> (25)..(534)
   <223> serine-carboxyl protease
<400> 30
   Met Arg Ala Leu Ala His Leu Ala Ile Gly Ala Ile Ala Ser Gly Val
                   -20                 -15                 -10
   Phe Ala Ala Pro Val Ala Phe Ala Ser Pro Val Gln Glu Arg Val Val
               -5              -1  1               5
   Val Ala Ser Pro Asp Pro Arg Thr Arg Ser Val His Ala Asp Gly Glu
       10                  15                  20
   Ile Ser Pro Ser Gln Pro Met His Leu Val Ile Thr Leu Arg Leu Arg
   25                  30                  35                  40
   His Glu Ala Gln Leu Glu Gln Leu Ile Arg Asp Leu Tyr Thr Pro Gly
                   45                  50                  55
   Ser Pro Asp Ala Gly His Phe Leu Thr Pro Ala Ala Phe Asn Ala Ala
               60                  65                  70
   Tyr Ala Pro Thr Ala Glu Asp Val Gln Ala Val Val Gln Gly Leu Arg
   75 80 85
   Ala Tyr Gly Leu Arg Val Glu Pro Thr Val Asn Pro Met Val Leu Thr
   90 95 100
   Val Ser Gly Arg Ala Arg Asp Val Glu Arg Ala Phe Gly Val His Glu
   105 110 115 120
   Leu Gln Phe Gly Arg Gly Ala Gly Ala Trp Tyr Ala Pro Asp Gly Ala
   125 130 135
   Ala Thr Leu Pro Ala Pro Leu Ala Ala Arg Val Ser Ala Val Val Gly
   140 145 150
   Leu Thr Ser Asp Ala Met Glu Arg His Leu Val Leu Ala His Val Ala
   155 160 165
   Pro Ala Gly Gly Gly Tyr Thr Pro Ala Gln Ile Gln Arg Ala Tyr Asp
   170 175 180
   Tyr Thr Pro Leu Tyr Ser Gln Tyr Met Gly Arg Gly Gln Val Ile Ala
   185 190 195 200
   Val Val Thr Ser Gly Ser Val Leu Arg Ser Asp Leu Leu Ala Phe Asp
   205 210 215
   Arg Ala Phe Gly Leu Pro Asn Pro Val Val Arg Gln Arg Val Ile Asp
   220 225 230
   Gly Ser Ser Thr Ser Pro Asp Asp Glu Thr Thr Leu Asp Cys Glu Trp
   235 240 245
   Ala His Ala Ile Ala Pro Thr Ala Ser Leu Ala Val Tyr Glu Ala Ala
   250 255 260
   Gln Pro Asp Ala Gln Ser Phe Ile Asp Ala Phe Ala Gln Val Ala Ala
   265 270 275 280
   Asp Asp Gly Ala His Val Val Thr Thr Ser Trp Gly Ala Pro Glu Ser
   285 290 295
   Glu Thr Asp Ala Ala Thr Met Gln Ala Glu His Gln Ile Phe Met Gln
   300 305 310
   Met Ala Ala Gln Gly Gln Ser Val Phe Ala Ala Ala Gly Asp Ser Gly
   315 320 325
   Ser Ser Asp Gly Thr Ser Gly Thr Asp Val Asp Tyr Pro Ser Ser Asp
   330 335 340
   Pro Tyr Val Thr Ala Cys Gly Gly Thr Arg Leu Val Leu Gly Ala Gly
   345 350 355 360
   Ala Lys Arg Leu Gln Glu Thr Ala Trp Ala Asp Thr Gly Gly Gly Ala
   365 370 375
   Ser Ser Val Tyr Gly Glu Pro Trp Trp Gln Tyr Gly Pro Gly Val Pro
   380 385 390
   Gln Thr Gly Tyr Arg Gln Thr Cys Asp Val Ala Leu Asn Ala Asp Pro
   395 400 405
   Ala Thr Gly Tyr Asp Phe Ile Tyr Glu Gly Gln Trp Glu Val Ala Gly
   410 415 420
   Gly Thr Ser Phe Val Ala Pro Met Met Ala Ala Thr Phe Ala Leu Ile
   425 430 435 440
   Asp Gln Ala Arg Ala Leu Glu Gly Lys Pro Pro Val Gly Leu Ala Asp
   445 450 455
   Val Gly Ile Tyr Ala Met Ala Arg Asn Ala Ser Tyr Ala Pro Tyr Ala
   460 465 470
   Phe His Asp Ile Thr Ala Gly Ser Asn Gly Ala Tyr Ser Ala Gly Pro
   475 480 485
   Gly Trp Asp His Pro Thr Gly Phe Gly Ser Ile Asp Ala Tyr Tyr Phe
   490 495 500
   Leu His Gly Leu Asp
   505
<210> 31
   <211> 360
   <212> PRT
   <213> Alicyclobacillus sp.
<220>
   <221> SIGNAL
   <222> (1)..(41)
<220>
   <221> mat_peptide
   <222> (42)..(411)
   <223> protease or a HtrA-like serine protease
<400> 31 Tyr Ile Leu Gly Ile Trp Thr Gly Ala Ala Leu Thr Arg Gly His Ser -5 -1 1 5 Gln Thr Thr Val Glu Tyr Val Pro Pro Gln Thr Gly Asn Thr Ala Ser 10 15 20 Thr Ser Gly Ser Leu Thr Pro Ile Pro Gly Val Glu Asp Thr Thr Ile 25 30 35 Val Thr Gln Ile Tyr Asn Arg Val Lys Asn Ser Ile Phe Thr Ile Thr 40 45 50 55 Ala Val Ser Gly Gly Lys Pro Thr ser ser Asp Ala Glu Glu Asp Ile 60 65 70 Gly Thr Gly Phe Leu Ile Asp His Asn Gly Asp Leu Leu Thr Asn Ala 75 80 85 His Val Val Gly Ser Ala Thr Thr Val Gln Val Ser Gly Asp Asn Arg 90 95 100 Gln Phe Val Gly Arg Val Ile Asp Ala Asp Gln Leu Asp Asp Leu Ala 105 110 115 Ile Val Arg Ile Pro Ala Pro Lys Ser Leu Glu Pro Leu Pro Leu Gly 120 125 130 135 Ser Val Lys Ser Leu Gln Pro Gly Ser Leu Val Ile Ala Ile Gly Asn 140 145 150 Pro Phe Glu Leu Thr Ser Ser Val Ser Ser Gly Ile Val Ser Gly Leu 155 160 165 Asn Arg Ser Met Ser Glu Ser Asn Gly His Val Met Asn Gly Met Ile 170 175 180 Gln Thr Asp Ala Pro Leu Asn Pro Gly Asn Ser Gly Gly Pro Leu Leu 185 190 195 Asn Ala Ala Gly Gln Val Val Gly Ile Asn Thr Leu Ile Glu Ser Pro 200 205 210 215 Ile Glu Gly ser Ile Gly Ile Gly Phe Ala Ile Pro Ile Asp Arg Phe 220 225 230 Ile Gln Leu Glu Pro Glu Leu Leu Ala Gly Lys Pro Val Ala His Ala 235 240 245 Trp Leu Gly Ile Glu Gly Met Asp Ile Asp Asn Leu Met Arg Gln Ala 250 255 260
<210> 32
   <211> 211
   <212> PRT
   <213> Alicyclobacillus sp.
<220>
   <221> SIGNAL
   <222> (1)..(30)
<220>
   <221> mat_peptide
   <222> (31)..(212)
   <223> disulfide isomerase
<400> 32
   Met Arg Arg Ser Trp Ser Val Leu Met Ala Val Cys Met Ser Trp Leu
   -30 -25 -20 -15
   Ala Val Gly Cys Gly Thr Pro Ala Asn Ser Leu Ser Gln Ala Thr Ala
   -10 -5 -1 1
   Ala Ser Gly Arg His Ala Pro His Pro Leu Val Phe Gln Asn Leu Thr
   5 10 15
   Gly Ala Met Asn Glu Gly Gln Asp Pro Arg Trp Asp Pro Lys Ala Ala
   20 25 30
   Pro Thr Gly Val Tyr Asp Asp Val Thr Val Val Thr Ala Ser Gly Arg
   35 40 45 50
   Gln Glu Val Leu Ser Val Arg Asp Ala Pro Leu Leu Phe Ala Ala Tyr
   55 60 65
   Trp Cys Pro His Cys Gln Arg Thr Leu Gln Leu Leu Thr Ser Ile Glu
   70 75 80
   Ser Arg Leu Lys Gln Lys Pro Ile Leu Val Asn Val Gly Tyr Pro Pro
   85 90 95
   Gly Thr Thr Leu Gln Thr Ala Ala Arg Ile Ala Arg Glu Glu Ser Gln
   100 105 110
   Val Leu His Leu Ala Pro Phe Gln Glu Val Phe Ile Leu Asn Pro Asp
   115 120 125 130
   Ala Gly Asp Arg Tyr Ala Pro Leu Gly Tyr Pro Thr Leu Ala Phe Tyr
   135 140 145
   Arg Ala Gly Arg Asp Trp Thr Leu Tyr Gly Glu His Arg Ala Ser Ile
   150 155 160
   Trp Glu Lys Ala Leu Ser Glu Ser Thr Ser Lys Ala Tyr Asn Gly Ser
   165 170 175
   Glu Glu Ser
   180
<210> 33
   <211> 266
   <212> PRT
   <213> Alicyclobacillus sp.
<220>
   <221> SIGNAL
   <222> (1)..(29)
<220>
   <221> mat_peptide
   <222> (30)..(266)
   <223> gamma-D-glutamyl-L-diamino acid endopeptidase
<400> 33
   Met Asp Glu Met Asn Ile Arg Ser Trp Cys Val Ala Ala Cys Thr Val
                   -25                 -20                 -15
   Ala Leu Thr Ser Ala Val Gly Ala Thr Thr Ala Phe Ala Gln Thr Val
               -10                 -5              -1  1
   Thr Val Gln Pro Gly Gln Ser Leu Trp Thr Ile Ala Arg Ala His Gly
       5                   10                  15
   Met Pro Val Gln Leu Val Ala Ser Ala Asn Pro Gln Tyr Asn Pro Leu
   20                  25                  30                  35
   Asn Leu Pro Val Gly Ala Thr Val Thr Leu Pro Ser Leu Lys Asp Val
                   40                  45                  50
   Ala Val Gln Pro Gly Asp Ser Leu Phe Leu Ile Gly Arg Gln Tyr Gly
               55                  60                  65
   Val Ser Leu Ala Glu Met Leu Ala Ala Asn Pro Asn Val Asp Pro Leu
           70                  75                  80
   Asn Leu Gln Val Gly Ser Ser Val Arg Val Pro Leu Ala Ser Ser Ser
   85 90 95
   Thr Lys Ser Ser Thr Val Ser Ala His Val Ala Ala Ser Thr Pro Glu
   100 105 110 115
   Asn Ser Asn Asn Leu Tyr Trp Leu Glu Arg Val Ile His Ala Glu Ala
   120 125 130
   Gly Gly Glu Ser Leu Gln Ala Gln Ile Ala Val Ala Asp Val Ile Leu
   135 140 145
   His Arg Met Ala Ala Gly Gly Tyr Gly Ser Thr Val Gln Gln Val Val
   150 155 160
   Phe Gln Val Ser Asp Gly His Tyr Gln Phe Glu Ser Val Ala Asn Gly
   165 170 175
   Ser Ile Tyr Gly Gln Pro Asp Ala Gln Asn Val Gln Ala Ala Leu Asp
   180 185 190 195
   Ala Leu Asn Gly Asp Asp Val Val Pro Gly Ala Leu Val Phe Tyr Asn
   200 205 210
   Pro Ala Gln Thr Pro Ser Gly Ser Trp Val Trp Gln Gln Pro Val Val
   215 220 225
   Ala His Ile Gly His Leu Val Phe Ala Lys
   230 235
<210> 34
   <211> 768
   <212> PRT
   <213> Alicyclobacillus sp.
<220>
   <221> SIGNAL
   <222> (1)..(26)
<220>
   <221> mat_peptide
   <222> (27)..(768)
   <223> endo-beta-N-acetylglucosaminidase
<400> 34
   Met Lys Thr His Arg Leu Leu Ala Val Ala Ala Leu Pro Ala Thr Val
   -25 -20 -15
   Leu Leu Thr Thr Pro Ala Pro Ala Leu Ala Glu Thr Ser Ser Ser Gln
   -10 -5 -1 1 5
   Ser Ala Ser Ala Pro Ser Leu Asn Val Pro Val Ala Ala Leu Thr Leu
   10 15 20
   Ala Gly Val Gln Ser Tyr Pro Met Leu Ser Tyr Gly Ser Thr Gly Val
           25                  30                  35
   Tyr Val Glu Ile Leu Gln Asn Ala Leu Asn Ala Leu Gly Tyr Asp Val
       40                  45                  50
   Gly Gln Ala Ser Gly Leu Phe Asp Ala Thr Thr Gln Ala Glu Val Lys
   55                  60                  65                  70
   Ala Phe Gln Gln Ala Met Gly Leu Gln Thr Asp Gly Ile Val Gly Pro
                   75                  80                  85
   Leu Thr Trp Gly Ala Leu Ala Lys Ala Val Ala Asp Tyr Arg Gln Val
               90                  95                  100
   Met Thr Val Leu Ser Ser Arg Ser Ser Leu Val Gln Gln Val Glu Trp
           105                 110                 115
   Lys Arg Ile Val Trp Asn Gly Arg Leu Ile Ser Lys Pro Ile Gly Phe
       120                 125                 130
   Thr Tyr Gln Gly Thr Ala Tyr Met Pro Ile Trp Tyr Val Met Gln Ala
   135                 140                 145                 150
   Leu Ser Lys Ala Gly Ile Ala Ser Thr Trp Gln Gly Gly Val Trp Thr
                   155                 160                 165
   Leu Thr Pro Pro Gly Gly Gln Thr Val Asn Tyr Gly Lys Ile Ser Tyr
               170                 175                 180
   Gly Pro Gly Ser Ala Ala Ile Ala Ile Gly Gln Thr Val Val Ala Asn
           185                 190                 195
   Val Pro Ala Val Val Tyr Pro Asp Pro Ala Ser Gly Lys Leu Thr Thr
       200                 205                 210
   Phe Met Pro Val Trp Tyr Val Met Asn Ala Leu Gln Arg Leu Gly Ile
   215                 220                 225                 230
   Gly Ser Thr Trp Gln Gly Thr Glu Trp Asp Met Lys Pro Ala Pro Val
                   235                 240                 245
   Val Ile Glu Thr Gly Asp Pro Ser Asn Asn Thr Thr Gly Ser Asp Pro
               250                 255                 260
   Ala Asn Ser Thr Gly Asn Gly Thr Gly Asn Ser Thr Gly Asn Ala Thr
           265                 270                 275
   Gly Ala Val Pro Gly Gly Asn Thr Val Thr Asn Val Thr Thr Gly Ser
       280                 285                 290
   Ser Asn Val Thr Gly Asn Ser Thr Gly Asn Ser Leu Gly Asn Ser Thr
   295 300 305 310
   Gly Asn Ser Leu Gly Asn Ser Thr Ser Asn Ala Thr Gly Asn Ala Thr
   315 320 325
   Gly Asn Thr Thr Gly Asn Ala Thr Gly Asn Ser Thr Gly Thr Ser Ser
   330 335 340
   Gly Ser Phe Thr Asn Val Asp Leu Arg Tyr Pro Ala Pro Ser Asn Ile
   345 350 355
   Asn Ala Gln Ser Ile Asn Gln Phe Leu Leu Gln Asn Ser Ser Pro Leu
   360 365 370
   Asn Gly Leu Gly Asn Ser Phe Met Asp Ala Gln Asn Leu Tyr Ser Val
   375 380 385 390
   Asp Ala Asn Tyr Leu Val Ser His Ala Ile Leu Glu Ser Ala Trp Gly
   395 400 405
   Gln Ser Gln Ile Ala Leu Gln Lys Asn Asn Leu Phe Gly Tyr Gly Ala
   410 415 420
   Tyr Asp Ser Asn Pro Gly Gln Asp Ala Gly Val Phe Pro Ser Asp Asp
   425 430 435
   Tyr Ala Ile Arg Phe Glu Ala Trp Thr Val Arg Met Asn Tyr Leu Thr
   440 445 450
   Pro Gly Ala Ser Leu Tyr Val Thr Pro Thr Leu Ser Gly Met Asn Val
   455 460 465 470
   Asn Tyr Ala Thr Ala Lys Thr Trp Ala Ser Gly Ile Ala Ala Ile Met
   475 480 485
   Thr Gln Phe Ala Ser Ser Val Gly Ser Asn Val Asn Ala Tyr Val Gln
   490 495 500
   Tyr Thr Pro Ser Asn Asn Pro Pro Ala Pro Arg Ser Thr Ala Glu Pro
   505 510 515
   Val Tyr Tyr Met Asn Gly Ala Gln Gly Val Thr Gln Gln Asp Pro Tyr
   520 525 530
   Tyr Pro Asn Gly Gly Val Pro Tyr Tyr Pro Thr Ile Ala Gln Gly Glu
   535 540 545 550
   Asn Gln Gln Phe Phe Gly Gln Leu Ser Val Gly Ser Phe Gly Gln Pro
   555 560 565
   Val Val Glu Val Gln Gln Phe Leu Asn Arg Thr Ile Asn Ala Gly Leu
   570 575 580
   Thr Val Asp Gly Gln Phe Gly Pro Leu Thr Gln Ala Ala Val Glu Lys
   585 590 595
   Phe Gln Ser Gln Val Met His Met Ser Asn Pro Asn Gly Ile Trp Thr
   600 605 610
   Phe Ser Met Trp Val Gln Tyr Ile Gln Pro Ser Gln Ser Asn Ala Asn
   615 620 625 630
   Leu Ile Pro Ala Gly Thr Thr Val Lys Ile Asp Gln Val Ala Glu Gly
   635 640 645
   Met Ala Gly Pro Tyr Val Val Pro Trp Tyr His Val Val Gly Tyr Gly
   650 655 660
   Trp Val Asp Ser Gln Tyr Ile Lys Leu Thr Asn Val Tyr Arg Val Ile
   665 670 675
   Val Gln Asn Pro Ala Gly Thr Ala Thr Thr Ile Pro Val Tyr Gln Val
   680 685 690
   Gly Asn Leu Ser Ser Val Leu Leu Asn Leu His Ser Gly Asp Trp Val
   695 700 705 710
   Val Ala Asn Ser Ala Gln Pro Ser Gly Gly Val Tyr Thr Ile Gln Ile
   715 720 725
   Ala Ala Gln Asp Pro Pro Cys Arg Thr Ala Thr Pro Pro Gly Arg Ser
   730 735 740
<210> 35
   <211> 597
   <212> PRT
   <213> Alicyclobacillus sp.
<220>
   <221> SIGNAL
   <222> (1)..(49)
<220>
   <221> mat_peptide
   <222> (50)..(597)
   <223> multi copper oxidase
<220>
   <221> MISC_FEATURE
   <222> (139)..(139)
   <223> putative copper binding site
<220>
   <221> MISC_FEATURE
   <222> (141)..(141)
   <223> putative copper binding site
<220>
   <221> MISC_FEATURE
   <222> (181)..(181)
   <223> putative copper binding site
<220>
   <221> MISC_FEATURE
   <222> (183)..(183)
   <223> putative copper binding site
<220>
   <221> MISC_FEATURE
   <222> (514)..(514)
   <223> putative copper binding site
<220>
   <221> MISC_FEATURE
   <222> (566)..(566)
   <223> putative copper binding site
<400> 35
   Met Met Ala His Asp Arg Leu Asp Arg Arg Val Asn Glu Arg Arg Gln
                   -45                 -40                 -35
   Ala Met Arg Arg Ala Ala Lys Trp Ala Ile Ala Leu Gly Thr Thr Ala
               -30                 -25                 -20
   Val Val Ala Gly Val Ser Ser Val Phe Ala Leu Arg Ser Val Arg Glu
           -15                 -10                 -5
   Ala Asn Leu Asn Pro Asn Ala Pro Leu Ala Asn Val Pro Gly Pro Gln
   -1  1               5                   10                  15
   Gly Ala Tyr Thr Pro Ile Ser Ala Leu Gln Pro Val Val Pro Lys Asn
                   20                  25                  30
   Ala Arg Ile Asp His Tyr Thr Leu Thr Ala Glu Ser Arg Thr Leu Thr
               35                  40                  45
   Val Gly Gly His Ala Leu Gln Ala Met Thr Phe Asn Gly Thr Ala Pro
           50                  55                  60
   Gly Pro Leu Leu Val Ala His Gln Gly Asp Val Val Lys Val Thr Val
       65                  70                  75
   His Asn Arg Leu Ser Val Pro Leu Thr Ile His Trp His Gly Ile Ala
   80                  85                  90                  95
   Val Pro Gly Ala Glu Asp Gly Val Pro Gly Val Thr Gln Asn Pro Ile
                   100                 105                 110
   Pro Pro Gly Gly Ser Tyr Thr Tyr Glu Phe Gln Val Asn Gln Pro Gly
               115                 120                 125
   Thr Tyr Trp Tyr His Ser His Glu Ala Ser Phe Glu Glu Val Gly Leu
   130 135 140
   Gly Leu Tyr Gly Ala Phe Val Val Leu Pro Lys Arg Ala Val His Pro
   145 150 155
   Ala Asp Arg Asp Tyr Thr Leu Val Leu His Glu Trp Pro Thr Ala Ser
   160 165 170 175
   Thr Ala Gln Thr Met Met Ala Asn Leu Lys Ala Gly Asn Leu Gly Phe
   180 185 190
   Ser Ala Lys Gly Glu Ser Ala Gly Met Gly Gly Met Gly Met Gln Gln
   195 200 205
   Asn Gly Asp Met Asn Gly Met Gly Met Met Gly Ala Ala Asp Gly Thr
   210 215 220
   Gly Gln Gly Gly Asn Ser Ala Ser Asp Ile Ala His Val Leu Pro Gly
   225 230 235
   Pro Pro Leu Gln Leu Asn Gly Phe Ser Pro Thr Ala Asn Asp Trp Ala
   240 245 250 255
   Ala Leu Asp Glu Met Ala Gly Met Tyr Asp Ala Phe Thr Val Asn Gln
   260 265 270
   Asn Ala Ser Gly Thr Thr Leu Leu Pro Ala Lys Pro Gly Gln Leu Val
   275 280 285
   Arg Leu Arg Ile Val Asn Ser Gly Asn Met Thr His Leu Phe Thr Leu
   290 295 300
   Val Gly Ala Pro Phe Arg Val Val Ala Leu Asp Gly His Asp Ile Ala
   305 310 315
   Asn Pro Gly Trp Ile Arg Gly Val Leu Leu Pro Val Gly Ala Ala Glu
   320 325 330 335
   Arg Tyr Asp Ile Glu Phe Arg Val Pro Lys Ser Gly Ala Ala Phe Leu
   340 345 350
   Val Cys Ala Asp Pro Asp Thr Thr Ala Gln Arg Glu Leu Arg Ala Ala
   355 360 365
   Ile Gly Leu Pro Asp Ala Trp Ser Gln Phe Lys Glu Thr Asp Ala Ala
   370 375 380
   Ser Leu Glu Arg Ala Pro Trp Phe Asp Phe Thr His Tyr Gly Ser Gly
   385 390 395
   Arg Leu Pro Gly Glu Ala Val Phe Arg Leu His Gln Ala Tyr Gln Val
   400 405 410 415
   Arg Tyr Asn Met Lys Leu Thr Val Gly Met Ser Met Asn Gly Met Val
   420 425 430
   Tyr Ala Ile Asn Gly Lys Val Phe Pro Asn Ile Pro Pro Ile Val Val
   435 440 445
   Arg Lys Gly Asp Ala Val Leu Val His Ile Val Asn Asp Ser Pro Tyr
   450 455 460
   Ile His Pro Met His Leu His Gly His Asp Phe Gln Val Leu Thr Arg
   465 470 475
   Asp Gly Lys Pro Val Ser Gly Ser Pro Ile Phe Leu Asp Thr Leu Asp
   480 485 490 495
   Val Phe Pro Gly Glu Ser Tyr Asp Ile Ala Phe Arg Ala Asp Asn Pro
   500 505 510
   Gly Leu Trp Met Phe His Cys His Asp Leu Glu His Ala Ala Ala Gly
   515 520 525
   Met Asp Val Met Val Gln Tyr Ala Gly Ile Arg Asp Pro Tyr Pro Met
   530 535 540
   Ser Glu Met Ser Glu
   545
<210> 36
   <211> 245
   <212> PRT
   <213> Alicyclobacillus sp.
<220>
   <221> SIGNAL
   <222> (1)..(29)
<220>
   <221> mat_peptide
   <222> (30)..(246)
   <223> peptidyl-prolyl-isomerase
<400> 36
   Met Lys Arg Arg Thr Leu Leu Ala Gly Ile Thr Leu Ala Ala Leu Val
   -25 -20 -15
   Ala Val Ala Gly Cys Gly Thr Pro Ala Gly Asn Thr Ala Ser Pro Asp
   -10 -5 -1 1
   Asn Thr Ala Asn Leu Ser Asn Thr Asn Ala Pro Asp Thr Leu Ser Asn
   5 10 15
   Glu Thr Gly Gln Thr Leu Asp Thr Ala Asn Pro Pro Tyr Leu His Thr
   20 25 30 35
   Ser Thr Glu Gln Trp Lys Ser Met Pro Lys Met Phe Ile Asn Pro Asn
   40 45 50
   Lys Thr Tyr Asp Ala Ile Val His Thr Asn Tyr Gly Thr Phe Thr Ile
   55 60 65
   Gln Leu Phe Ala Lys Asp Ala Pro Ile Thr Val Asn Asn Phe Val Phe
   70 75 80
   Leu Ala Glu His Asn Phe Tyr His Asp Cys Thr Phe Phe Arg Ile Val
   85 90 95
   Lys Asn Phe Val Ile Gln Thr Gly Asp Pro Arg Asn Asp Gly Thr Gly
   100 105 110 115
   Gly Pro Gly Tyr Thr Ile Pro Asp Glu Leu Ser His Gln Val Pro Phe
   120 125 130
   Thr Lys Gly Ile Val Ala Met Ala Asn Thr Gly Gln Pro His Thr Gly
   135 140 145
   Gly Ser Gln Phe Phe Ile Cys Thr Ala Asn Asp Thr Gln Val Phe Gln
   150 155 160
   Pro Pro Asn Asn Arg Tyr Thr Glu Phe Gly Arg Val Ile Ser Gly Met
   165 170 175
   Asp Val Ile Asp Lys Ile Ala Ala Ile Pro Val Thr Glu Asn Pro Met
   180 185 190 195
   Thr Gln Glu Asp Ser Tyr Pro Leu Lys Thr Ala Tyr Ile Glu Ser Ile
   200 205 210
   Gln Ile Gln Glu Ser
   215
<210> 37
   <211> 608
   <212> PRT
   <213> Alicyclobacillus sp.
<220>
   <221> SIGNAL
   <222> (1)..(27)
<220>
   <221> mat_peptide
   <222> (28)..(608)
   <223> acid phosphatase or a phytase or a phospholipase C
<400> 37
   Met Lys Lys Gly Lys Arg Trp Ser Ala Ala Leu Ala Thr Ser Val Ala
           -25                 -20                 -15
   Leu Phe Ala Thr Leu Ser Pro Gln Ala Leu Ala Ser Asp Thr Val Val
       -10                 -5                  -1  1               5
   Pro Gln Val Asn Thr Leu Thr Pro Ile His His Leu Val Val Ile Phe
                   10                  15                  20
   Asp Glu Asn Val Ser Phe Asp His Tyr Phe Ala Thr Tyr Pro Asn Ala
               25                  30                  35
   Ala Asn Pro Ala Gly Glu Pro Pro Phe Tyr Ala Ala Pro Gly Thr Pro
           40                  45                  50
   Ser Val Asn Gly Leu Ser Gly Ser Leu Leu Thr His Asn Pro Asn Gly
       55                  60                  65
   Val Asn Pro Gln Arg Leu Asp Arg Ser Gln Ala Val Thr Pro Asp Met
   70                  75                  80                  85
   Asn His Asn Tyr Thr Pro Glu Gln Gln Ala Val Asp Gly Gly Arg Met
                   90                  95                  100
   Asp Asn Phe Ile Asn Thr Val Gly Arg Gly Asn Pro Ile Asp Leu Asp
               105                 110                 115
   Tyr Tyr Asp Gly Asn Thr Val Thr Ala Leu Trp Tyr Tyr Ala Gln His
           120                 125                 130
   Phe Ala Leu Asn Asp Asn Ala Tyr Cys Thr Gln Tyr Gly Pro Ser Thr
       135                 140                 145
   Pro Gly Ala Ile Asn Leu Ile Ser Gly Asp Thr Ala Gly Ala Thr Val
   150                 155                 160                 165
   Tyr Ser Ser Ser Glu Thr Ser Gly Ala Ala Gln Val Val Pro Pro Gly
                   170                 175                 180
   Ser Lys Asn Phe Pro Asn Ala Val Thr Pro Asn Gly Val Asp Ile Gly
               185                 190                 195
   Asp Ile Asp Pro Tyr Tyr Asp Ser Ala Ser Lys Gly Met Thr Met Ala
           200                 205                 210
   Met Ala Gly Lys Asn Ile Gly Asp Leu Leu Asn Ala Lys Gly Val Thr
       215                 220                 225
   Trp Gly Trp Phe Gln Gly Gly Phe Ala Asn Pro Asn Ala Lys Asp Asn
   230                 235                 240                 245
   Asn Ile Ala Gly Thr Asp Glu Thr Thr Asp Tyr Ser Ala His His Glu
   250 255 260
   Pro Phe Gln Tyr Tyr Ala Ser Thr Ala Asn Pro Asn His Leu Pro Pro
   265 270 275
   Thr Ser Val Ala Met Ile Gly Arg Thr Asp Gln Ala Asn His Gln Tyr
   280 285 290
   Asp Ile Thr Asn Phe Phe Gln Ala Leu Gln Asn Gly Asn Met Pro Ala
   295 300 305
   Val Ser Phe Leu Lys Ala Pro Glu Tyr Glu Asp Gly His Ala Gly Tyr
   310 315 320 325
   Ser Asp Pro Leu Asp Glu Gln Arg Trp Leu Val Gln Thr Ile Asn Gln
   330 335 340
   Ile Glu Ala Ser Pro Asp Trp Ser Ser Thr Ala Ile Ile Ile Thr Tyr
   345 350 355
   Asp Asp Ser Asp Gly Trp Tyr Asp His Val Met Pro Pro Leu Val Asn
   360 365 370
   Gly Ser Ser Asp Lys Ala Val Asp Val Leu Gly Gly Thr Pro Val Leu
   375 380 385
   Gln Asn Gly Thr Asp Arg Ala Gly Tyr Gly Pro Arg Val Pro Phe Leu
   390 395 400 405
   Val Ile Ser Pro Tyr Ala Lys His Asn Phe Val Asp Asn Thr Leu Ile
   410 415 420
   Asp Gln Thr Ser Val Leu Arg Phe Ile Glu Glu Asn Trp Gly Leu Gly
   425 430 435
   Ser Leu Gly Pro Ala Ser Tyr Asp Ser Leu Ala Gly Ser Ile Met Asn
   440 445 450
   Met Phe Asp Trp Asn Thr Gln Asn Pro Pro Val Phe Leu Asp Pro Thr
   455 460 465
   Thr Gly Glu Pro Val Ser Pro Asp Met Gln Pro Glu Val Ile Arg Gly
   470 475 480 485
   Thr Thr Tyr Leu Ser Leu Asn His Tyr Ala Gln Asn Leu Asp Val Val
   490 495 500
   Leu Gln Thr Ser Arg Gly Met Ala Arg Phe Ser Tyr Glu Gly His Glu
   505 510 515
   Val Glu Ile Asp Glu Arg Ser Gly Leu Val Arg Val Asp Gly Glu Ala
   520 525 530
   Val His Leu Lys Ala Pro Leu Val Arg Val Asp Gly Val Trp Met Val
   535 540 545
   Pro Val Glu Glu Met Asp Ser Leu Ile Gly Ala Thr Leu His Thr Tyr
   550 555 560 565
   Thr Asp Gly His Leu Thr Tyr Tyr Leu Phe Ser Pro Gln Asp Ala His
   570 575 580
<210> 38
   <211> 250
   <212> PRT
   <213> Alicyclobacillus sp.
<220>
   <221> SIGNAL
   <222> (1)..(25)
<220>
   <221> mat_peptide
   <222> (26)..(251)
   <223> polysaccharide deacetylase or a xylan deacetylase
<400> 38
   Met Leu Ser Leu Trp Lys Arg Ile Arg Thr Gly Thr Leu Ser Leu Leu
   -25                 -20                 -15                 -10
   Ala Ala Cys Ala Cys Ala Leu Ser Ala Met Gly Ala Gly Ala Gly Trp
                   -5              -1  1               5
   Val His Ala Ala Glu Ser Gln Ala Gln Ala Pro Arg Ala Ile Tyr Lys
           10                  15                  20
   Val Asp Thr Lys Glu Lys Val Val Ala Leu Thr Phe Asp Ile Ser Trp
       25                  30                  35
   Gly His Arg Thr Pro Glu Pro Val Leu Glu Thr Leu Lys Lys Cys Gly
   40                  45                  50                  55
   Val Thr Lys Ala Thr Phe Phe Leu Ser Gly Pro Trp Thr Met His His
                   60                  65                  70
   Ala Asp Ile Ala Lys Lys Ile Lys Ala Met Gly Tyr Glu Ile Gly Ser
               75                  80                  85
   His Gly Tyr Leu His Lys Asp Tyr Ser Asn Tyr Pro Asp Ser Trp Ile
           90                  95                  100
   Arg Glu Gln Ala Met Leu Ala Asp Lys Ala Ile Gln Gln Val Thr Gly
       105                 110                 115
   Val Lys Pro Lys Leu Phe Arg Thr Pro Asn Gly Asp Leu Asn Pro Arg
   120                 125                 130                 135
   Val Ile Arg Cys Leu Thr Ser Met Gly Tyr Thr Val Val Gln Trp Asn
                   140                 145                 150
   Thr Asp Ser Leu Asp Trp Lys Asn Pro Gly Val Asp Ala Ile Val Asn
               155                 160                 165
   Arg Val Thr Lys Arg Val Val Pro Gly Asp Ile Ile Leu Met His Ala
           170                 175                 180
   Ser Asp Ser Ser Lys Gln Ile Val Glu Ala Leu Pro Arg Ile Ile Glu
       185                 190                 195
   Ser Leu Arg Gln Gln Gly Tyr Arg Phe Val Thr Val Ser Glu Leu Leu
   200                 205                 210                 215
   Ala Gly Ala Ser Val Gln Ser Lys Val Gln
                   220                 225
<210> 39
   <211> 324
   <212> PRT
   <213> Alicyclobacillus sp.
<220>
   <221> SIGNAL
   <222> (1)..(21)
<220>
   <221> mat_peptide
   <222> (22)..(324)
   <223> polysaccharide deacetylase or a xylan deacetylase
<400> 39
   Met Arg Lys Thr Ala Ala Gly Ala Cys Ala Leu Ala Leu Met Gly Val
       -20                 -15                 -10
   Leu Gly Gly Trp Ala Gly Ala Ala Gly Thr Ala Val Asn Ala His Ala
   -5              -1  1               5                   10
   Pro Ala Ala Ser Ala Pro Ser Val Ser Ala His Val Trp Glu Glu Val
               15                  20                  25
   Ser Arg Thr Trp Gly Thr Leu Pro Val Asp Ala Arg His Asp Gly Val
           30                  35                  40
   Trp His Asn Ile Pro Gly Leu Ser Gly Phe Ala Leu Asp Thr Ala Ala
       45                  50                  55
   Ser Glu Arg Glu Thr Ala Arg Arg His Asp Gly Ala Leu His Leu Val
   60 65 70 75
   Trp Arg Thr Leu Pro Pro Lys Arg Arg Leu Gly Asp Leu Ser Pro Asp
   80 85 90
   Val Ile Tyr Arg Gly Pro Ala Gln Glu Lys Ser Val Ala Leu Met Val
   95 100 105
   Asn Val Ser Trp Gly Asp Ala Tyr Val Pro Arg Met Leu Glu Val Leu
   110 115 120
   Arg Ser Ala His Val Lys Ala Thr Phe Phe Val Asp Gly Ala Phe Ala
   125 130 135
   Lys Lys Phe Pro Asp Leu Val Arg Ala Met Ala Arg Asp Gly His Ala
   140 145 150 155
   Val Glu Ser His Gly Phe Gly His Pro Asp Phe Arg Arg Leu Ser Asp
   160 165 170
   Ala Lys Leu Ala Ala Gln Leu Asp Glu Thr Asn Arg Val Leu Ala Gly
   175 180 185
   Ile Thr Gly Lys Val Pro Arg Leu Ile Ala Pro Pro Ala Gly Ser Tyr
   190 195 200
   Asp Ala Arg Leu Ala Pro Leu Ala His Ser Arg Arg Met Tyr Ala Ile
   205 210 215
   Leu Trp Thr Ala Asp Thr Val Asp Trp Lys Asn Pro Pro Ala Asp Val
   220 225 230 235
   Ile Val Gln Arg Val Gln Arg Gly Ala Glu Pro Gly Ala Leu Ile Leu
   240 245 250
   Met His Pro Thr Ala Pro Thr Ala Glu Ala Leu Pro Asp Val Ile Arg
   255 260 265
   Trp Leu Glu Gly His Gly Tyr Arg Leu Lys Thr Val Glu Asp Val Ile
   270 275 280
   Asp Glu Arg Pro Ala Val Thr Pro Pro Thr Thr Leu Ala Asn Glu Thr
   285 290 295
   Phe His Ser Ala
   300
<210> 40
   <211> 214
   <212> PRT
   <213> Alicyclobacillus sp.
<220>
   <221> SIGNAL
   <222> (1)..(29)
<220>
   <221> mat_peptide
   <222> (30)..(214)
   <223> sulfite oxidase
<400> 40
   Met Met Arg Trp Asn Trp Lys Val Ala Val Gly Ser Leu Ala Leu Ala
                   -25                 -20                 -15
   Ala Leu Gly Ala Gly Ala Ala Val Ser Pro Val Phe Ala Ala Ala Lys
               -10                 -5              -1  1
   Ser Ser Lys Ala Ala Gln Ser His Ala Glu Ala Ser Ala Ala Val Val
       5                   10                  15
   Met Ala Gly Lys Leu Tyr Gly Asn Ile Pro Asn Val Thr Ile Arg Gly
   20                  25                  30                  35
   Val Glu Ala Gly Lys Ala Pro Trp Val Val Asp Gly Ser Tyr Gln Leu
                   40                  45                  50
   Lys Ser Asn Leu Phe Thr Ala Ser Gly Lys Trp Leu Ile Ile Pro Lys
               55                  60                  65
   Gln Gly Tyr Met Glu Asn Gly Gln Pro Val Pro Ala Lys Ile Gly Gly
           70                  75                  80
   Thr Thr Asn Asn Ile Pro Ala Val Gly Ala Glu Ile Thr Phe Ala Asn
       85                  90                  95
   Ala Ala Pro Ile Val Leu Pro Pro Val Lys Leu Ser Ser Gln Gly Asp
   100                 105                 110                 115
   Phe Ser Phe His Asp Ala Ile Gln Trp Pro Lys Gly Ala Ala Gln Pro
                   120                 125                 130
   Val Ile Leu Ile Gly Pro Glu Lys Asn Gly Gln Leu Val Ala Trp Phe
               135                 140                 145
   Ala Ala Ser Asp Phe Leu Ala Asp Tyr Gly Gln Ala Thr Gly Met Gly
           150                 155                 160
   Gly Gly Trp Val Asn Ala Ala His Pro Glu Thr Pro Val Arg His Thr
       165                 170                 175
   His Leu Ala Ser Lys Lys
   180                 185
<210> 41
   <211> 257
   <212> PRT
   <213> Alicyclobacillus sp.
<220>
   <221> SIGNAL
   <222> (1)..(21)
<220>
   <221> mat_peptide
   <222> (22)..(257)
   <223> functional polypeptide
<400> 41
   Met Asn Trp Ala Arg Val Gly Ala Trp Val Ser Thr Trp Leu Val Ala
       -20                 -15                 -10
   Thr Ala Leu Gly Ala Gly Cys Gly Thr Ala Ser Gln Glu His Pro Ser
   -5              -1  1               5                   10
   Asn Thr Ser Thr Ser Asp His Arg Val Ala Pro Ala Ala Pro Gly Gly
               15                  20                  25
   Ser Ala Ser Met Gln Asn Arg His Ile Leu Gln Glu Pro Leu Pro Arg
           30                  35                  40
   Gly Val Lys Thr Glu Thr Asp Leu Tyr Asn Trp Leu Leu Trp Gln Arg
       45                  50                  55
   Leu Ala Glu Ile Asn Asn Pro Ala Gln Gly Glu Ile Cys Leu Asp Ala
   60                  65                  70                  75
   Ala Cys Lys Ile Ala Ala Thr Val Phe Ser Gly Pro Ala Lys Ala Ala
                   80                  85                  90
   Ala Gly Thr Pro Val Thr Leu Val Ala Phe Ser Pro Arg Ala Gly Trp
               95                  100                 105
   Gln Val Leu Val Gly Pro Leu Pro Gln Ser Asp Asn Pro Pro Arg Gln
           110                 115                 120
   Ala Gln Ser Ile Thr Gly Gln Ser Ala Arg Leu Pro Ala Gln Arg Gly
       125                 130                 135
   Arg Met Arg Arg Ser Asn Pro Arg Asn Arg Leu Val Leu Asp Ser Gly
   140                 145                 150                 155
   Arg Thr Pro Ala Ala Asp Ala Ser Ala Ala Arg Met Thr Arg Gln Leu
                   160                 165                 170
   Arg Arg Ser Ala Ser Ser Thr Asn Ala Ser Arg Ser Arg Arg Ala Lys
               175                 180                 185
   Ser Met Ala Arg Cys Gln Lys Ser Gly Cys Val Arg Ser Ala Pro Met
           190                 195                 200
   Cys Phe Trp Ala Arg Ser Ser Thr Arg Met Arg Pro Val Ser Arg Ser
       205                 210                 215
   Asn Ala Thr Tyr Leu Ser Ala Asn Pro Val Pro Ser Ala Glu Ala Met
   220                 225                 230                 235
   Ala
<210> 42
   <211> 1130
   <212> PRT
   <213> Alicyclobacillus sp.
<220>
   <221> SIGNAL
   <222> (1)..(24)
<220>
   <221> mat_peptide
   <222> (25)..(1130)
   <223> functional polypeptide
<400> 42
   Met Lys Arg Thr Leu Ser Gly Ile Ala Ser Ala Ala Ile Val Leu Gly
                   -20                 -15                 -10
   Ala Ile Ser Pro Met Ala Phe Ala Gln Thr Ser Ser Ser Gly Leu Thr
               -5              -1  1               5
   Pro Ala Gly Gln Leu Pro Ile Val Val Asn Gly Gln Val Leu Ser Asn
       10                  15                  20
   Pro Tyr Glu Met Val Gly Met Asp Ser Gly Asn Lys Thr Gly Phe Phe
   25                  30                  35                  40
   Pro Ile Tyr Tyr Phe Asp Gln Ala Leu Glu Lys Ile Gly Ile Thr Ala
                   45                  50                  55
   Thr Trp Asn Gly Ala Thr His Thr Trp Ala Leu Thr Asp Ser Asn Val
               60                  65                  70
   Asn Ala Ser Asn Val Gln Val Ala Gly Gly Met Gly Thr Gly Asn Thr
           75                  80                  85
   Thr Val Thr Leu Asn Gly Thr Pro Ile Lys Met Phe Tyr Thr Gln Val
       90                  95                  100
   Ala Lys Asp Pro Ala Gly Gly Pro Val Thr Thr Tyr Met Pro Ile Tyr
   105                 110                 115                 120
   Tyr Ile Asn Asn Ile Leu Ser Ala Leu Gly Ile His Gly Thr Phe Ser
   125 130 135
   Gly Gln Thr Gly Leu Asn Ile Thr Thr Gly Gln Thr Leu Ala Gly Ser
   140 145 150
   Leu Ser Ala Ile Thr Val Thr Gly Ala Thr Ser Gly Thr Gly Thr Ser
   155 160 165
   Ser Ser Pro Ala Val Ala Leu Asn Asn Gly Lys Val Thr Leu Ser Thr
   170 175 180
   Thr Leu Thr Asp Ser Asn Gly Asn Pro Ile Gly Asn Ala Ala Val Thr
   185 190 195 200
   Phe Asn Phe Ser Glu Tyr Gly Ala Leu Pro Ser Asn Ala Pro Thr Val
   205 210 215
   Thr Asn Ala Ser Gly Ala Thr Ile Pro Ala Thr Thr Gly Ser Thr Ala
   220 225 230
   Tyr Gln Tyr Thr Val Tyr Thr Asn Ser Ser Gly Val Ala Ser Ile Thr
   235 240 245
   Val Ser Gly Pro Val Gly Leu Thr Tyr Ala Tyr Gln Val Thr Ala Thr
   250 255 260
   Ala Pro Ile Ser Asn Gly Ser Asn Gln Met Ile Ser Ser Gln Pro Ala
   265 270 275 280
   Tyr Val Glu Phe Val Ala Asn Asn Gln Ala Gly Ile Ala Pro Tyr Gly
   285 290 295
   Thr Ala Ser Gln Pro Tyr Ser Ala Ser Leu Gly Thr Ala Val Pro Ile
   300 305 310
   Thr Val Ile Leu Pro Pro Gly Ala Asn Gly Gln Pro Gln Ala Asn Val
   315 320 325
   Leu Val Thr Leu Ser Leu Ser Asn Pro Asn Gly Gly Thr Asn Tyr Ala
   330 335 340
   Tyr Phe Thr Asn Ser Ser Gly Ala Asn Leu Gly Thr Gln Ile Gln Val
   345 350 355 360
   Thr Thr Asn Ser Ser Gly Val Ala Gln Ala Trp Val Ser Asp Ala Asn
   365 370 375
   Ala Gln Pro Val Val Val Thr Ala Asn Val Ser Asn Ala Thr Asn Val
   380 385 390
   Ser Asn Thr Ser Val Ser Thr Tyr Leu Asn Phe Gly Gln Ala Gly Val
           395                 400                 405
   Pro Ala Ser Ile Ala Asn Tyr Asn Asp Pro Tyr Ser Ala Leu Val Ala
       410                 415                 420
   Asn Gly Gln Gln Pro Leu Ala Gly Thr Thr Val Thr Ile Thr Gly Thr
   425                 430                 435                 440
   Leu Val Asp Ala Ala Gly Asn Pro Val Ala Asn Gly Gln Val Leu Val
                   445                 450                 455
   Thr Gly Ser Ser Ser Ser Gly Asp Phe Gly Tyr Val Thr Thr Ser Asn
               460                 465                 470
   Gly Lys Ser Thr Thr Thr Asp Phe Pro Ser Val Gly Thr Leu Gln Pro
           475                 480                 485
   Gly Gln Pro Val Ser Ser Ala Leu Gly Asp Val Ile Thr Ala Asp Ala
       490                 495                 500
   Asn Gly Asn Phe Ser Leu Gln Val Thr Asp Thr Gln Asn Glu Gln Ala
   505                 510                 515                 520
   Ser Leu Thr Phe Tyr Ser Val Ser Asn Gly Val Ile Ser Pro Val Gly
                   525                 530                 535
   Val Ile Lys Thr Asp Thr Leu Lys Phe Ala Val Asn Asn Gln Leu Ser
               540                 545                 550
   Thr Ile Ala Leu Gly Ala Thr Asp Ala Gln Ala Asp Gly Asn Gln Tyr
           555                 560                 565
   Thr Asn Leu Thr Gly Leu Thr Gly Ser Asp Asn Ala Pro Val Pro Val
       570                 575                 580
   Tyr Val Asp Pro Gln Asn Pro Ser Gly Thr Met Val Thr Asn Gln Ser
   585                 590                 595                 600
   Ile Thr Tyr Thr Leu Ser Val Ser Ser Gly Asp Ile Val Gly Ile Gly
                   605                 610                 615
   Ser Gly Ala Tyr Leu Ala Pro Thr Asn Ala Asn Asn Ser Thr Ile Pro
               620                 625                 630
   Ile Asn Ser Gly Asn Gly Leu Ser Ser Val Gln Val Thr Val Thr Ala
           635                 640                 645
   Leu Gly Asn Asn Gln Tyr Gln Ile Ser Val Pro Gly Gln Gln Gly Val
       650                 655                 660
   Leu Thr Thr Ser Ser Pro Asp Phe Thr Val Leu Val Lys Gly Ser Thr
   665                 670                 675                 680
   Gly Ser Thr Lys Leu Thr Val Ser Ser Gly Ser Leu Ser Ser Thr Ala
                   685                 690                 695
   Thr Ile Thr Phe Thr Ser Ser Asn Pro Thr Val Val Ala Ser Leu Thr
               700                 705                 710
   Pro Val Ser Ser Val Leu Ala Ala Gly Gln Asn Glu Thr Val Thr Phe
           715                 720                 725
   Thr Val Glu Asp Ala Asp Gly Asn Pro Val Ser Gly Asn Thr Gln Val
       730                 735                 740
   Ala Ile Thr Ala His Asp Ser Asn Asp Pro Leu Trp Ile Thr Ala Val
   745                 750                 755                 760
   Asn Gly Thr Asn Leu Ser Glu Tyr Glu Thr Ile Asn Gly Ala Ala Thr
                   765                 770                 775
   Ser Val Ser Thr Pro Ile Pro Leu Gly Thr Ser Ser Tyr Ala Thr Ser
               780                 785                 790
   Gly Gly Ser Thr Leu Tyr Pro Ala Tyr Thr Asn Ser Gly Tyr Phe Lys
           795                 800                 805
   Asn Gly Val Ser Ile Ser Gly Val Val Ser Trp Asp Gly Thr Val Gly
       810                 815                 820
   Asp Pro Ile Tyr Val Thr Thr Asn Ser Gln Gly Gln Val Thr Leu Thr
   825                 830                 835                 840
   Leu Gln Asn Gly Asn Val Thr Tyr Phe Asp Gly Asn Asn Thr Thr Leu
                   845                 850                 855
   Ser Asn Gly Ile Ser Val Ala Gly Thr Ser Gly Ser Glu Gly Phe Tyr
               860                 865                 870
   Thr Tyr Ser Ser Asp Thr Ala Ala Thr Ala Ser Asp Leu Thr Asn Met
           875                 880                 885
   Gly Val Leu Val Ile Gly Gln Ala Asn Gly Asp Ala Ser Thr Ser Leu
       890                 895                 900
   Gly Thr Ile Tyr Ile Gly Ser Gly Gly Ala Thr Gln Thr Pro Ala Ala
   905                 910                 915                 920
   Phe Thr Tyr Val Asp Ala Asn Asn His Ser Tyr Thr Tyr Ser Asn Thr
                   925                 930                 935
   Ser Asp Thr Phe Thr Val Ser Ser Thr Gln Ser Val Ser Gly Gly Asn
   940 945 950
   Tyr Ala Ile Thr Ser Phe Thr Pro Val Gly Gly Thr Ala Thr Ser Thr
   955 960 965
   Ile Pro Ser Gly Val Ser Val Asn Ser Ser Thr Gly Thr Val Ser Val
   970 975 980
   Ser Gln Asn Ala Ala Val Gly Thr Tyr Thr Val Ser Tyr Tyr Leu Asn
   985 990 995 1000
   Gly Val Thr Glu Ser Thr Gly Thr Phe Lys Val Tyr Ser Gly Ser
   1005 1010 1015
   Gly Val Ala Pro Thr Glu Ile Thr Gly Ser Ser Val Thr Val Pro
   1020 1025 1030
   Ala Ala Thr Tyr Ser Gly Thr Leu Lys Val Thr Val Ser Asn Gly
   1035 1040 1045
   Gly Ser Pro Leu Tyr Val Asn Val Thr Ala Gly Glu Ser Ala Asn
   1050 1055 1060
   Ala Val Ala Ala Ala Ile Tyr Asn Ala Leu Val Asn Ala Asn Ile
   1065 1070 1075
   Ser Gly Asp Thr Phe Ser Val Ser Gly Ser Thr Val Ser Val Thr
   1080 1085 1090
   Ala Ala Ser Gly Ser Pro Thr Leu Thr Val Val Asp Ala Thr Asn
   1095 1100 1105
   Phe
<210> 43
   <211> 248
   <212> PRT
   <213> Alicyclobacillus sp.
<220>
   <221> SIGNAL
   <222> (1)..(41)
<220>
   <221> mat_peptide
   <222> (42)..(248)
   <223> functional polypeptide
<400> 43
   Met Arg Ile Met Lys Val Leu Gly Trp Ile Leu Val Pro Tyr Ile Met
   -40 -35 -30
   Leu Phe Ile Gln Trp Gly Arg Met Asn Arg Ile Leu Arg Phe Ala Gly
   -25 -20 -15 -10
   Ser Leu Trp Ala Leu Ile Val Phe Ala Asn Thr Val Tyr Met Ile Arg
   -5 -1 1 5
   Gly Asn Thr Pro Arg Asn Ala Ser Thr Val Ser Ala Thr Thr Ser Leu
   10 15 20
   Val Asn Ser Thr Asn Ser Ser Gln Val Ala Lys Gln Glu Gln Asn Ser
   25 30 35
   Ser Thr Ser Pro Ala His Lys Ser Thr Asn Ser Leu Gln His Ala Gln
   40 45 50 55
   His Gln Ala Ala Thr Thr Ser Ser Ser Gln Ser Lys Leu Arg Tyr Ile
   60 65 70
   Pro Phe His Thr Tyr Gly Lys Val Gly Asp Leu Glu Ile Arg Val Asn
   75 80 85
   Ser Leu Gln Gln Val Lys Ser Val Gly Tyr Asp Gly Ile Gly Glu Thr
   90 95 100
   Ala Asn Gly Ala Phe Trp Val Ile Asn Ile Thr Ile Arg Asn Asp Gly
   105 110 115
   Ser Thr Pro Met Glu Val Val Asp Gly Ile Phe His Leu Gln Asn Leu
   120 125 130 135
   Asn Gly Asn Val Tyr Gln Pro Asp Ser Thr Ala Glu Ile Tyr Ala Asn
   140 145 150
   Thr Asn Ser Gly Thr Ile Pro Thr Asp Leu Asn Pro Gly Val Ser Met
   155 160 165
   Thr Thr Asn Leu Val Phe Asp Met Pro Asp Phe Met Thr Tyr Gly His
   170 175 180
   Val Gly Gln His Tyr Ser Leu Val Ala Ser Met Gly Phe Phe Gly Ser
   185 190 195
   Asp Glu Thr Thr Tyr Ala Leu Pro
   200 205
<210> 44
   <211> 172
   <212> PRT
   <213> Alicyclobacillus sp.
<220>
   <221> SIGNAL
   <222> (1)..(25)
<220>
   <221> mat_peptide
   <222> (26)..(172)
   <223> functional polypeptide
<400> 44
   Met Asn Arg Lys Ser Met Leu Ser Val Leu Gly Val Ala Ala Ala Val
   -25                 -20                 -15                 -10
   Ala Leu Met Val Thr Gly Cys Gly Thr Ala Asn Ser Thr Asn Asn Thr
                   -5              -1  1               5
   Ala Ser Ser Gly Ala Ala Ser Thr Ala Val Thr Val Lys His Glu His
           10                  15                  20
   Lys Gly Ala Asn Ala Ser Lys Thr Glu Thr Lys Gln Thr Glu Ala Lys
       25                  30                  35
   Ser Ser Asn Lys Ala Gly Glu Thr Ala Lys Ser Ser Val Lys Leu Thr
   40                  45                  50                  55
   Ala Pro Val Ala Gly Ala Thr Val Thr Ala Gly Gly Thr Leu Lys Val
                   60                  65                  70
   Ser Gly Gln Val Ser Ser Asn Leu Ala Lys Lys Asp Val Gln Ile Thr
               75                  80                  85
   Leu Thr Asn Ser Ala Lys Lys Val Leu Val Gln Gln Ile Val Gly Thr
           90                  95                  100
   Asn Ser Thr Gly Ala Phe Val Asp Thr Leu Lys Leu Pro Lys Tyr Leu
       105                 110                 115
   Gly Lys Ala Gly Ser Asp Leu Thr Leu Ser Val Ser Val Val Gly Glu
   120                 125                 130                 135
   Asn Gly Val Val Ser Thr Leu Ser Leu His Val Lys
                   140                 145
<210> 45
   <211> 242
   <212> PRT
   <213> Alicyclobacillus sp.
<220>
   <221> SIGNAL
   <222> (1)..(30)
<220>
   <221> mat_peptide
   <222> (31)..(242)
   <223> functional polypeptide
<400> 45
   Met Arg Arg Ala Val Arg Ile Leu Ala Ala Leu Leu Phe Gly Leu Ala
   -30 -25 -20 -15
   Thr Val Thr Ala Thr Leu Met Phe Val Pro Gln Ala Arg Ala Ala Thr
   -10 -5 -1 1
   Val Thr Gly Ala Leu Ala Gln Ser Gln Val Val Ser Ile Thr Gly Gly
   5 10 15
   Tyr Asn Thr Thr Thr Gln Met Tyr Glu Gln Thr Gly Gln Gln Thr Val
   20 25 30
   Val Thr Asn Trp Thr Phe Ser Leu Gln Gln Thr Val Asn Gln Asn Asn
   35 40 45 50
   Glu Asn Pro Ser Tyr Ala Gln Cys Thr Val Leu Ala Gly Asn Gln Gln
   55 60 65
   Val Thr Cys Thr Ser Asp Ala Thr Asn Asn Gly Ala Ile Cys Thr Ser
   70 75 80
   Pro Tyr Pro Gly Ala Ile Asp Lys Gln Cys Thr Asn Leu Ile Gly Phe
   85 90 95
   Thr Gly Asn Ile Ser Val Ser Ser Gln Asn Gly Asn Pro Thr Phe Thr
   100 105 110
   Phe Ser Leu Pro Ser Ile Asp Pro Ser Thr Met Lys Pro Val Gly Ile
   115 120 125 130
   Phe Val Thr Pro Glu Thr Ile Tyr Gly Gln Met Gly Thr Gly Ser Glu
   135 140 145
   Ser Tyr Leu Ser Ser Gly Gln Ser Gly Gly Trp Ser Phe Asn Phe Ser
   150 155 160
   Asn Val Ser Asp Pro Gln Asp Trp Tyr Phe Leu Leu Glu Phe Leu Ala
   165 170 175
   Asn Pro Ile Val Ala Ala Ile Ala Val Pro Thr Thr Gln Thr Val Pro
   180 185 190
   Ile Tyr Ser Trp Val Thr Thr Thr Val Trp His Pro Val Gln Ile Ser
   195 200 205 210
   Tyr Ser
<210> 46
   <211> 180
   <212> PRT
   <213> Alicyclobacillus sp.
<220>
   <221> SIGNAL
   <222> (1)..(24)
<220>
   <221> mat_peptide
   <222> (25)..(180)
   <223> functional polypeptide
<400> 46
   Val Val Arg Met Arg Lys Arg Leu Gly Leu Val Leu Ser Met Val Thr
                   -20                 -15                 -10
   Ser Val Leu Val Gly Cys Gly Ala Ser His Pro Ser Pro Leu Asn Gln
               -5              -1  1               5
   Asp Lys Ser Leu Leu Thr Trp Asn Ala Ala Lys His Glu Val Arg Trp
       10                  15                  20
   Lys Val Val Ala Gly Asp Gly Arg Ala Asn Gly Gly Met Asn Phe Asp
   25                  30                  35                  40
   Gly Tyr Ala Asn Gly Ser Met Thr Leu Val Val Pro Ile Gly Trp Arg
                   45                  50                  55
   Val Val Ile Asp Phe Asp Asn Ala Ser Leu Met Pro His Ser Ala Met
               60                  65                  70
   Val Val Pro Tyr Gly Asp Arg Glu Arg Ser Asn Phe Asp Ala Thr Met
           75                  80                  85
   Val Ala Phe Pro Gly Ala Glu Thr Pro Asn Pro Ser Gln Gly Asp Pro
       90                  95                  100
   Gln Gly Thr His Arg Asp Val Ile Phe Thr Ala Ala Lys Val Gly Thr
   105                 110                 115                 120
   Tyr Ala Leu Val Cys Gly Val Pro Gly His Ala Leu Ala Gly Met Trp
                   125                 130                 135
   Asp Gln Leu Val Val Ser Asp Glu Ala Lys His Pro Ser Leu Arg Val
               140                 145                 150
   Gln Arg Asp Ser
           155
<210> 47
   <211> 477
   <212> PRT
   <213> Alicyclobacillus sp.
<220>
   <221> SIGNAL
   <222> (1)..(25)
<220>
   <221> mat_peptide
   <222> (26)..(477)
   <223> functional polypeptide
<400> 47 <211> 340
   <212> PRT
   <213> Alicyclobacillus sp.
<220>
   <221> SIGNAL
   <222> (1)..(19)
<220>
   <221> mat_peptide
   <222> (20)..(340)
   <223> functional polypeptide
<400> 48
<210> 49
   <211> 341
   <212> PRT
   <213> Alicyclobacillus sp.
<220>
   <221> SIGNAL
   <222> (1)..(29)
<220>
   <221> mat_peptide
   <222> (30)..(341)
   <223> functional polypeptide
<400> 49
   Met Val Met Arg Thr Arg Trp Ile Arg Trp Met Ala Leu Ala Leu Ala
   -25 -20 -15
   Val Cys Val Trp Leu Ser Pro Phe Pro Phe Ser Trp Gly Ala Thr Ser
   -10 -5 -1 1
   Leu Asp Ala Asp Leu Pro Gln Pro Thr Ile Pro Pro Ser Ala Trp Ser
   5 10 15
   Asn Leu Asn Gln Asp Trp Lys Asp Leu Gln Arg Leu Ala Gln Asn Thr
   20 25 30 35
   Val Pro Pro Ser Lys Glu Ser Ser Gln Thr His Ala Pro Thr His Lys
   40 45 50
   Ser Ser Gln Pro Pro Ala Gln Val Pro Gln Gly Pro Leu Val Gly Val
   55 60 65
   Gly Asp Thr Gly Glu Ala Ala Arg Trp Leu Asn Glu Ala Leu Ala Val
   70 75 80
   Leu Gly Tyr Leu Pro Ala Val Phe Ser Pro Ala Ala Gln Thr Ser Thr
   85 90 95
   Arg Gln Val Arg Leu Ala Leu Ala Ala Ser Ala Glu His Gln Thr Leu
   100 105 110 115
   Val Pro Ile Pro Gly Ser Phe Gln Leu Leu Tyr His Ala Pro Ser Ser
   120 125 130
   Trp Val Ala Leu Trp Ser Ala Asp Glu Asp Thr Pro Ile Thr Glu Gly
   135 140 145
   Ala Val Met Ala Phe Glu Ala Gln His His Leu Gly Val Asp Gly Ile
   150 155 160
   Ala Gly Pro Asp Val Ile His Ala Leu Ala Gln Ala Leu Ala Gly Asn
   165 170 175
   Glu Thr Ala Glu Lys Ala Pro Tyr Ser Tyr Ile Leu Val Thr Thr Ser
   180 185 190 195
   Leu Pro Glu Thr Leu Glu Leu Trp Val Asn Gly Gln Leu Val Leu Lys
   200 205 210
   Ser Leu Cys Asn Thr Gly Ile Ala Gln Ser Pro Thr Pro Tyr Gly Thr
   215 220 225
   Tyr Gly Val Tyr Val Gln Tyr Thr Ser Gln Glu Met Lys Gly Lys Asp
   230 235 240
   Pro Asp Gly Thr Pro Tyr Asp Asp Pro Gly Val Pro Trp Val Ser Tyr
   245 250 255
   Phe Tyr Lys Gly Cys Ala Val His Gly Phe Leu Arg Ala Lys Tyr Gly
   260 265 270 275
   Phe Pro Gln Ser Leu Gly Cys Val Glu Leu Pro Tyr Ala Ala Ala Lys
   280 285 290
   Thr Val Phe Ser Tyr Thr His Ile Gly Thr Leu Val Thr Val Thr Ala
   295 300 305
   Ser Pro Leu Ser Ala
   310
<210> 50
   <211> 399
   <212> PRT
   <213> Alicyclobacillus sp.
<220>
   <221> SIGNAL
   <222> (1)..(28)
<220>
   <221> mat_peptide
   <222> (30)..(399)
   <223> functional polypeptide
<400> 50
   Met Asp Arg Leu Leu Asn Asn Lys Val Ala Leu Arg Leu Thr Ala Leu
                   -25                 -20                 -15
   Val Leu Ala Cys Ile Leu Trp Leu Ala Val His Ala Glu Gln Gly Ser
               -10                 -5              -1  1
   Gly Ser Ser Ala Ser Thr Gly Val Thr Glu Ser Phe Glu Leu Pro Val
       5                   10                  15
   Arg Val Glu Thr Ser Ala Asp Glu Val Leu Val Ser Gln Val Pro Thr
   20                  25                  30                  35
   Ile Thr Ala Arg Val Thr Thr Asn Leu Leu Ser Leu Pro Thr Leu Ala
                   40                  45                  50
   Ser Asp Met Met Lys Ala Glu Ile Val Ala Asp Ala Glu Asn Leu Gly
               55                  60                  65
   Pro Gly Thr Tyr Thr Leu His Val Ala Ala Val Asn Met Pro Ala Gly
           70                  75                  80
   Val Arg Ser Tyr Thr Leu Thr Pro Ser Thr Ile Thr Val Thr Leu Glu
       85                  90                  95
   Pro Lys Val Thr Val Glu Arg Thr Val Arg Val Asn Val Val Gly Thr
   100                 105                 110                 115
   Pro Gly Gln Gly Tyr Val Leu Gly Lys Pro Glu Leu Gly Ala Gly Val
                   120                 125                 130
   Val Glu Val Ser Gly Ala Glu Ser Ser Val Gln Ala Val Ala Glu Val
               135                 140                 145
   Ala Gly Val Val Asp Ala Ser Gly Leu Ser Gln Thr Ala Thr Lys Leu
           150                 155                 160
   Val Glu Leu Leu Pro Leu Asp Gln Ala Gly Lys Ala Val Pro Gly Val
       165                 170                 175
   Thr Val Thr Pro Ser Ala Ile Ser Val Thr Leu Pro Ile Thr Ser Ala
   180                 185                 190                 195
   Asn Gln Ala Val Lys Leu Thr Pro Ala Val Thr Gly Ser Pro Ala Pro
                   200                 205                 210
   Gly Tyr Ala Val Ala Ser Val His Leu Glu Pro Ala Ser Ala Val Glu
               215                 220                 225
   Gln Gly Leu Ala Ala Ser Gln Leu Pro Gln Arg Gly Leu Leu Val Pro
           230                 235                 240
   Ile Asp Val Thr Gly Leu Asn Arg Pro Thr Thr Val Ser Val Pro Val
       245                 250                 255
   Pro Leu Leu Pro Gly Met Thr Ser Val Ser Pro Thr Ala Val Thr Ala
   260                 265                 270                 275
   Val Ile Asp Val Glu Pro Ser Ala Val Tyr Thr Val Ser Asn Val Pro
                   280                 285                 290
   Val Ala Ile Thr Gly Ala Thr Gly Val Lys Leu Val Thr Pro Arg Thr
               295                 300                 305
   Val Asn Val Thr Val Thr Gly Ile Glu Ala Asp Val Arg Ala Val Glu
           310                 315                 320
   Arg Asp Pro Ala Ala Val Gln Ala Phe Val Asp Ala Thr Gly Leu Thr
       325                 330                 335
   His Gly Ser Ala Thr Leu Pro Asp Ser Asn Ser Ser Ala Val Leu Ser
   340                 345                 350                 355
   Leu Val Ile Arg Pro Arg Glu Arg Arg Lys Arg Thr His Val Val
                   360                 365                 370
<210> 51
   <211> 34
   <212> DNA
   <213> Primer SigA2NotU-P
<400> 51
   tcgcgatccg ttttcgcatt tatcgtgaaa cgct 34
<210> 52
   <211> 33
   <212> DNA
   <213> Primer SigA2NotD-P
<400> 52
   ccgcaaacgc tggtgaaagt aaaagatgct gaa 33
<210> 53
   <211> 20
   <212> DNA
   <213> Primer A2up
<400> 53
   agcgtttgcg gccgcgatcc 20
<210> 54
   <211> 21
   <212> DNA
   <213> Primer B
<400> 54
   ttattcggtc gaaaaggatc c 21
<210> 55
   <211> 282
   <212> PRT
   <213> Aspergillus niger
<220>
   <221> SIGNAL
   <222> (1)..(18)
<220>
   <221> PROPEP
   <222> (19)..(59)
<220>
   <221> CHAIN
   <222> (60)..(98)
<220>
   <221> PROPEP
   <222> (99)..(109)
<220>
   <221> CHAIN
   <222> (110)..(282)
<220>
   <221> MOD_RES
   <222> (110)..(110)
<220>
   <221> DISULFID
   <222> (115)..(139)
<220>
   <221> DISULFID
   <222> (127)..(210)
<400> 55
   Met Lys Phe Ser Thr Ile Leu Thr Gly Ser Leu Phe Ala Thr Ala Ala
   1               5                   10                  15
   Leu Ala Ala Pro Leu Thr Glu Lys Arg Arg Ala Arg Lys Glu Ala Arg
               20                  25                  30
   Ala Ala Gly Lys Arg His Ser Asn Pro Pro Tyr Ile Pro Gly Ser Asp
           35                  40                  45
   Lys Glu Ile Leu Lys Leu Asn Gly Thr Thr Asn Glu Glu Tyr Ser Ser
       50                  55                  60
   Asn Trp Ala Gly Ala Val Leu Ile Gly Asp Gly Tyr Thr Lys Val Thr
   65                  70                  75                  80
   Gly Glu Phe Thr Val Pro Ser Val Ser Ala Gly Ser Ser Gly Ser Ser
                   85                  90                  95
   Gly Tyr Gly Gly Gly Tyr Gly Tyr Trp Lys Asn Lys Arg Gln Ser Glu
               100                 105                 110
   Glu Tyr Cys Ala Ser Ala Trp Val Gly Ile Asp Gly Asp Thr Cys Glu
           115                 120                 125
   Thr Ala Ile Leu Gln Thr Gly Val Asp Phe Cys Tyr Glu Asp Gly Gln
       130                 135                 140
   Thr Ser Tyr Asp Ala Trp Tyr Glu Trp Tyr Pro Asp Tyr Ala Tyr Asp
   145                 150                 155                 160
   Phe Ser Asp Ile Thr Ile Ser Glu Gly Asp Ser Ile Lys Val Thr Val
                   165                 170                 175
   Glu Ala Thr Ser Lys Ser Ser Gly Ser Ala Thr Val Glu Asn Leu Thr
               180                 185                 190
   Thr Gly Gln Ser Val Thr His Thr Phe Ser Gly Asn Val Glu Gly Asp
           195                 200                 205
   Leu Cys Glu Thr Asn Ala Glu Trp Ile Val Glu Asp Phe Glu Ser Gly
       210                 215                 220
   Asp Ser Leu Val Ala Phe Ala Asp Phe Gly Ser Val Thr Phe Thr Asn
   225                 230                 235                 240
   Ala Glu Ala Thr Ser Gly Gly Ser Thr Val Gly Pro Ser Asp Ala Thr
                   245                 250                 255
   Val Met Asp Ile Glu Gln Asp Gly Ser Val Leu Thr Glu Thr Ser Val
               260                 265                 270
   Ser Gly Asp Ser Val Thr Val Thr Tyr Val
           275                 280
<210> 56
   <211> 252
   <212> PRT
   <213> sclerotinia sclerotiorum
<220>
   <221> MISC_FEATURE
   <222> (1)..(252)
   <223> endopeptidase EapC
<400> 56
   Met Lys Phe Ser Ile Val Ala Ala Thr Ala Leu Leu Ala Gly Ser Ala
   1               5                   10                  15
   Val Ala Ala Pro Gly Thr Ala Leu Arg Gln Ala Arg Ala Val Lys Arg
               20                  25                  30
   Ala Ala Arg Thr His Gly Asn Pro Val Lys Tyr Val Glu Gly Pro Thr
           35                  40                  45
   Asn Lys Thr Asp Val Ser Tyr Ser Ser Asn Trp Ala Gly Ala Val Leu
       50                  55                  60
   Val Gly Thr Gly Tyr Thr Ser Val Thr Gly Thr Phe Thr Ala Pro Ser
   65                  70                  75                  80
   Pro Ser Thr Ala Gly Ser Gly Ser Ala Trp Val Gly Ile Asp Gly Asp
                   85                  90                  95
   Thr Cys Gly Thr Ala Ile Leu Gln Thr Gly Ile Asp Trp Asp Lys Ser
               100                 105                 110
   Gly Asn Ser Ile Thr Tyr Asp Ala Trp Tyr Glu Trp Tyr Pro Asp Tyr
           115                 120                 125
   Ala Tyr Asp Phe Ser Gly Ile Ser Ile Ser Ala Gly Asp Ser Ile Lys
       130                 135                 140
   Val Thr Val Thr Ala Ser Ser Lys Thr Thr Gly Thr Ala Thr Val Asp
   145                 150                 155                 160
   Asn Leu Thr Lys Gly Lys Ser Val Thr His Thr Phe Ser Gly Gly Val
                   165                 170                 175
   Asp Gly Asp Leu Cys Glu Tyr Asn Ala Glu Trp Ile Val Glu Asp Phe
               180                 185                 190
   Glu Glu Gly Ser Ser Leu Val Gln Phe Ala Asn Phe Gly Thr Val Thr
           195                 200                 205
   Phe Thr Gly Ala Ser Ala Thr Gln Asn Gly Glu Ser Val Gly Val Thr
       210                 215                 220
   Gly Ala Gln Ile Ile Asp Leu Gln Gln Asn Ser Val Leu Thr Ser Val
   225 230 235 240
   Ser Thr Ser Ser Asn Ser Val Thr Val Lys Tyr Val
   245 250
<210> 57
   <211> 269
   <212> PRT
   <213> Cryphonectria parasitica
<220>
   <221> MISC_FEATURE
   <222> (1)..(269)
   <223> endopeptidase EapC
<400> 57
   Met Lys Tyr Ala Thr Val Val Ala Ala Leu Leu Gly Ala Asn Ala Ala
   1 5 10 15
   Leu Gly Ala Arg Phe Thr Glu Lys Arg Arg Glu Arg Asn Glu Ala Arg
   20 25 30
   Leu Ala Arg Arg Ser Gly Ser Val Arg Leu Pro Ala Thr Asn Ser Glu
   35 40 45
   Gly Val Ala Ile Asp Ala Ala Glu Ser Arg Asn Asp Thr Thr Asn Val
   50 55 60
   Glu Tyr Ser Ser Asn Trp Ala Gly Ala Val Leu Ile Gly Ser Gly Tyr
   65 70 75 80
   Lys Ser Val Thr Gly Ile Phe Val Val Pro Thr Pro Lys Ser Pro Gly
   85 90 95
   Ser Gly Asn Thr Glu Tyr Ala Ala Ser Ala Trp Val Gly Ile Asp Gly
   100 105 110
   Asp Thr Ala Gln Asn Ser Ile Leu Gln Thr Gly Val Asp Phe Tyr Val
   115 120 125
   Glu Gly Ser Ser Val Ala Tyr Asp Ala Trp Tyr Glu Trp Tyr Pro Asp
   130 135 140
   Tyr Ala Tyr Asp Phe Ser Gly Ile Ser Ile Ser Ala Gly Asp Thr Ile
   145 150 155 160
   Lys Val Thr Val Thr Ala Thr Thr Thr Thr Ser Gly Thr Ala Val Val
   165 170 175
   Glu Asn Val Thr Lys Gly Thr Thr Val Thr His Thr Phe Thr Gly Gln
   180 185 190
   Ser Ala Ala Leu Gln Glu Leu Asn Ala Glu Trp Ile Val Glu Asp Phe
   195 200 205
   Glu Glu Gly Asp Glu Leu Val Pro Phe Ala Asn Phe Gly Thr Val Thr
   210 215 220
   Phe Thr Gly Ala Glu Ala Thr Thr Ser Ser Gly Thr Val Thr Ala Ala
   225 230 235 240
   Asp Ala Thr Leu Ile Asp Ile Glu Gln Asn Gly Glu Val Leu Thr Ser
   245 250 255
   Val Thr Val Ser Gly Ser Thr Val Thr Val Lys Tyr Val
   260 265
<210> 58
   <211> 204
   <212> PRT
   <213> scytalidium lignicolum
<220>
   <221> MISC_FEATURE
   <222> (1)..(204)
   <223> scytalidoglutamic peptidase
<400> 58
   Thr Val Glu Ser Asn Trp Gly Gly Ala Ile Leu Ile Gly Ser Asp Phe
   1               5                   10                  15
   Asp Thr Val Ser Ala Thr Ala Asn Val Pro Ser Ala Thr Gly Ala Ser
               20                  25                  30
   Gly Gly Ser Ser Ala Ala Trp Val Gly Ile Asp Gly Asp Thr Cys Gln
           35                  40                  45
   Thr Ala Ile Leu Gln Thr Gly Phe Asp Trp Tyr Gly Asp Gly Thr Tyr
       50                  55                  60
   Asp Ala Trp Tyr Glu Trp Tyr Pro Glu Val Ser Asp Asp Phe Ser Gly
   65                  70                  75                  80
   Ile Thr Ile Ser Glu Gly Asp Ser Ile Gln Met Ser Val Thr Ala Thr
                   85                  90                  95
   Ser Asp Thr Ser Gly Ser Ala Thr Leu Glu Asn Leu Thr Thr Gly Gln
               100                 105                 110
   Lys Val Ser Lys Ser Phe Ser Asn Glu Ser Ser Gly Leu Cys Arg Thr
           115                 120                 125
   Asn Ala Glu Phe Ile Ile Glu Asp Phe Glu Glu Cys Asn Ser Asp Gly
       130                 135                 140
   Ser Asp Glu Phe Val Pro Phe Ala Ser Phe Ser Pro Ala Val Glu Phe
   145                 150                 155                 160
   Thr Asp Cys Ser Val Thr Ser Asp Gly Glu Ser Val Ser Leu Asp Asp
                   165                 170                 175
   Ala Gln Ile Thr Gln Val Ile Ile Asn Asn Gln Asp Val Thr Asp Cys
               180                 185                 190
   Ser Val Ser Gly Thr Thr Val Ser Cys Ser Tyr Val
           195                 200
<210> 59
   <211> 268
   <212> PRT
   <213> Cryphonectria parasitica
<220>
   <221> MISC_FEATURE
   <222> (1)..(268)
   <223> endopeptidase EapB
<400> 59
   Met Lys Tyr Thr Ala Ala Leu Ala Ala Leu Val Thr Leu Ala Ala Ala
   1 5 10 15
   Ala Pro Thr Asp Gly Ile Ile Asp Ile Gly Asp Gly Val Lys Leu Val
   20 25 30
   Pro Arg Glu Pro Arg Ala His Thr Arg Leu Glu Arg Leu Arg Thr Phe
   35 40 45
   Arg Arg Gly Leu Met Glu Gly Leu Glu Ser Gly Glu Arg Asn Ser Ser
   50 55 60
   Asp Val Ser Tyr Asp Ser Asn Trp Ala Gly Ala Val Lys Ile Gly Thr
   65 70 75 80
   Gly Leu Asn Asp Val Thr Gly Thr Ile Val Val Pro Thr Pro Ser Val
   85 90 95
   Pro Ser Gly Gly Ser Ser Thr Ala Lys Tyr Ala Ala Ser Ala Trp Val
   100 105 110
   Gly Ile Asp Gly Asp Thr Cys Thr Ser Ala Ile Leu Gln Thr Gly Val
   115 120 125
   Asp Phe Tyr Ala Gly Arg Gly Gly Val Ser Phe Asp Ala Trp Tyr Glu
   130 135 140
   Trp Tyr Pro Asn Tyr Ala Tyr Asp Phe Ser Gly Phe Ser Val Ser Ala
   145 150 155 160
   Gly Asp Thr Ile Val Met Thr Ala Ser Ala Ser Ser Leu Lys Ala Gly
   165 170 175
   Thr Val Thr Leu Glu Asn Ser Thr Thr Gly Lys Lys Val Thr Gln Ser
   180 185 190
   Phe Ser Ala Glu Ser Ser Glu Leu Cys Glu Tyr Asn Ala Glu Trp Ile
   195 200 205
   Val Glu Asp Phe Glu Ser Gly Ser Ser Leu Val Asn Phe Ala Asp Phe
   210 215 220
   Asp Thr Val Thr Phe Lys Asp Cys Ser Pro Ser Val Ser Gly Ser Thr
   225 230 235 240
   Ile Val Asp Ile Arg Gln Ser Leu Glu Val Leu Thr Glu Cys Ser Thr
   245 250 255
   Thr Gly Thr Thr Thr Val Thr Cys Glu Tyr Val Gly
   260 265
<210> 60
   <211> 147
   <212> PRT
   <213> Talaromyces emersonii
<220>
   <221> MISC_FEATURE
   <222> (1)..(147)
<400> 60
   Asn Trp Ala Gly Ala Val Leu Thr Ser Pro Pro Ser Gly Ser Thr Phe
   1               5                   10                  15
   Thr Ser Val Ser Ala Gln Phe Thr Val Pro Ser Pro Ser Leu Pro Gln
               20                  25                  30
   Gly Ser Gln Gln Ala Ser Ser Ala Ser Ala Trp Val Gly Ile Asp Gly
           35                  40                  45
   Asp Thr Tyr Thr Asn Ala Ile Leu Gln Thr Gly Val Asp Phe Asn Val
       50                  55                  60
   Asp Thr Asn Gly Gln Val Ser Tyr Asp Ala Trp Tyr Glu Trp Tyr Pro
   65                  70                  75                  80
   Asp Tyr Ala His Asp Phe Thr Gly Ile Ser Phe Gln Ser Gly Asp Val
                   85                  90                  95
   Val Ser Val Ser Val Thr Ser Ser Ser Asn Ser Glu Gly Thr Ala Val
               100                 105                 110
   Ile Glu Asn Leu Thr Asn Gly Gln Lys Val Thr Lys Thr Leu Ser Ala
           115                 120                 125
   Pro Ser Ser Ser Ala Thr Leu Gly Gly Gln Asn Ala Glu Trp Ile Val
       130                 135                 140
   Glu Asp Phe
   145

## Description

### FIELD OF THE INVENTION

The present invention relates to functional and operational polypeptides encoded by polynucleotides comprised in the genome of *Alicyclobacillus sp.* deposited under deposit accession number DSM 15716. The invention relates further to the polynucleotides and constructs of such polynucleotides encoding such polypeptides or facilitating their expression as well as to method for preparing the polypeptide. Still further the invention relates to compositions comprising the polypeptides and polynucleotides and to uses of the polypeptide. Still further the invention relates to a bacterium *Alicyclobacillus sp*. as deposited under accession number DSM 15716.

### BACKGROUND OF THE INVENTION

Some enzymes from the genus of *Alicyclobacillus species* are known such as described in Matzke et al.; Gene cloning, nucleotide sequence and biochemical properties of a cytoplasmic cyclomaltodextrinase (neopullulanase) from Alicyclobacillus acidocaldarius ATCC 2700; reclassification of a group of enzymes; Submitted (MAR-1999) to the EMBL/GenBank/DDBJ databases or Koivula et al.; Cloning and sequencing of a gene encoding acidophilic amylase from Bacillus acidocaldarius. J. Gen. Microbiol. 139:2399 (1993) or Bartolucci et al.; Thioredoxin from Bacillus acidocaldarius: characterization, high-level expression in Escherichia coli and molecular modeling; Biochem. J. 328:277 (1997) or Tsuruoka et al.; Collagenolytic Serine-Carboxyl Proteinase from Alicyclobacillus sendainensis Strain NTAP-1: Purification, Characterization, Gene Cloning, and Heterologous Expression; Submitted (MAY-2002) to the EMBL/GenBank/DDBJ databases; Eckert K. & Schneider E., A thermoacidophilic endoglucanase (celB) from Alicyclobacillus acidocaldarius displays high sequence similarity to arabinofuranosidases belonging to family 51 of glycosyl hydrolases; Eur. J. Biochem., 270: 3593-3602, 2003.

In the pursuit of novel enzymes it is also known to screen for such new enzymes by subjecting potential candidates to specific enzyme assays. This approach is limited to the availability of enzyme assays and does not allow the identification of functional enzymes or polypeptides for which the activity is still unknown.

Further, whole genome sequencing is a known method to obtain the information on all genes from a given microorganism e.g. as described in Fleischmann et al.; Whole genome sequences and assembly of Haemophilus influenzae Rd; Nature 269: 496- 512; (1995).

Most enzymes for industrial use are enzymes which are secreted to the medium by a microorganism. However, only a few percent of a microorganisms' genome encodes secreted proteins. For example only approx. 4% of the *Bacillus subtilis* genome or its closest relatives encode secreted proteins (Van Dijl et al.: Protein transport pathways in Bacillus subtilis: a genome-based road map; in "Bacillus subtilis and its closest relatives" - From genes to cells; p.337-355; A. L. Sonenshein (ed.); ASM Press 2002).

One disadvantage of genome sequencing is that the vast majority of the obtained sequences encode non secreted proteins.

Jara P. et al. Cloning and characterization of the eapB and eapC genes of Cryoptonectria parasitica encoding two new acid proteinases, and disruption of eapC. Mol. Gen. Genet. 250: 97-105, discloses the isolation of two new genes encoding acid proteinases from the ascomycete fungus *Cryptonectria parasitica.*

Poussereau N. et al. Regulation of acp1, encoding a non-aspartyl acid protease expressed during pathogenisis of Sclerotina sclerotinicum. Microbiology 147: 717-726, discloses the isolation of a gene encoding an acid protease expressed during pathogenisis by the necrotrophic fungus *Sclerotina scleroticum.*

Sims, A. H. et al. Glutamic protease distribution is limited to filamentuous fungi. FEMS Microbiology Letters 239: 95-101 identified and analysed the distribution of over 20 putative glutamic proteases from all fungal species whose genome had been sequences so far. They concluded that although absent in the Saccharomycetales class, glutamic proteases appear to be present in all other ascomycetes species examined. Further it is mentioned that this class of peptidases are thought to be found only in fungi.

Also known is signal trapping which is a method to identify genes including nucleotides encoding a signal peptide using a translational fusion to an extra cellular reporter gene lacking its own signal (WO 01/77315).

### SUMMARY OF THE INVENTION

The present inventors have found a strain of Alicyclobacillus namely Alicyclobacillus sp. DSM 15716 which grows at low pH (approx 4-5) and at high temperature (50-60 °C). This strain is interesting because the phylogenetic distance between the public known strains and strain DSM 15716 is significant and because the growth conditions are similar to conditions for several applications for industrial enzymes.

The genome of a microorganism contains thousands of different genes; some encoding polypeptides some coding for RNAs. Only a limited number of the genes in the genome of a microorganism encode functional polypeptides which are secreted by the microorganism to the surrounding medium serving an external purpose for the microorganism. Such polypeptides are interesting for industry from the point of view that such polypeptides may be produced in considerable amounts in continuous processes without destroying the cells producing the polypeptides.

It is an object of the present invention to identify and provide polypeptides secreted from *Alicyclobacillus sp.* deposited under deposit accession number DSM 15716 which have functional purpose for the *Alicyclobacillus sp.* because such polypeptides may not only be used for industrial purposes but they may also be produced in industrially relevant processes and amounts. In a first aspect the invention provides a polypeptide comprising an amino acid sequence which has at least 90% identity with the sequence of SEQ ID NO: 27, wherein the polypeptide has glutamic peptidase activity.

In further aspects the invention provides a polynucleotide encoding the polypeptide of the invention; a nucleotide construct comprising the polynucleotide encoding the polypeptide, operably linked to one or more control sequences that direct the production of the polypeptide in a host cell; a recombinant expression vector comprising the nucleotide construct of the invention and to a recombinant host cell comprising the nucleotide construct of the invention.

In still further aspects the invention provides a method of preparing a polypeptide of the invention comprising:
(a) cultivating a strain comprising a nucleotide sequence encoding a polypeptide of the invention which strain is capable of expressing and secreting the polypeptide and
(b) recovering the polypeptide.

In a further aspect the invention provides a composition comprising a polypeptide of the invention and a method for preparing such a composition comprising admixing the polypeptide of the invention with an excipient.

In a further aspect the invention provides a composition comprising a polynucleotide of the invention and a method for preparing such a composition comprising admixing the polynucleotide of the invention with an excipient.

In further aspects the invention provides use of the polypeptide of the invention or a composition comprising said polypeptide in various applications.

In a further aspect the invention relates to a bacterium *Alicyclobacillus sp.* as deposited under accession number DSM 15716.

### SEQUENCE LISTING

The regions of SEQ ID NO: 1 to SEQ ID NO: 25 encoding a mature polypeptide encode the mature polypeptides of SEQ ID NO: 26 to SEQ ID NO: 50. The region of SEQ ID NO: 1 encoding a mature polypeptide thus encodes the mature polypeptide sequence comprised in SEQ ID NO: 26, the region of SEQ ID NO: 2 encoding a mature polypeptide encode the mature polypeptide comprised in SEQ ID NO: 27 and so on.

### DETAILED DESCRIPTION OF THE INVENTION

### Definitions

The term **"identity"** as used herein, is to be understood as the homology between two amino acid sequences or between two nucleotide sequences. For purposes of the present invention, the degree of identity between two amino acid sequences was determined by using AlignX in the program of Vector NTI ver. 7.1 (Informax inc., 7600 Wisconsin Avenue, Suite #1100, Bethesda, MD 20814, USA). Amino acid alignment was created using the Clustal W algorithm (Nucleic Acid Research, 22 (22): 4673-4680, 1994). The following additional parameters are used: Gap opening penalty of 10, Gap extension penalty of 0.05, Gap separation penalty range of 8. Pairwise alignment parameters were Ktuple = 1, gap penalty = 3, gap length opening penalty = 10, gap extension penalty = 0.1, window size = 5 and diagonals = 5. The degree of identity between two nucleotide sequences is determined using the same algorithm and software package as described above for example with the following settings: Gap penalty of 10, and gap length penalty of 10. Pairwise alignment parameters is Ktuple=3, gap penalty=3 and windows=20.

The term **"functional polypeptide"** as used herein in the context of the present invention means a polypeptide which can be expressed and secreted by a cell and which constitutes an operational unit capable of operating in accordance with the function it is designed to fulfil by the cell. Optionally, co-factors may be required for the polypeptide to adopt the intended function. One example of functional polypeptides is catalytically active polypeptides or enzymes which help the cell catalyzing reactions in the environment surrounding the cell. Another example could be polypeptides which serve as signal substance. Further examples are polypeptides which function as sensors (receptors) for environmental parameters (chemicals in the environment surrounding the cell) or polypeptides, which are active against other organisms (antimicrobial (poly)peptides) or polypeptides, which contributes to the structural integrity of the cell.

The term **"mature region"** as used herein about portion of an amino acid sequences or polypeptide means the portion or region or domain or section of the amino acid sequences or polypeptide which is the mature functional polypeptide.

The term **"region of nucleotide sequence encoding a mature polypeptide"** as used herein means the region of a nucleotide sequence counting from the triplet encoding the first amino acid of a mature polypeptide to the last triplet encoding the last amino acid of a mature polypeptide.

The term **"secreted polypeptide"** as used herein is to be understood as a polypeptide which after expression in a cell is either transported to and released to the surrounding extracellular medium or is associated/embedded in the cellular membrane so that at least a part of the polypeptide is exposed to the surrounding extracellular medium.

### Polypeptides of the invention

The present invention relates to an isolated mature functional polypeptide which is at least 90 % identical to and exhibits glutamic peptidase activity of a corresponding secreted polypeptide having the sequence disclosed in SEQ ID NO: 27 and obtainable from the bacterium Alicyclobacillus sp. deposited under accession number DSM 15716.

Moreover, surprisingly the glutamic peptidase of SEQ ID NO: 27 expressed by the Alicyclobacillus sp. DSM 15716, is the first glutamic peptidase ever to have been isolated from a bacterium. Hence, the invention also provides a bacterial glutamic peptidase (EC 3.4.23.19)

Polypeptides of the invention are, in particular, secreted by *Alicyclobacillus sp.* DSM 15716 with the purpose of serving a function for that particular cell.

Among the thousands of potential genes in the genome of *Alicyclobacillus sp.* DSM 15716 the polynucleotides of this genome encoded 25 secreted functional mature polypeptides comprised in SEQ ID NO: 26 to SEQ ID NO: 50, which were determined to be functional, that is translated into functional polypeptides by the chosen host cell.

Accordingly, *Alicyclobacillus sp.* DSM 15716 expresses and secretes the functional mature polypeptides comprised in SEQ ID NO: 26 to SEQ NO: 50 and in the genome of that particular strain, the regions of SEQ ID NO: 1 to SEQ ID NO: 25 encoding a mature polypeptide are the genes encoding the mature polypeptides comprised in SEQ ID NO: 26 to SEQ NO: 50. Further, the genes encoding the mature polypeptides comprised in of SEQ ID NO: 26 to SEQ NO: 50 can all be expressed and their corresponding mature polypeptides can be secreted when culturing an E. coli host transformed with polynucleotides comprising those regions of SEQ ID NO: 1 to SEQ ID NO: 25 encoding a mature polypeptide. By comparing homology or identity of the sequences of the 25 polypeptide sequences to known sequences the particular function of the polypeptides were annotated. At least 15 of the 25 secreted functional polypeptides were determined to be enzymes.

Disclosed here with are isolated polypeptides selected from the group consisting of:
(a) a polypeptide having an amino acid sequence which has at least 90% identity with an amino acid sequence selected from the group consisting of the mature polypeptides comprised in SEQ ID NO: 26 to SEQ ID NO: 50 and
(b) a polypeptide which is encoded by a nucleotide sequence which hybridize under high stringency conditions with a polynucleotide probe selected from the group consisting of
   (i) the complementary strand to a nucleotide sequence selected from the group consisting of regions of SEQ ID NO: 1 to SEQ ID NO: 25 encoding a mature polypeptide,
   (ii) the complementary strand to the cDNA sequence contained in a nucleotide sequences selected from regions of SEQ ID NO: 1 to SEQ ID NO: 25 encoding a mature polypeptide;
wherein the polypeptide exhibits the function of the corresponding mature polypeptide of SEQ ID NO: 26 to SEQ ID NO: 50.

The polypeptide of the invention is an isolated polypeptide, preferably the preparation of the polypeptide of the invention contains at the most 90% by weight of other polypeptide material with which it may be natively associated (lower percentages of other polypeptide material are preferred, e.g. at the most 80% by weight, at the most 60% by weight, at the most 50% by weight, at the most 40% at the most 30% by weight, at the most 20% by weight, at the most 10% by weight, at the most 9% by weight ,at the most 8% by weight, at the most 6% by weight, at the most 5% by weight, at the most 4% at the most 3% by weight, at the most 2% by weight, at the most 1% by weight and at the most ½% by weight). Thus, it is preferred that the isolated polypeptide of the invention is at least 92% pure, i.e. that the polypeptide of the invention constitutes at least 92% by weight of the total polypeptide material present in the preparation, and higher percentages are preferred such as at least 94% pure, at least 95% pure, at least 96% pure, at least 96% pure, at least 97% pure, at least 98% pure, at least 99%, and at the most 99.5% pure. In particular, it is preferred that the polypeptide of the invention is in "essentially pure form", i.e. that the polypeptide preparation is essentially free of other polypeptide material with which it is natively associated. This can be accomplished, for example, by preparing the polypeptide of the invention by means of well-known recombinant methods.

The polypeptide of the invention of the invention may be synthetically made, naturally occurring or a combination thereof. In a particular embodiment the polypeptide of the invention may be obtained from a microorganism such as a prokaryotic cell, an archaeal cell or a eukaryotic cell. The cell may further have been modified by genetic engineering

In a particular embodiment, the polypeptide of the invention is an enzyme exhibiting optimum enzyme activity at a temperature within the range from about 10°C to about 80°C, particularly in the range from about 20°C to about 60°C.

In a particular embodiment the polypeptide of the invention is an enzyme, which is functionally stabile at a temperature of up to 100°C, in particular up to 80°C, more particularly up to 60°C.

In a particular embodiment the polypeptide of the invention is an enzyme exhibiting at least 20%, in particular at least 40%, such as at least 50%, in particular at least 60%, such as at least 70%, more particularly at least 80%, such as at least 90%, most particularly at least 95%, such as about or at least 100% of the enzyme activity of an enzyme selected from mature enzymes comprised in SEQ ID NO: 27.

In particular the isolated mature functional polypeptide is at least 90 % identical to and exhibits the same function of the corresponding secreted polypeptide obtainable from the bacterium Alicyclobacillus sp. deposited under accession number DSM 15716 and specically the polypeptide of the invention comprises, contains or consists of an amino acid sequence which has at least 90% identity with the polypeptide sequence in SEQ ID NO: 27. The percent identity is particularly at least 95%, e.g. at least 96%, such as at least 97%, and even more particularly at least 98%, such as at least 99% or even 100% identity.

In a particular embodiment, the amino acid sequence of the polypeptide of the invention differs by at the most ten amino acids (e.g. by ten amino acids), in particular by at the most five amino acids (e.g. by five amino acids), such as by at the most four amino acids (e.g. by four amino acids), e.g. by at the most three amino acids (e.g. by three amino acids), in particular by at the most two amino acids (e.g. by two amino acids), such as by one amino acid from the mature polypeptide comprised in SEQ ID NO: 27.

The polypeptide of the invention may be a wild-type polypeptide isolated from a natural source such as the strain *Alicyclobacillus sp.* DSM 15716 or another wild type strain, however the present invention also encompass artificial variants, where a polypeptide of the invention has been mutated for example by adding, substituting and/or deleting one or more amino acids from said polypeptide while retaining the function of the polypeptide and/or other properties. Hence, the polypeptide of the invention may be an artificial variant, wherein at least one substitution, deletion and/or insertion of an amino acid has been made to an amino acid sequence comprising or consisting of the mature polypeptide comprised in SEQ ID NO: 27.

Artificial variants may be constructed by standard techniques known in the art usually followed by screening and/or characterization. Standard techniques includes classical mutagenesis, e.g. by UV irradiation of the cells or treatment of cells with chemical mutagens as described by Gerhardt et al. (1994); in vivo gene shuffling as described in WO 97/07205; in vitro shuffling as described by Stemmer, (1994) or WO 95/17413, random mutagenesis as described by Eisenstadt E. et al., (1994); PCR techniques as described by Poulsen et al. (1991); family shuffling as described by J.E. Ness, et al, Nature Biotechnology, vol. 17, pp. 893-896 (1999); site-directed mutagenesis as described by Sambrook et al. (1989), Sambrook et al., Molecular Cloning, A Laboratory Manual, Cold Spring Harbor, NY. A general description of nucleotide substitution can be found in e.g. Ford et al., 1991, Protein Expression and Purification 2, p. 95-107.

Such standard genetic engineering methods may also be used prepare a diversified library of variant nucleotide sequences from the genes encoding one or more parent enzymes of the invention, expressing the enzyme variants in a suitable host cell and selecting a preferred variant(s). A diversified library can be established by a range of techniques known to the art (Reetz MT; Jaeger KE, in Biocatalysis - from Discovery to Application edited by Fessner WD, Vol. 200, pp. 31-57 (1999); Stemmer, Nature, vol. 370, p.389-391, 1994; Zhao and Arnold, Proc. Natl. Acad. Sci., USA, vol. 94, pp. 7997-8000, 1997; or Yano et al., Proc. Natl. Acad. Sci., USA, vol. 95, pp 5511-5515, 1998).

In a particular embodiment of the invention, amino acid changes (in the artificial variant as well as in wild-type enzyme) are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine and threonine). Amino acid substitutions which do not generally alter and or impair the function of a protein are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

In a particular embodiment the amino acid changes are of such a nature that the physico-chemical properties of the polypeptides are altered. For example, amino acid changes may be performed, which improve the thermal stability of the enzyme, which alter the substrate specificity, which changes the pH optimum, and the like.

Particularly, the number of such substitutions, deletions and/or insertions in the polypeptide of the invention, particularly in those polypeptides selected from the group consisting of mature polypeptides comprised in SEQ ID NO: 27 to produce an artificial variant is at the most 10, such as at the most 9, e.g. at the most 8, more preferably at the most 7, e.g. at the most 6, such as at the most 5, most preferably at the most 4, e.g. at the most 3, such as at the most 2, in particular at the most 1.

In a particular embodiment the artificial variant is a variant, which has an altered, preferably reduced, immunogenicity, especially allergenicity, in animals including man as compared to a parent enzyme. The term "immunogenicity" in this context is to be understood as the artificial variant capability of invoking a an altered, in particular reduced, immunological response when administered to an animal, including intravenous, cutaneous, subcutaneous, oral and intratracheal administration. The term "immunological response" in this context means that the administration of the artificial variant causes an alteration in the immunoglobulin levels in the animal body, such as in IgE, IgG and IgM or an alteration in the cytokine level in the animal body. Methods for mapping immunogenic/antigenic epitopes of a protein, preparing variants with altered immunogenicity and methods for measuring an immunological response is well known to the art and are described e.g. in WO 92/10755, WO 00/26230, WO 00/26354 and WO 01/31989. The term "allergenicity" in this context is to be understood as the artificial variant ability of invoking an altered, in particular reduced, production of IgE in an animal as well as the ability to bind IgE from said animal. Particularly allergenicity arising from intratracheal administration of the polypeptide variant to the animal is particularly of interest (also known as respiratory allergenicity).

In particular, the polypeptide of the invention is encoded by a polynucleotide comprising a nucleotide sequence selected from the group of regions of SEQ ID NO: 2 encoding a mature polypeptide or a sequences differing there from by virtue of the degeneracy of the genetic code. More particularly, the polypeptide of the invention is encoded by a polynucleotide consisting of a nucleotide sequence selected from the group of regions of SEQ ID NO: 2 encoding a mature polypeptide or a sequence differing there from by virtue of the degeneracy of the genetic code.

The nucleotide sequences of regions of SEQ ID NO: 2 encoding a mature polypeptide or a subsequence thereof, as well as the amino acid sequences of the mature polypeptides comprised in SEQ ID NO: 27 or a fragment thereof, may be used to design a polynucleotide probe to identify and clone DNA encoding enzymes of the invention from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, more preferably at least 35 nucleotides in length, such as at least 70 nucleotides in length. It is; however, preferred that the polynucleotide probe is at least 100 nucleotides in length. For example, the polynucleotide probe may be at least 200 nucleotides in length, at least 300 nucleotides in length, at least 400 nucleotides in length or at least 500 nucleotides in length. Even longer probes may be used, e.g., polynucleotide probes which are at least 600 nucleotides in length, at least 700 nucleotides in length, at least 800 nucleotides in length, or at least 900 nucleotides in length. Both DNA and RNA probes can be used. The probes are typically labelled for detecting the corresponding gene (for example, with ³²P, ³H, ³⁵S, biotin, or avidin).

Thus, a genomic DNA or cDNA library prepared from such other organisms may be screened for DNA, which hybridizes with the probes described above and which encodes enzymes of the invention. Genomic or other DNA from such other organisms may be separated by agarose or polyacrylamide gel electrophoresis, or other separation techniques. DNA from the libraries or the separated DNA may be transferred to, and immobilized, on nitrocellulose or other suitable carrier materials. In order to identify a clone or DNA which has the required homology and/or identity or is homologous and/or identical with of nucleotides selected from regions of SEQ ID NO: 2 encoding a mature polypeptide, the carrier material with the immobilized DNA is used in a Southern blot.

For purposes of the present invention, hybridization indicates that the nucleotide sequence hybridizes to a labelled polynucleotide probe which again hybridizes to a nucleotide sequence selected from regions of SEQ ID NO: 2 encoding a mature polypeptide under high to very high stringency conditions. Molecules to which the polynucleotide probe hybridizes under these conditions may be detected using X-ray film or by any other method known in the art. Whenever the term "polynucleotide probe" is used in the present context, it is to be understood that such a probe contains at least 15 nucleotides.

In an interesting embodiment, the polynucleotide probe is the complementary strand of a nucleotide sequence selected from regions of SEQ ID NO: 2 encoding a mature polypeptide.

In another interesting embodiment, the polynucleotide probe is the complementary strand of a nucleotide sequence which encodes an enzyme selected from the group of SEQ ID NO: 27. In a further interesting embodiment, the polynucleotide probe is the complementary strand of a mature polypeptide coding region of a nucleotide sequence selected from regions of SEQ ID NO: 2 encoding a mature polypeptide.

For long probes of at least 100 nucleotides in length, high to very high stringency conditions are defined as pre-hybridization and hybridization at 42°C in 5X SSPE, 1.0% SDS, 5X Denhardt's solution, 100 microgram/ml sheared and denatured salmon sperm DNA, following standard Southern blotting procedures. Preferably, the long probes of at least 100 nucleotides do not contain more than 1000 nucleotides. For long probes of at least 100 nucleotides in length, the carrier material is finally washed three times each for 15 minutes using 0.1 x SSC, 0.1% SDS at 60°C (high stringency), in particular washed three times each for 15 minutes using 0.1 x SSC, 0.1% SDS at 68°C (very high stringency).

Although not particularly preferred, it is contemplated that shorter probes, e.g. probes which are from about 15 to 99 nucleotides in length, such as from about 15 to about 70 nucleotides in length, may be also be used. For such short probes, stringency conditions are defined as pre-hybridization, hybridization, and washing post-hybridization at 5°C to 10°C below the calculated Tm using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to 99 nucleotides in length, the carrier material is washed once in 6X SCC plus 0.1% SDS for 15 minutes and twice each for 15 minutes using 6X SSC at 5°C to 10°C below the calculated Tm.

### SEQ ID NO: 27 glutamic peptidase

The polypeptide of the invention is a glutamic peptidase comprising or consisting of an amino acid sequence which has at least 90%, particularly at least 95%, more particularly at least 96%, more particularly at least 97%, more particularly at least 98%, more particularly at least 99% or most particularly 100% identity with the glutamic peptidase obtainable from Alicyclobacillus sp.asd having the sequence of SEQ ID NO: 27. More specifically the mature glutamic peptidase comprises or consists of the sequences from position 1 - 240 of SEQ ID NO: 27. In the present context a glutamic peptidase is defined as an enzyme, which hydrolyses proteins or peptides, and which contains conserved active site residues Q and E.

The glutamic peptidase (PepG) (EC 3.4.23.19) was previously categorized as an aspartyl protease (A4) but was reclassified by MEROPS (http://merops.sanger.ac.uk/), which published that "As a result of the exciting paper of Fujinaga, Cherney, Oyama, Oda & James (2004), **The molecular structure and catalytic mechanism of a novel carboxyl peptidase from *Scytalidium lignicolum.* PubMed**, we now recognise a sixth catalytic type of peptidases: the glutamic peptidases. The known glutamic peptidases are all contained in the the family that was formerly A4, and now becomes G1." (Fujinaga M, Cherney MM, Oyama H, Oda K, James MN.; The molecular structure and catalytic mechanism of a novel carboxyl peptidase from Scytalidium lignicolum; Proc. Natl. Acad. Sci. U. S. A.; 101(10); pp. 3364-9; Epub 01-Mar-2004; 09-Mar-2004.)

That the polypeptide of SEQ ID NO: 27 is a glutamic peptidase also appears from the following multiple sequence alignment confirming that active site residues Q and E are conserved in SEQ ID NO: 27:
CLUSTAL W (1.81) multiple sequence alignment

Hence, the present inventors has identified and isolated the first (G1) glutamic peptidase from a Bacterium ever known, and in particular one that is active at low pH and high temp. The closest relatives are fungal G1 proteases (e.g. Aspergillopepsin II).

Furthermore, surprisingly this glutamic peptidase differs from most known fungal glutamic peptidases by the absence of disulphide bridges in the molecule. The glutamic peptidase comprised in SEQ ID NO: 27 contains only one Cystein and thus no disulphide bridges in the protease structure as compared to e.g. SEQ ID NO: 55 disclosing a known fungal glutamic peptidase, which are composed of two peptides cross linked by 2 disulphide bridges. Hence, the glutamic peptidase of *Alicyclobacillus sp.* specifically that deposited under DSM accession No. 15716 a second propeptide is missing and thus requires one less maturation step less in its production. This is an advantage for the cellular production.

### Polynucleotides

The present invention also relates to polynucleotides, particularly isolated polynucleotides, comprising or consisting of a nucleotide sequence encoding a polypeptide of the invention. In a particular embodiment, the nucleotide sequence is set forth in SEQ ID NO: 2 including nucleotide sequences differing there from by virtue of the degeneracy of the genetic code. In a further embodiment the polynucleotide of the invention is a modified nucleotide sequence which comprises or consists of a nucleotide sequence selected from SEQ ID NO: 2 and encoding a mature polypeptide and which comprises at least one modification/mutation compared with the parent nucleotide sequence comprised in SEQ ID NO: 2.

The techniques used to isolate and/or clone a nucleotide sequence encoding an enzyme are known in the art and include isolation from genomic DNA, preparation from cDNA, or a combination thereof. The cloning of the nucleotide sequences of the present invention from such genomic DNA can be effected, *e.g*., by using the well known polymerase chain reaction (PCR) or antibody screening of expression libraries to detect cloned DNA fragments with shared structural features. See, *e.g*., Innis et al., 1990, PCR: A Guide to Methods and Application, Academic Press, New York. Other amplification procedures such as ligase chain reaction (LCR), ligated activated transcription (LAT) and nucleotide sequence-based amplification (NASBA) may be used.

The nucleotide sequence may be obtained by standard cloning procedures used in genetic engineering to relocate the nucleotide sequence from its natural location to a different site where it will be reproduced. The cloning procedures may involve excision and isolation of a desired fragment comprising the nucleotide sequence encoding the polypeptide, insertion of the fragment into a vector molecule, and incorporation of the recombinant vector into a host cell where multiple copies or clones of the nucleotide sequence will be replicated. The nucleotide sequence may be of genomic, cDNA, RNA, semi-synthetic, synthetic origin, or any combinations thereof.

In particular the polynucleotide comprises, preferably consists of, a nucleotide sequence which has at least 50% identity with a nucleotide sequence selected from SEQ ID NO: 2 and encoding a mature polypeptide. Particularly, the nucleotide sequence has at least 65% identity, more particularly at least 70% identity, more particularly at least 80% identity, more particularly at least 90% identity, more particularly at least 95% identity, more particularly at least 96% identity, more particularly at least 97% identity, more particularly at least 98% identity, more particularly at least 99% identity or most particularly 100% identity with a nucleotide sequence selected from SEQ ID NO: 2 and encoding a mature polypeptide. Particularly, the nucleotide sequence comprises a nucleotide sequence selected from SEQ ID NO: 2 and encoding a mature polypeptide. In an even more particular embodiment, the nucleotide sequence consists of a nucleotide sequence selected from SEQ ID NO: 2 and encoding a mature polypeptide.

In particular the polynucleotide comprises, preferably consists of, a nucleotide sequence encoding a mature enzyme being a glutamic peptidase and which has at least 50% identity, particularly at least 65% identity, more particularly at least 70% identity, more particularly at least 80% identity, more particularly at least 90% identity, more particularly at least 95% identity, more particularly at least 96% identity, more particularly at least 97% identity, more particularly at least 98% identity, more particularly at least 99% identity or most particularly 100% identity with a nucleotide sequence encoding a mature enzyme being a glutamic peptidase secreted from the strain of *Alicyclobacillus sp.* Deposited under DSM accession No. 15716.

### SEQ ID NO: 2

The polynucleotide of the invention encodes a glutamic peptidase.

Modification of a nucleotide sequence encoding a polypeptide of the present invention may be necessary for the synthesis of a polypeptide which comprises an amino acid sequence that has at least one substitution, deletion and/or insertion as compared to an amino acid sequence selected from mature polypeptide comprised in SEQ ID NO: 27.

It will be apparent to those skilled in the art that such modifications can be made to preserve the function of the enzyme i.e. made outside regions critical to the function of the enzyme. Amino acid residues which are essential to the function are therefore preferably not subject to modification, such as substitution. Amino acid residues essential to the function may be identified according to procedures known in the art, such as site-directed mutagenesis or alanine-scanning mutagenesis (see, *e.g.,* Cunningham and Wells, 1989, Science 244: 1081-1085). Sites of substrate-enzyme interaction can be determined by analysis of the three-dimensional structure as determined by such techniques as nuclear magnetic resonance analysis, crystallography or photoaffinity labeling (see, *e.g.,* de Vos et al., 1992, Science 255: 306-312; Smith et al., 1992, Journal of Molecular Biology 224: 899-904; Wlodaver et al., 1992, FEBS Letters 309: 59-64).

Moreover, a nucleotide sequence encoding an enzyme of the invention may be modified by introduction of nucleotide substitutions which do not give rise to another amino acid sequence of the enzyme encoded by the nucleotide sequence, but which correspond to the codon usage of the host organism intended for production of the enzyme.

The introduction of a mutation into the nucleotide sequence to exchange one nucleotide for another nucleotide may be accomplished by site-directed mutagenesis using any of the methods known in the art. Particularly useful is the procedure, which utilizes a super coiled, double stranded DNA vector with an insert of interest and two synthetic primers containing the desired mutation. The oligonucleotide primers, each complementary to opposite strands of the vector, extend during temperature cycling by means of *Pfu* DNA polymerase. On incorporation of the primers, a mutated plasmid containing staggered nicks is generated. Following temperature cycling, the product is treated with *Dpn*I*,* which is specific for methylated and hemimethylated DNA to digest the parental DNA template and to select for mutation-containing synthesized DNA. Other procedures known in the art may also be used. For a general description of nucleotide substitution, one may consult with *e.g.,* Ford et al., 1991, Protein Expression and Purification 2: 95-107.

The present invention also relates to a polynucleotide comprising, preferably consisting of, a nucleotide sequence which encodes a polypeptide of the invention.

Disclosed is also encompasses a storage medium suitable for use in an electronic, preferably digital, device comprising information of the amino acid sequence of polypeptides of the invention or the nucleotide sequences of the polynucleotide of the invention, in particular any af the polypeptide or polynucleotide sequences of the invention in an electronic or digital form, such as binary code or other digital code. The storage medium may suitably be a magnetic or optical disk and the electronic device a computing device and the information may in particular be stored on the storage medium in a digital form.

### Nucleotide constructs

The present invention also relates to nucleic acid constructs comprising a nucleotide sequence of the invention operably linked to one or more control sequences that direct the expression of the coding sequence in a suitable host cell under conditions compatible with the control sequences.

A nucleotide sequence encoding an enzyme of the invention may be manipulated in a variety of ways to provide for expression of the enzyme. Manipulation of the nucleotide sequence prior to its insertion into a vector may be desirable or necessary depending on the expression vector. The techniques for modifying nucleotide sequences utilizing recombinant DNA methods are well known in the art.

The control sequence may be an appropriate promoter sequence, a nucleotide sequence that is recognized by a host cell for expression of the nucleotide sequence. The promoter sequence contains transcriptional control sequences, which mediate the expression of the polypeptide. The promoter may be any nucleotide sequence which shows transcriptional activity in the host cell of choice including mutant, truncated, and hybrid promoters, and may be obtained from genes encoding extra cellular or intracellular polypeptides either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*)*, Bacillus licheniformis* alpha-amylase gene *(amyL), Bacillus stearothermophilus* maltogenic amylase gene (*amyM*), *Bacillus amyloliquefaciens* alpha-amylase gene (*amyQ*), *Bacillus licheniformis* penicillinase gene (*penP*), *Bacillus subtilis xylA* and *xylB* genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook *et al.,* 1989, *supra.*

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, and *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase), and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH2/GAP), and *Saccharomyces cerevisiae* 3-phosphoglycerate kinase. Other useful promoters for yeast host cells are described by Romanos et al., 1992, Yeast 8: 423-488.

The control sequence may also be a suitable transcription terminator sequence, a sequence recognized by a host cell to terminate transcription. The terminator sequence is operably linked to the 3' terminus of the nucleotide sequence encoding the enzyme. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be a suitable leader sequence, a non-translated region of an mRNA which is important for translation by the host cell. The leader sequence is operably linked to the 5' terminus of the nucleotide sequence encoding the polypeptide. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995, Molecular Cellular Biology 15: 5983-5990.

The control sequence may also be a signal peptide coding region that codes for an amino acid sequence linked to the amino terminus of a polypeptide and directs the encoded enzyme into the cell's secretory pathway. The 5' end of the coding sequence of the nucleotide sequence may inherently contain a signal peptide coding region naturally linked in translation reading frame with the segment of the coding region which encodes the secreted enzyme. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the enzyme. However, any signal peptide coding region which directs the expressed enzyme into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT, nprS, nprM*)*,* and *Bacillus subtilis prsA.* Further signal peptides are described by Simonen and Palva, 1993, Microbiological Reviews 57: 109-137.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding regions are described by Romanos *et al.,* 1992, *supra.*

The control sequence may also be a propeptide coding region that codes for an amino acid sequence positioned at the amino terminus of a enzyme. The resultant polypeptide may be denoted a pro-enzyme or propolypeptide. A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*), *Bacillus subtilis* neutral protease (*nprT*), *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences.

Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleotide sequence encoding the polypeptide would be operably linked with the regulatory sequence.

### Recombinant expression vectors

The present invention also relates to recombinant expression vectors comprising the nucleic acid construct of the invention. The various nucleotide and control sequences described above may be joined together to produce a recombinant expression vector, which may include one or more convenient restriction sites to allow for insertion or substitution of the nucleotide sequence encoding the polypeptide at such sites. Alternatively, the nucleotide sequence of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g*., a plasmid or virus) that can be conveniently subjected to recombinant DNA procedures and can bring about the expression of the nucleotide sequence. The choice of the vector will typically depend on the compatibility of the vector with the host cell into which the vector is to be introduced. The vectors may be linear or closed circular plasmids.

The vector may be an autonomously replicating vector, *i.e.,* a vector that exists as an extrachromosomal entity, the replication of which is independent of chromosomal replication, *e.g*., a plasmid, an extrachromosomal element, a minichromosome, or an artificial chromosome.

The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers that permit easy selection of transformed cells. A selectable marker is a gene the product of which provides for biocide or viral resistance, resistance to heavy metals, prototrophy to auxotrophs, and the like.

Examples of bacterial selectable markers are the *dal* genes from *Bacillus subtilis* or *Bacillus licheniformis,* or markers that confer antibiotic resistance such as ampicillin, kanamycin, chloramphenicol or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits stable integration of the vector into the host cell's genome or autonomous replication of the vector in the cell independent of the genome.

For integration into the host cell genome, the vector may rely on the nucleotide sequence encoding the polypeptide or any other element of the vector for stable integration of the vector into the genome by homologous or nonhomologous recombination. Alternatively, the vector may contain additional nucleotide sequences for directing integration by homologous recombination into the genome of the host cell. The additional nucleotide sequences enable the vector to be integrated into the host cell genome at a precise location(s) in the chromosome(s). To increase the likelihood of integration at a precise location, the integrational elements should preferably contain a sufficient number of nucleotides, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.* Examples of origins of replication for use in a yeast host cell are the 2 micron origin of replication, ARS1, ARS4, the combination of ARS1 and CEN3, and the combination of ARS4 and CEN6.

The origin of replication may be one having a mutation which makes its functioning temperature-sensitive in the host cell (see, *e.g.,* Ehrlich, 1978, Proceedings of the National Academy of Sciences USA 75: 1433).

More than one copy of a nucleotide sequence of the present invention may be inserted into the host cell to increase production of the gene product. An increase in the copy number of the nucleotide sequence can be obtained by integrating at least one additional copy of the sequence into the host cell genome or by including an amplifiable selectable marker gene with the nucleotide sequence where cells containing amplified copies of the selectable marker gene, and thereby additional copies of the nucleotide sequence, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra*).

### Recombinant host cells

The present invention also relates to recombinant a host cell comprising the nucleic acid construct of the invention, which are advantageously used in the recombinant production of the polypeptides. A vector comprising a nucleotide sequence of the present invention is introduced into a host cell so that the vector is maintained as a chromosomal integrant or as a self-replicating extra-chromosomal vector as described earlier.

The host cell may be a unicellular microorganism, *e.g.,* a prokaryote or a non-unicellular microorganism, *e.g.,* a eukaryote.

Useful unicellular cells are bacterial cells such as gram positive bacteria including, but not limited to, a *Bacillus* cell, *e.g., Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a *Streptomyces* cell, *e.g., Streptomyces lividans* or *Streptomyces murinus,* or gram negative bacteria such as *E*. *coli* and *Pseudomonas* sp. In a preferred embodiment, the bacterial host cell is a *Bacillus lentus, Bacillus licheniformis, Bacillus stearothermophilus,* or *Bacillus subtilis* cell. In another preferred embodiment, the *Bacillus* cell is an alkalophilic *Bacillus.*

The introduction of a vector into a bacterial host cell may, for instance, be effected by protoplast transformation (see, *e.g.,* Chang and Cohen, 1979, Molecular General Genetics 168: 111-115), using competent cells (see, *e.g.,* Young and Spizizin, 1961, Journal of Bacteriology 81: 823-829, or Dubnau and Davidoff-Abelson, 1971, Journal of Molecular Biology 56: 209-221), electroporation (see, *e.g.,* Shigekawa and Dower, 1988, Biotechniques 6: 742-751), or conjugation (see, *e.g.,* Koehler and Thorne, 1987, Journal of Bacteriology 169: 5771-5278).

The host cell may be a eukaryote, such as a mammalian, insect, plant, or fungal cell.

In a preferred embodiment, the host cell is a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*)*.* In a more preferred embodiment, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9,1980).

In an even more preferred embodiment, the yeast host cell is a *Candida, Hansenula, Kluyveromyces, Pichia, Saccharomyces, Schizosaccharomyces,* or *Yarrowia* cell.

In a most preferred embodiment, the yeast host cell is a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. In another most preferred embodiment, the yeast host cell is a *Kluyveromyces lactis* cell. In another most preferred embodiment, the yeast host cell is a *Yarrowia lipolytica* cell.

In another more preferred embodiment, the fungal host cell is a filamentous fungal cell. "Filamentous fungi" include all filamentous forms of the subdivision Eumycota and Oomycota (as defined by Hawksworth *et al.,* 1995, *supra*). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In an even more preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium,* or *Trichoderma.*

In a most preferred embodiment, the filamentous fungal host cell is an *Aspergillus awamori, Aspergillus foetidus, Aspergillus japonicus, Aspergillus nidulans, Aspergillus niger* or *Aspergillus oryzae* cell. In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In an even most preferred embodiment, the filamentous fungal parent cell is a *Fusarium venenatum* (Nirenberg sp. nov.) cell. In another most preferred embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### The donor strain

The invention also provides a bacterium, *Alicyclobacillus sp.,* as deposited under accession number DSM 15716, and compositions comprising this microorganism.

### Methods for preparing enzyme polypeptides

The present invention also relates to methods for producing an enzyme of the invention comprising (a) cultivating a strain comprising a nucleotide sequence encoding an enzyme of the invention which strain is capable of expressing and secreting the enzyme and (b) recovering the enzyme. In a particular embodiment the strain is a wild type strain such as the *Alicyclobacillus sp.* DSM 15716, while in another embodiment the strain is a recombinant host cell as described, *supra.*

In these methods of the invention, the cells are cultivated in a nutrient medium suitable for production of the enzyme using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection). As the enzyme is secreted into the nutrient medium, the enzyme can be recovered directly from the medium.

The resulting enzyme may be recovered by methods known in the art. For example, the enzyme may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (*e.g.*, ion exchange, affinity, hydrophobic, chromatofocusing, and size exclusion), electrophoretic procedures (*e.g*., preparative isoelectric focusing), differential solubility (*e.g*., ammonium sulfate precipitation), SDS-PAGE, or extraction (see, *e.g.,* Protein Purification, J.-C. Janson and Lars Ryden, editors, VCH Publishers, New York, 1989).

Disclosed is also the TAST method of WO 01/77315 A1 on a sample of the *Alicyclobacillus sp.* DSM 15716, i.e. by fusing genes (e.g. from a gene library) from the genome of the *Alicyclobacillus sp.* DSM 15716 with a gene encoding a signalless reporter, such as a beta-lactamase, via a transposon tag, growing host cell clones comprising the genes of the *Alicyclobacillus sp.* DSM 15716 fused with a gene encoding a signalless reporter, such as a beta-lactamase, via a transposon tag in a medium revealing the presence of the reporter, such as an ampicillin containing medium, detetcting clones secreting the reporter and isolating gene and polypeptide of the *Alicyclobacillus sp.* DSM 15716 comprised in that clone.

When growing host cell clones comprising the genes of the *Alicyclobacillus sp.* DSM 15716 fused with a gene encoding a signalless reporter, such as a beta-lactamase, via a transposon tag in a medium revealing the presence of the reporter, such as an ampicillin containing medium, only those clones expressing and secreting the reporter (e.g. beta-lactamase) will be detected (e.g. survive). However the reporter will only be secreted if the gene to which the reporter gene is fused has an intact promotor and ribosome binding site (i.e. a gene which is expressed by the cell to produce a polypeptide in real life), which can be recognized in the host strain, and if the reporter is translated so that the synthesized polypeptide is transported across the cytoplasma membrane and folded correctly. Hence, when inserting the fused gene into a selected host cell, those clones, for which a reporter presence is detected (e.g. ampicillin resistance), will contain a gene from the *Alicyclobacillus sp*. DSM 15716, which encodes a functional secreted polypeptide.

### Transgenic plants

The present invention also relates to a transgenic plant, plant part, or plant cell that has been transformed with a nucleotide sequence encoding an enzyme of the invention so as to express and produce the enzyme. In one embodiment the plant could be used as host for production of enzyme in recoverable quantities. The enzyme may be recovered from the plant or plant part. Alternatively, the plant or plant part containing the recombinant enzyme may be used as such for improving the quality of a food or feed, *e.g*., improving nutritional value, palatability, and rheological properties, or to destroy an antinutritive factor. In particular the plant or plant parts expressing the enzyme may be used as an improved starting material for production of fuel-alcohols or bio-ethanol

The transgenic plant can be dicotyledonous (a dicot) or monocotyledonous (a monocot). Examples of monocot plants are grasses, such as meadow grass (blue grass, Poa), forage grass such as festuca, lolium, temperate grass, such as Agrostis, and cereals, *e.g*., wheat, oats, rye, barley, rice, sorghum, and maize (com).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers. Also specific plant tissues, such as chloroplast, apoplast, mitochondria, vacuole, peroxisomes, and cytoplasm are considered to be a plant part. Furthermore, any plant cell, whatever the tissue origin, is considered to be a plant part.

Also included within the scope of the present invention are the progeny of such plants, plant parts and plant cells.

The transgenic plant or plant cell expressing an enzyme of the invention may be constructed in accordance with methods known in the art. Briefly, the plant or plant cell is constructed by incorporating one or more expression constructs encoding an enzyme of the invention into the plant host genome and propagating the resulting modified plant or plant cell into a transgenic plant or plant cell.

Conveniently, the expression construct is a nucleic acid construct which comprises a nucleotide sequence encoding an enzyme of the present invention operably linked with appropriate regulatory sequences required for expression of the nucleotide sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the enzyme is desired to be expressed. For instance, the expression of the gene encoding an enzyme of the invention may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV promoter may be used (Franck et al., 1980, Cell 21: 285-294). Organ-specific promoters may be, for example, a promoter from storage sink tissues such as seeds, potato tubers, and fruits (Edwards & Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant and Cell Physiology 39: 885-889), a *Vicia faba* promoter from the legumin B4 and the unknown seed protein gene from *Vicia faba* (Conrad et al., 1998, Journal of Plant Physiology 152: 708-711), a promoter from a seed oil body protein (Chen et al., 1998, Plant and Cell Physiology 39: 935-941), the storage protein napA promoter from *Brassica napus,* or any other seed specific promoter known in the art, *e.g*., as described in WO 91/14772. Furthermore, the promoter may be a leaf specific promoter such as the *rbcs* promoter from rice or tomato (Kyozuka et al., 1993, Plant Physiology 102: 991-1000, the chlorella virus adenine methyltransferase gene promoter (Mitra and Higgins, 1994, Plant Molecular Biology 26: 85-93), or the *aldP* gene promoter from rice (Kagaya et al., 1995, Molecular and General Genetics 248: 668-674), or a wound inducible promoter such as the potato pin2 promoter (Xu et al., 1993, Plant Molecular Biology 22: 573-588).

A promoter enhancer element may also be used to achieve higher expression of the enzyme of the invention in the plant. For instance, the promoter enhancer element may be an intron which is placed between the promoter and the nucleotide sequence encoding an enzyme of the present invention. For instance, Xu *et al.*, 1993, *supra* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including *Agrobacterium*-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

Presently, *Agrobacterium tumefaciens*-mediated gene transfer is the method of choice for generating transgenic dicots (for a review, see Hooykas and Schilperoort, 1992, Plant Molecular Biology 19: 15-38). However it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992, Plant Journal 2: 275-281; Shimamoto, 1994, Current Opinion Biotechnology 5: 158-162; Vasil et al., 1992, Bio/Technology 10: 667-674). An alternative method for transformation of monocots is based on protoplast transformation as described by Omirulleh et al., 1993, Plant Molecular Biology 21: 415-428.

Following transformation, the transformants having incorporated therein the expression construct are selected and regenerated into whole plants according to methods well known in the art.

The present invention also relates to methods for producing an enzyme of the invention comprising (a) cultivating a transgenic plant or a plant cell comprising a nucleotide sequence encoding an enzyme of the invention under conditions conducive for production of the enzyme and (b) recovering the enzyme.

### Compositions comprising polypeptides and methods for their preparation

The invention provide a composition comprising a polypeptide of the invention and preferably an excipient and a method for preparing such a composition comprising admixing the polypeptide of the invention with an excipient. Preferably the composition comprises all polypeptides secreted when fermenting a sample of Alicyclobacillus sp. DSM 15716 or a mutant thereof wherein one or more genes has been deleted or added.

In a particular embodiment the polypeptide of the invention is the major (polypeptide) component of the composition, *e.g*., a mono-component composition. The excipient in this context is to be understood as any auxilliary agent or compound used to formulate the composition and includes solvent, carriers, stabilizers and the like.

The composition may further comprise one or more additional enzymes, such as an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase.

The compositions may be prepared in accordance with methods known in the art and may be in the form of a liquid or a solid composition. For instance, the enzyme composition may be formulated using methods known to the art of formulating polypeptides and/or pharmaceutical products, e.g. into coated or uncoated granules or micro-granules. The polypeptide of the invention may thus be provided in the form of a granule, preferably a non-dusting granule, a liquid, in particular a stabilized liquid, a slurry or a protected polypeptide. For certain applications, immobilization of the polypeptide on a solid matrix may be preferred.

The polypeptide to be included in the composition may be stabilized in accordance with methods known in the art e.g. by stabilizing the polypeptide in the composition by adding and antioxidant or reducing agent to limit oxidation of the polypeptide or it may be stabilized by adding polymers such as PVP, PVA, PEG or other suitable polymers known to be beneficial to the stability of polypeptides in solid or liquid compositions

In a further embodiment the composition of the invention is a detergent composition which, in addition to the polypeptide of the invention, comprises a surfactant and optionally compounds selected from the group consisting of builders such as zeolites, bleaching agents such as percarbonate, bleach enhancers such as TAED or NOBS, suds suppressors, fragrants, etc.

In a further embodiment the composition of the invention is a feed composition that in addition to the polypeptide of the invention comprises a cereal or grain product.

In a further embodiment the composition of the invention is a food composition such as a baker's flour composition, a brewed product, a fruit juice, an oil or lard product comprising the polypeptide of the invention.

In a further embodiment the composition of the invention comprises a polysaccharide or a mixture of polysaccharides and comprises the polypeptide of the invention.

In a further embodiment the composition of the invention is a pulping composition, which in addition to the polypeptide of the invention, comprises pulp.

In a further embodiment the composition of the invention is a biocidal composition, which comprises in addition to the polypeptide of the invention, an oxidoreductase enhancer.

### Use of polypeptides or compositions comprising them

In still further aspects the invention provides use of the polypeptides or polynucleotides of the invention or a composition comprising said polypeptides or polynucleotides in various applications, particularly (technical) processes such as processes performed in industry or household, herein under for commercial research purposes. Hence the invention encompasses a process comprising employing a polypeptide of the invention or a polynucleotide of the invention in a (technical) industrial, research or household process.

In one embodiment the polypeptide or the composition of the invention is used for cleaning a cellulosic fabric.

In another embodiment the polypeptide or the composition of the invention is used to prepare a food or feed additive.

In yet another embodiment the polypeptide or the composition of the invention is used for treatment of lignolosic materials and pulp.

### DETERGENT DISCLOSURE

The polypeptide of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the polypeptide of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase®, Savinase®, Primase®, Duralase®, Esperase®, and Kannase® (Novozymes A/S), Maxatase®, Maxacal®, Maxapem®, Properase®, Purafect®, Purafect OxP®, FN2®, and FN3® (Genencor International Inc.).

Lipases: Suitable lipases include those of bacterial or fungal origin. Chemically modified or protein engineered mutants are included. Examples of useful lipases include lipases from Humicola (synonym Thermomyces), e.g. from H. lanuginosa (T. lanuginosus) as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g. from B. subtilis (Dartois et al. (1993), Biochemica et Biophysica Acta, 1131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include LipolaseTM, Lipolase UltraTM and Lipex (Novozymes A/S).

Amylases: Suitable amylases (alpha and/or beta) include those of bacterial or fungal origin. Chemically modified or protein engineered mutants are included. Amylases include, for example, alpha-amylases obtained from Bacillus, e.g. a special strain of B. licheniformis, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl^{™}, Termamyl^{™}, Fungamyl^{™} and BAN^{™} (Novozymes A/S), Rapidase^{™} and Purastar^{™} (from Genencor International Inc.).

Cellulases: Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme®, and Carezyme® (Novozymes), Clazinase®, and Puradax HA® (Genencor International Inc.), and KAC-500(B) ® (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from Coprinus, e.g. from C. cinereus, and variants thereof as those described in WO 93/24618, WO 95/10602, and WO 98/15257.

Commercially available peroxidases include Guardzyme® (Novozymes A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1 % to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a H2O2 source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-100 mg of enzyme protein per litre of wash liquor, preferably 0.05-5 mg of enzyme protein per liter of wash liquor, in particular 0.1-1 mg of enzyme protein per litre of wash liquor.

The enzyme of the invention may additionally be incorporated in the detergent formulations disclosed in WO 97/07202.

### DEPOSITED MICROORGANISMS

The following microorganism were deposited by the applicant according to the Budapest Treaty on the International Recognition of the Deposits of Microorganisms for the Purpose of Patent Procedures at Deutsche Sammlung von Mikroorganismen und Zellkulturen GmbH, MascheroderWeg 1b, D-38124 Braunschweig, Germany:
June 30, 2003: *Alicyclobacillus sp.* CS81 thermo-acidophile; DSM accession No. 15716.

### EXAMPLES

### Example 1 Identifying functional polypeptides secreted by Alicyclobacillus sp. DSM 15716

### Genomic library construction

Chromosomal DNA from *Alicyclobacillus sp.* DSM 15716 was prepared by using standard molecular biology techniques (Ausuble et al. 1995 "Current protocols in molecular biology" Publ: John Wiley and sons). The prepared DNA was partially cleaved with Sau3A and separated on an agarose gel. Fragments of 3 to 8 kilobases were eluted and precipitated and resuspended in a suitable buffer.

A genomic library was made by using the Stratagene ZAP Express™ predigested Vector kit and Stratagene ZAP Express ™ predigested Gigapack ® cloning kit (Bam HI predigested) (Stratagene Inc., USA) following the instructions/recommendations from the vendor. The resulting lambdaZAP library comprised 38000 pfu of which 10000 were collected for mass excision. The resulting 70000 E. coli colonies were pooled and plasmids were prepared by using the Qiagen Spin Mini prep kit (Qiagen, Germany). The eluate of approx. 1 ml containing the plasmid DNA was precipitated in a centrifuge with 1 volume part of Na-acetate pH5 and 2 volume parts 96% ethanol at 20000 rpm at 4C, washed with 70% v/v ethanol, dried at room temperature and resuspended in 200 microl TE buffer. The DNA concentration of the plasmid pool DNA of the Alicyclobacillus sp. genomic library was 5.2 microgram/microliter.

### Transposon construction and preparation

The rationale behind the methology of Transposon Assisted Signal Trapping (TAST) as described in WO 01/77315 A1 is to fuse all genes within a selected genome with a gene encoding a signalless beta-lactamase via a transposon tag. Hence when growing host cell clones comprising the genes of a genome fused with a gene encoding a signalless beta-lactamase via a transposon tag in an ampicillin containing medium only those clones expressing and secreting a beta-lactamase will survive. However the beta-lactamase will only be secreted if the gene to which the beta-lactamase gene is fused has an intact promotor and ribosome binding site (i.e. a gene which is expressed by the cell to produce a polypeptide in real life), which can be recognized in the host strain, and if the beta-lactamase is translated so that the synthesized polypeptide is transported across the cytoplasma membrane and folded correctly. Hence, when inserting the fused gene into a selected host cell, those clones, which are ampicillin resistant contains a gene which encodes a functional secreted polypeptide.

Usually, when employing the TAST methodology it is even not necessary to express the entire gene. When tagging the genes with a transposon, expression of the N-terminal part of the genes as protein fusion shows that the genes contain intact transcription, translation and secretion sequences. Hence expression of the N-terminal part of the genes as protein fusion is usually regarded as sufficient for assuring expression and secretion of the entire gene.

Thus it can be concluded that the genes obtained by the TAST method actually do encode secreted functional polypeptides.

### Construction of a SigA4 transposon containing the β-lactamase reporter gene:

Following the instructions of WO 01/77315 A1, the construction of a transposon containing a signal-less β-lactamase gene was carried out using standard molecular biology techniques. The signal-less β-lactamase gene was initially PCR amplified from the vector pUC19) using a proofreading polymerase (Pfu Turbo, Stratagene, USA). The resulting PCR fragment contained the restriction sites *Not*I and *Eco*RI in order to aid cloning. The plasmid pEntranceposon(Cam^{r}) containing the Entranceposon and the antibiotic resistance markers *CAT* (encoding chloramphencol resistance in the transposon) was obtained from Finnzymes, OY (Espoo Finland). The plasmid was digested with the restriction enzymes *Not*I and *Eco*RI, gel purified and ligated with the signal-less β-lactamase containing fragment. The ligation was transformed into electro-competent DH10B cells and the *E.coli* clone containing the recombinant plasmid with the signal-less β-lactamase was identified by restriction analysis and named SigA2.

For transposon preparation, a smaller derivative of SigA2 was constructed, which lacked the *bla* gene encoding beta-lactamase: Two oligonucleotide primers SigA2NotU-P 5'-TCG CGA TCC GTT TTC GCA TTT ATC GTG AAA CGC T-3' (SEQ ID NO: 51) and SigA2NotD-P 5'-CCG CAA ACG CTG GTG AAA GTA AAA GAT GCT GAA-3' (SEQ ID NO: 52), which bind to the start and stop of the *bla* gene of SigA2 directing outwards were used PCR amplify SigA2 without the *bla* gene. An amplificate of approx. 3,6 kb generated in the this PCR reaction was relegated and transformed in to a suitable E.coli strain. A plasmid of 3,6 kb was isolated from a transformant which was able to grow on LB chloramphenicol but not on LB ampicillin. This plasmid maintained both BgIII sites and lacks the active *bla* gene and was called pSig4. 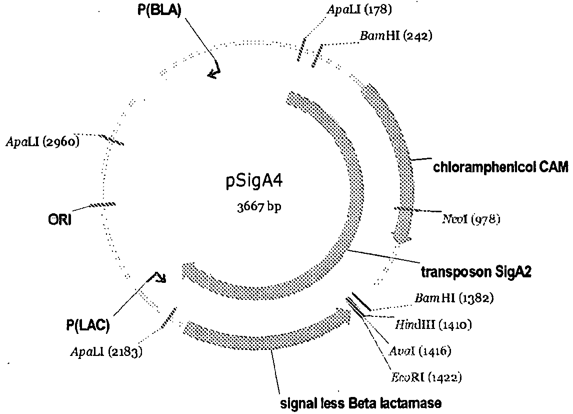 60 microliter of pSigA4 plasmid DNA preparation with a concentration of 0.3 microgram/microliter was digested with BgIII and separated on an agarose gel. The SigA2 transposon DNA band of 2 kb was eluted and purified by using the "GFX™PCR, DNA and Gel Band Purification Kit" (Amersham Pharmacia Biotech Inc,USA) according to the instructions of the vender and eluted in 200 microliter EB buffer.

### C. Transposon tagging

The transposon prepared from pSigA4 carries a 5'-truncated bla-gene encoding a β-lactamase from which the secretion signal has been removed. The β-lactamase conveys ampicillin resistance on *E. coli* only when the protein is secreted to the periplasm, whereas cytoplasmic expression of β-lactamase does not confer ampicillin resistance. Without a signal sequence, the β-lactamase enzyme will not be transported to the periplasm and therefore the clone will not grow on media containing ampicillin. The signal-less β-lactamase gene was contained within the transposon in such a way that there was a continuous open reading frame between the transposon border and the β-lactamase coding region. In this way the modified transposon, when it transposes into a gene encoding a protein that is secreted, could cause an in-frame fusion with the target gene. This resulted in a fusion gene product that is secreted to the periplasm of *E. coli* and conveys resistance to the ampicillin. If the transposon integrated even in-frame into a gene encoding a non-secreted protein, the respective host will not become ampicillin resistance.

For the in vitro transposon tagging of the Alicyclobacillus sp. library, 4 or 8 microliter of SigA2 transposon containing approx. 2,6 ug DNA were mixed with 1 microliter of the DNA concentration of the plasmid pool DNA of the Alicyclobacillus sp. genomic library, 2 microliter of Finnzymes MuA Transposase (0,22 microgram/microliter) and 5 microliter of 5x buffer from Finnzymes OY, Espoo, Finland) in a total volume of 50 microliter and incubated at 30 °C for 3,5 h and followed by heat inactivation at 75 °C for 10 min. The DNA was precipitated by addition of 5microliter 3M Na-acetate pH5 and 110 microliter 96% ethanol and centrifugation for 30 min at 20000 rpm. The pellet was washed and dried and resuspended in 10 microliter TE buffer.

### D. Transformation and selection

Electro-competent *E*. *coli* DH10B cells were transformed by electroporation in a Biorad Gene Pulse device (50uF, 25mAmp, 1.8 kV with 5 microliter of the transposon tagged plasmid pool, mixed with 1 ml SOC medium, pre-incubated for 1 at 37C and plated on LB with 25 microliter/mililiter ampicillin, 50 microliter/mililiter kanamycin, 10 microliter/mililiter chloramphenicol and incubated for 2-3 days. Out of the resistant transformants 1056 colonies were selected and plasmids were prepared by applying the Qiaprep 96 Turbo Biorobot kit according to the instructions of the vender.

### E. Plasmid preparation and sequencing

1056 transposon tagged plasmids were sequenced in with the A2up primer AGCGTTTGCGGCCGCGATCC (SEQ ID NO: 53) which read upstream into the into the transposon tagged gene, and, in a second reaction, with B primer TTATTCGGTCGAAAAGGATCC (SEQ ID NO: 54) which read downstream into the transposon tagged gene.

### F. Sequence assembly and annotation

The obtained sequences were assembled into contigs by using the program PhredPhrap (Brent Ewing, LaDeana Hillier, Michael C. Wendl, and Phil Green, Base-calling of automated sequencer traces using phred I. Accuracy assessment (1998) Genome Research 8:175-185; Brent Ewing and Phil Green, Base-calling of automated sequencer traces using phred II. Error probabilities (1998) Genome Research 8:186-194). The obtained contigs were subsequently compared to sequences available in standard public DNA and protein sequences databases by using the program BLASTX 2.0a19MP-WashU [14-Jul-1998] [Build linux-x86 18:51:44 30-Jul-1998] (Gish, Warren (1994-1997). Unpublished; Gish, Warren and David J. States (1993). Identification of protein coding regions by database similarity search. Nat. Genet. 3:266-72).

The obtained sequences were functional genes which encoded intact and functional polypeptides, because they were obtained as ampicillin resistant clones as explained *supra.*

### Example 2. Determining function by homology

The function of the polypeptides SEQ ID NO: 26 to SEQ ID NO: 50 were annotated by sequences comparison with genes or polypeptides of known function. The polypeptides of the invention were compared to a list of closest related sequences from public and inhouse databases of contig's. The contigs, from which SEQ ID NO: 26 to SEQ ID NO: 50 were derived, were subsequently compared to sequences available in standard public DNA and protein sequences databases by using the program BLASTX 2.0a19MP-WashU [14-Jul-1998]. A careful analysis of sequence alignments of SEQ ID NO: 26 to SEQ ID NO: 40 to their closest related sequences with known function from other databases made it possible to predict the function of these polypeptides on the basis of the degree of amino acid identity. Even when the overall amino acid identity was below 40%, which usually makes it difficult to make a good prediction, we were able to predict the function of SEQ ID NO: 26 to SEQ ID NO: 40 by carefully analysing and interpreting the amino acid residues in the catalytic sites or in important regions of the polypeptide sequences. If the amino acids of the catalytic site of a known sequences were also present in the polypeptide of the invention, combined with a sufficient overall amino acid identity, it was concluded that the polypeptide from Alicyclobacillus sp DSM 15716 had the same function as the known sequence.

### Example 3 Preparing polypeptides of SEQ ID NO: 26 to SEQ ID NO: 50

To prepare the polypeptides of SEQ ID NO: 26 to SEQ ID NO: 50, the genes comprised in SEQ ID NO: 1 to SEQ ID NO: 25 encoding these polypeptides are expressed by fusing the DNA encoding the open reading frame to DNA a promoter, ribosome- binding site and terminator suitable for genes expression in an appropriate host strain, for example *Escherichia coli, Bacillus subtilis, Bacillus licheniformis* or *Bacillus clausii* or a derivative of *Alicyclobacillus sp*. The promoter can either be an inducible promotor or a constitutive promoter. Any signal sequences of SEQ ID NO: 26 to SEQ ID NO: 50 can be exchanged with a suitable signal peptide of another bacterium. The expression construct can either be part of a plasmid or of a linear DNA. It can be integrated into the chromosome of the host strain by recombination or it can be present in the host cell on a plasmid. Then the transformed cells carrying the gene of interest are grown in a suitable medium in the desired volume. If an inducible promoter is used, the gene expression is started by adding the inducer. Otherwise a no inducer is needed and the cells will be grown until a suitable amount of protein from the gene of interest is produced. Then the culture is harvested and the proteins are recovered by standard methods.

### Example 4. Determining serine-carboxyl protease activity

The culture fluid or a cell lysate of a host strain synthesising and secreting a serine-carboxy protease in a suitable buffer may be assayed for that activity. A suitable volume of such a sample is spotted on agarose plates which contain the insoluble chromogenic substrate AZCL-collagen (Megazyme ™) or Azocoll (Sigma-Aldrich) and a suitable buffer at acidic pH, e.g. pH is 3-5. The plate is incubated for an appropriate time, e.g. one day at an appropriate temperature, e.g. 55°C. The activity is visible as blue halos around the spots. As an alternative to AZCL-collagen or Azocoll, non-labelled collagen is added to agar plates, on which enzyme activity can be detected as clearing zones. By addition of pepstatin, the protease activity of a serine carboxyl protease cannot be inhibited. As an alternative, the activity determination of a sample containing a serine-carboxyl protease can be measured as described in Tsuruoka N, Nakayama T, Ashida M, Hemmi H, Nakao M, Minakata H, Oyama H, Oda K, Nishino T; "Collagenolytic serine-carboxyl proteinase from Alicyclobacillus sendaiensis strain NTAP-1: purification, characterization, gene cloning, and heterologous expression." Appl Environ Microbiol. Vol. 69(1); pp 162-169; 2003 Jan.

### Example 5. Determining multi-copper oxidase activity

The culture fluid or a cell lysate of a host strain synthesising and secreting a multi-copper oxidase in a suitable buffer may be assayed for that activity as described in Schneider et al., Enzyme and Microbial Technology 25, (1999) p. 502-508).

For example a suitable volume, which can be 15 microliter, of such a sample is spotted on agarose plates which contain ABTS (2,2'-Azinobis 3-Ethylbenzthiazolin-6-sulfonic acid) at a suitable concentration, e.g. 1 mM, in a suitable puffer, e.g. 0,1 M sodium acetat buffer for pH 5,5. The plate is incubated for an appropriate time e.g. 16 hours, at an appropriate temperature, e.g. 55 °C. The activity is visible as a green zone around the sample. The assay works on supernatants and extracts.

### Example 6. Determining serine protease activity

The culture fluid or a cell lysate of a host strain synthesising and secreting a serine protease in a suitable buffer may be assayed for that activity. A suitable volume of such a sample is spotted on agarose plates which contain the insoluble chromogenic substrate AZCL-casein (Megazyme™) or AZCL-collagen (Megazyme™) and a suitable buffer at suitable pH. The plate is incubated for an appropriate time, e.g. one day, at an appropriate temperature, e.g. 55°C. The activity is visible as blue halos around the spots. As an alternative to AZCL-casein and AZCL-collagen (Megazyme™) non-labelled casein or non-labelled collagene can be used. On non-labelled collagen or non-labelled casein spotted on agarose plates, clearing zones form in the presence of a serine protease.

### Example 7. Determining glutamic peptidase activity

The culture fluid or a cell lysate of a host strain synthesising and secreting a glutamic peptidase in a suitable buffer was assayed for that activity. A suitable volume of such a sample can be spotted on agarose plates, which contain the insoluble chromogenic substrate AZCL-collagen (Megazyme™) and a suitable buffer at acidic pH, e.g. pH is 3-5. The plate can be incubated for an appropriate time, e.g. one day, at an appropriate temperature, e.g. 55°C. The activity is visible as blue halos around the spots. As an alternative to AZCL-collagen, non-labelled collagen can be used. On non-labelled collagen spotted on agarose plates, clearing zones form in the presence of a glutamic peptidase. Upon specifically testing the glutamic peptidase of ID NO: 27; the activity was determined as a spot test of 20 microliter culture fluid on 0.1% AZCL-collagen (Megazyme™) spotted on LB-PG agar plates at pH 3.4. The plates were incubated at 55 °C (over night) and the presence of the glutamic peptidase was visible as blue halos around the spots.

The glutamic peptidase comprised in SEQ ID NO: 27 showed significant sequence similarity to peptidases belonging to family A4 now reclassified as peptidase family G1 (PepG) (EC 3.4.23.19) by MEROPS see the section describing SEQ ID NO: 27, *supra* and Fujinaga M, Cherney MM, Oyama H, Oda K, James MN.; The molecular structure and catalytic mechanism of a novel carboxyl peptidase from Scytalidium lignicolum; Proc. Natl. Acad. Sci. U. S. A.; 101 (10); pp. 3364-9; Epub 01-Mar-2004; 09-Mar-2004. This family contains peptidase sequences, which have Q and E conserved in their active site. Both residues were conserved in the glutamic peptidase comprised in SEQ ID NO: 27. The glutamic peptidase comprised in SEQ ID NO: 27 is thus the first bacterial polypeptide of the G1 family previously counting only fungal peptidases.

SEQ ID NO: 27 was compared, inter alia, to a reference sequence of family G1 peptidases; Aspergillus niger aspergillopepsin II (SEQ ID NO: 55; Swissprot P24665; Takahashi,K.; Inoue,H.; Sakai,K.; Kohama,T.; Kitahara,S.; Takishima,K.; Tanji,M.; Athauda,S.B.P.; Takahashi,T.; Akanuma,H.; Mamiya,G.; Yamasaki, M); The primary structure of Aspergillus niger acid proteinase A.; J. Biol. Chem.; Vol 266; p. 19480; 1991). This polypeptide contained a signal peptide (aa1-aa18), and two propeptides (aa 19-58 and aa 99-109), which are removed after secretion during maturation. During maturation a heavy and a light chain are formed, which are cross-linked by disulfide bridges between cysteine residues. (Inoue,H.; Kimura,T.; Makabe,O.; Takahashi,K.; The gene and deduced protein sequences of the zymogene of Aspergillus niger acid proteinase A; J. Biol. Chem.; vol. 266; p. 19484; 1991). The amino acids similar to the second propeptide (aa99-109) and the amino acids corresponding to the cross-linking cysteine residues of SEQ ID NO.55 are missing in SEQ ID 27 (see alignment). Only a fungal G1 peptidase has previously been described to lack cysteine residues (Maita,T.; Nagata,S.; Matsuda,G.; Maruta,S.; Oda,K.; Murao,S.; Tsuru,D.; Complete amino acid sequence of Scytalidium lignicolum acid protease B; J. Biochem.; vol. 95; p. 465; 1984).

Alignment of SEQ ID NO: 55 with SEQ ID NO: 27

### Example 8. Determining acid beta-glucanase activity

The culture fluid or a cell lysate of a host strain synthesising and secreting an beta-glucanase in a suitable buffer may be assayed for that activity. A suitable volume of such a sample is spotted on agarose plates which contain the insoluble chromogenic substrate AZCL-beta-glucan (Megazyme™) and a suitable buffer at acidic pH, e.g. pH is 3-5. The plate is incubated for an appropriate time, e.g. one day, at an appropriate temperature, e.g. 55°C. The activity is visible as blue halos around the spots.

### Example 9. Determining acid phosphatase activity

A suitable volume of the culture fluid or a cell lysate of a host strain synthesising and secreting the acid phosphatase in a suitable buffer at a suitable pH at an appropriate temperature, e.g. 55°C is incubated with para-nitrophenolphosphate (pNPP) for measuring the enzyme activity. The products of the enzymatic reaction are p-nitrophenol and inorganic phosphate or Pi. NaOH is added to end the phosphatase assay after a suitable reaction time and forms p-nitrophenolate. The absorbation of p-nitrophenolate is measured optically at 405 nm.
As an alternative, a suitable volume of the culture fluid or a cell lysate of a host strain synthesising and secreting the acid phosphatase in a suitable buffer at a suitable pH at an appropriate temperature, e.g. 55 °C is used for measuring the enzyme activity with the EnzChek™ Acid Phosphatase Assay Kit (E-12020) (Molecular Probes Europe BV; PoortGebouw, Rijnsburgerweg 10; 2333 AA Leiden, The Netherlands).

### Example 10. Determining polysaccharide deacetylase activity

A suitable volume of the culture fluid or a cell lysate of a host strain synthesising and secreting the polysaccharide deacteylase in a suitable buffer at an appropriate temperature, e.g. 55°C is used for measuring the activity. Bacterial murein, *N,N'*-diacetylchitobiose (Sigma) or galactose pentaacetate (Sigma) or and cellulose acetate (Sigma) can be used as substrate(s) for this enzyme type. The acetate released from the substrate by the enzyme can be measured with an acetic acid assay kit (Biopharm) adapted for the physical requierments of the enzyme (Kosugi A, Murashima K, and Doi RH; Xylanase and Acetyl Xylan Esterase Activities of XynA, a Key Subunit of the Clostridium cellulovorans Cellulosome for Xylan Degradation; Appl. Environm. I Microbiol.; vol. 68; pp. 6399-6402; 2002)

### Example 11. Determining endo-beta-N-acetylglucosaminidase activity

A suitable volume of the culture fluid or a cell lysate of a host strain synthesising and secreting the endo-beta-N-acetylglucosaminidase activity in a suitable buffer, e.g. pH 3-5, at an appropriate temperature, e.g. 55 °C can be used for measuring the activity in accordance with MH Rashid, M Mori and J Sekiguchi; Glucosaminidase of Bacillus subtilis: cloning, regulation, primary strucfure and biochemical characterization; Microbiology; vol. 141; pp. 2391-2404; 1995.

### Example 12. Determining peptidyl proly-isomerase activity

A suitable volume of the culture fluid or a cell lysate of a host strain synthesising and secreting the polysaccharide deacteylase in a suitable buffer at an appropriate temperature, e.g. 55 °C is used for measuring the activity. The activity can be determined in accordance to Fischer, G., Bang, H. and Mech, C.; Determination of enzymatic catalysis for the cis-trans-isomerization of peptide binding in proline-containing peptides.; Biomed. Biochim. Acta; vol. 43; pp. 1101-1111;1984. This assay may be modified appropriately to suit the specific peptidyl proly-isomerase such as that comprised in SEQ ID NO: 36.

### Example 13. Determining acid cellulase activity

The culture fluid or a cell lysate of a host strain synthesising and secreting an acid cellulase in a suitable buffer may be assayed for that activity. A suitable volume of such a sample is spotted on agarose plates which contain the insoluble chromogenic substrate AZCL-HE-cellulose (Megazyme™) and a suitable buffer at acidic pH, e.g. pH is 3-5. The plate is incubated for an appropriate time, e.g. one day, at an appropriate temperature, e.g. 55°C. Presence of acid cellulase is visible as blue halos around the spots.

### Example 14. Determining xylan deacetylase activity

A suitable volume of the culture fluid or a cell lysate of a host strain synthesising and secreting the polysaccharide deacteylase in a suitable buffer at an appropriate temperature, e.g. 55°C can be used for measuring xylan deacetylase activity. Xylan deacetylase activity is measured as acetate release from acetylated xylan, which is prepared from birchwood xylan by the method of Johnson et al. 1988 (Johnson, K. G., J. D. Fontana, and C. R. Mackenzie. 1988. Measurement of acetylxylan esterase in Streptomyces. Methods Enzymol. 160:551-560). The acetate released from acetyl xylan is measured with an acetic acid assay kit (Biopharm) adapted for the physical requierments of the enzyme (Kosugi A, Murashima K, and Doi RH; Xylanase and Acetyl Xylan Esterase Activities of XynA, a Key Subunit of the Clostridium cellulovorans Cellulosome for Xylan Degradation; Appl. Environm. I Microbiol.; vol. 68; pp. 6399-6402; 2002).

### Example 15. Determining phytase activity

The culture fluid or a cell lysate of a host strain synthesising and secreting a phytase in a suitable buffer may be assayed for phytase activity. A suitable volume of such a sample is diluted in 0.1 M sodium acetate and 0.01% Tween-20, pH 5.5 in a suitable buffer, which can be -HCl at pH 3.0 to 3.5, sodium acetate at pH 4.0 to 5.5, morpholincethanesulfonic acid (MES) at pH 6.0 to 6.5, and Tris-HCl at pH 7.0 to 9.0, are further diluted in 26-fold into the substrate solution (5 mM sodium phytate [Sigma] in 0.1 M sodium acetate, and 0.01% Tween-20 [pH 5.5], and preincubated at 37°C) to start the reaction. After 30 min at 37°C, the reaction is stopped by adding an equal volume of 10% trichloroacetic acid. Free inorganic phosphate is measured by the addition of an equal volume of molybdate reagent containing, in 100 ml, 7.3 g of FeSO₄, 1.0 g of (NH₄)₆Mo₇O₂₄ · 4H₂O, and 3.2 ml of H₂SO₄. Absorbance was measured at 750 nm (Vmax microtiter plate reader; Molecular Devices) (Lassen SF; Breinholt J; Ostergaard PR; Brugger R; Bischoff A; Wyss M; Fuglsang CC; Expression, gene cloning, and characterization of five novel phytases from four basidiomycete fungi: Peniophora lycii, Agrocybe pediades, a Ceriporia sp., and Trametes pubescens; Appl. Environ. Micr.; 67; pp. 4701-4707; 2001).

### Example 16. Determining phospholipase activity

The culture fluid or a cell lysate of a host strain synthesising and secreting a phospholipase in a suitable buffer may be assayed for phospholipase activity. Lecithin is added to suitable volume of such a sample. The Lecithin is hydrolyzed under constant pH and temperature, and the phospholipase activity is determined as the rate of titrant (0.1 N NaOH) consumption during neutralization of the liberated fatty acid. The substrate is soy lecithin (L-α-Phosphotidyl-Choline), and the conditions are pH 8.00, 40.0°C, reaction time 2 min. The unit (LEU) is defined relative to a standard.

### Example 17: Expression of glutamic peptidase gene (SEQ ID NO: 2) in Bacillus subtilis.

The signal peptide from the protease SAVINASE™ (also known as subtilisin 309 from B. Licheniformis from Novozymes A/S) was fused by PCR in frame to the gene encoding the glutamic peptidase (SEQ ID NO: 2). The DNA coding for the resulting coding sequence was integrated by homologous recombination on the *Bacillus subtilis* host cell genome. The gene construct was expressed under the control of a triple promoter system (as described in WO 99/43835), consisting of the promoters from *Bacillus licheniformis* alpha-amylase gene (*amyL*), Bacillus amyloliquefaciens alpha-amylase gene (*amyQ*), and the *Bacillus thuringiensis cryIIIA* promoter including stabilizing sequence. The gene coding for Chloramphenicol acetyl-transferase was used as maker (Described e.g in Diderichsen et al., A useful cloning vector for Bacillus subtilis. Plasmid, 30, p. 312, 1993).

Chloramphenicol resistant transformants were analyzed by DNA sequencing to veriify the correct DNA sequence of the construct. One such clone was selected.

Fermentations of the glutamic peptidase (SEQ ID NO: 2) expression clone was performed on a rotary shaking table in 500 ml baffled Erlenmeyer flasks each containing 100 ml PS-1 media supplemented with 6 mg/l chloramphenicol. The clone was fermented for 6 days at 37 °C and sample was taken at day 3, 4, 5 and 6 and analyzed for proteolytic activity. The activity was determined (se example 7) as a spot test of 20 microliter culture fluid on 0.1 % AZCL-collagen (Megazyme™) LB-PG agar plates at pH 3.4. The plates were incubated at 55 °C (over night) and the activity was visible as blue halos around the spots.

### Example 18: Purification and characterization of the family A4 protease from Alicyclobacillus sp.

### Purification

Culture broth was centrifuged (20000 x g, 20 min) and the supernatants were carefully decanted from the precipitates. The combined supernatants were filtered through a Seitz EKS plate in order to remove the rest of the *Bacillus* host cells. The EKS filtrate was adjusted to pH 4.0 with citric acid and heated to 70°C with good stirring on a water bath. When the solution reached 70°C (it took approx. 15 minutes to get from 25°C to 70°C), the solution was immediately placed on ice. This heat treatment resulted in some precipitation, which was removed by another Seitz EKS filter plate filtration. Ammonium sulfate was added to the second EKS fitrate to 1.6M final concentration and the pool was applied to a Butyl Toyopearl S column equilibrated in 20mM CH₃COOH/NaOH, 1.6M (NH₄)₂SO₄, pH 4.5. After washing the Butyl column extensively with the equilibration buffer, the enzyme was eluted with a linear (NH₄)₂SO₄ gradient (1.6 to 0M) in the same buffer. Fractions from the column were analysed for protease activity (using the pH 4.0 Assay buffer and 37°C assay temperature) and fractions with activity were pooled. The pooled fractions were transferred to 20mM CH₃COOH/NaOH, pH 5.5 on a G25 sephadex column and applied to a SOURCE 30Q column equilibrated in the same buffer. After washing the SOURCE 30Q column extensively with the equilibration buffer, the protease was eluted with a linear NaCl gradient (0 to 0.5M) in the same buffer. Fractions from the column were analysed for protease activity (pH 4.0, 37°C) and fractions with activity were pooled. The pool, which was slightly coloured, was treated with 1% (w/v) Activated charcoal for 5 minutes and the charcoal was removed by a 0.45 µm filtration. The purity of the filtrate was analysed by SDS-PAGE, where only one band was seen on the coomassie stained gel.

### Assay:

A Protazyme OL (cross-linked and dyed collagen) assay was used. A Protazyme OL tablet (from Megazyme) was suspended in 2.0ml 0.01% Triton X-100 by gentle stirring. 500 microliter of this suspension and 500 microliter assay buffer were mixed in an Eppendorf tube and placed on ice. 20 microliter protease sample (diluted in 0.01% Triton X-100) was added. The assay was initiated by transferring the Eppendorf tube to an Eppendorf thermomixer, which was set to the assay temperature. The tube was incubated for 15 minutes on the Eppendorf thermomixer at its highest shaking rate (1400 rpm). The incubation was stopped by transferring the tube back to the ice bath. Then the tube was centrifuged in an icecold centrifuge for a few minutes, 200 microliter supernatant was transferred to a microtiter plate and OD₆₅₀ was read at 650 nm. A buffer blind was included in the assay (instead of enzyme). OD₆₅₀(enzyme) - OD₆₅₀(buffer blind) was a measure of protease activity."

Protease assay:

| | |
|---|---|
| Substrate : | Protazyme OL tablets (Megazyme T-PROL). |
| Temperature : | Controlled. |
| Assay buffers : | 100mM succinic acid, 100mM HEPES, 100mM CHES, 100mM CABS, 1 mM CaCl₂, 150mM KCI, 0.01% Triton X-100 adjusted to pH-values 2.0, 3.0, 4.0, 5.0, 6.0, 7.0, 8.0, 9.0, 10.0, 11.0 and 12.0 with HCl or NaOH. |

### Characterisation: pH-activity, pH-stability, and temperature-activity:

The above protease assay was used for obtaining the pH-activity profile, the pH-stability profile as well as the temperature-activity profile at pH 3.0. For the pH-stability profile the protease was diluted 5x in the Assay buffers and incubated for 2 hours at 37°C. After incubation the protease samples were transferred to pH 3.0, before assay for residual activity, by dilution in the pH 3 Assay buffer.

### pH-activity profile at 37°C

| **pH** | **Alicyclobacillus protease from EXP00663** |
|---|---|
| 2 | 0.90 |
| 3 | 0.98 |
| 4 | 1.00 |
| 5 | 0.93 |
| 6 | 0.77 |
| 7 | 0.28 |
| 8 | 0.04 |
| 9 | 0.02 |

### pH-stability profile (residual activity after 2 hours at 37°C)

| **pH** | **Alicyclobacillus protease from EXP00663** |
|---|---|
| 2.0 | 0.93 |
| 3.0 | 0.97 |
| 4.0 | 0.94 |
| 5.0 | 0.97 |
| 6.0 | 0.93 |
| 7.0 | 0.94 |
| 8.0 | 0.99 |
| 9.0 | 0.94 |
| 10.0 | 0.81 |
| 11.0 | 0.76 |
| 12.0 | 0.46 |
| 3.0 and after 2 hours at 5 °C | 1.00 |

### Temperature activity profile (al pH 3.0)

| **Temp (°C)** | **Alicyclobacillus protease from EXP00663** |
|---|---|
| 15 | 0.08 |
| 25 | 0.19 |
| 37 | 0.60 |
| 50 | 0.94 |
| 60 | 1.00 |
| 70 | 0.89 |
| 80 | 0.45 |

### Other characteristics:

The relative molecular weight of the A4 protease as determined by SDS-PAGE was: Mᵣ = 26 kDa.

### Example 19: Expression of acid cellulase gene (SEQ ID NO: 1) in Bacillus subtilis.

The signal peptide from Termamyl™ (Novozymes) was fused by PCR in frame to the gene encoding the acid cellulase (SEQ ID NO: 1). The DNA coding for the resulting coding sequence was integrated by homologous recombination on the *Bacillus subtilis* host cell genome. The gene construct was expressed under the control of a triple promoter system (as described in WO 99/43835), consisting of the promoters from *Bacillus licheniformis* alpha-amylase gene (*amyL*), Bacillus amyloliquefaciens alpha-amylase gene (*amyQ*), and the *Bacillus thuringiensis cryIIIA* promoter including stabilizing sequence. The gene coding for Chloramphenicol acetyl-transferase was used as maker (Described e.g in Diderichsen et al., A useful cloning vector for Bacillus subtilis. Plasmid, 30, p. 312, 1993).

Chloramphenicol resistant transformants were analyzed by DNA sequencing to verify the correct DNA sequence of the construct. One such clone was selected.

Fermentations of the acid cellulase (SEQ ID NO: 1) expression clone was performed on a rotary shaking table in 500 ml baffled Erlenmeyer flasks each containing 100 ml PS-1 media supplemented with 6 mg/l chloramphenicol. The clone was fermented for 3 days at 37 °C and sample was taken at day 1,2 and 3 and analyzed for cellulase activity. The activity was determined as a spot test of 20 microliter culture fluid on 0.1% AZCL-HE-cellulase (Megazyme ™) LB-PG agar plates at pH 3.4. The plates were incubated at 55 °C (over night) and the activity was visible as blue halos around the spots.

## Claims

1. An isolated polypeptide comprising an amino acid sequence which has at least 90% identity with SEQ ID NO: 27, wherein the polypeptide has glutamic peptidase activity.

2. The polypeptide of claim 1, wherein the polynucleotide encoding the polypeptide consists of the region of SEQ ID NO: 2 encoding a mature glutamic peptidase or a sequence differing there from by virtue of the degeneracy of the genetic code.

3. The polypeptide of claim 1, **characterised by** being free of disulphide bridges in the peptidase structure.

4. The polypeptide of claim 1, obtainable from the strain of *Alicyclobacillus sp.* deposited under DSM accession No. 15716.

5. The polypeptide of claim 1, comprising or consisting of the mature glutamic peptidase comprised in SEQ ID NO: 27.

6. The polypeptide enzyme of claim 5, comprising or consisting of the sequence from position 1 to 240 of SEQ ID NO: 27.

7. A bacterial strain deposited under accession number DSM 15716.

8. A composition comprising the polypeptide of claims 1 to 6.

9. The composition of claim 8, further comprising at least two different polypeptides, preferably at least 3, more preferable at least 5, more preferable at least 10, more preferable at least 15, more preferable at least 20 different polypeptides, preferably all polypeptides secreted when fermenting a sample of Alicyclobacillus sp. DSM 15716 or a mutant thereof wherein one or more genes has been deleted or added.

10. The composition of claim 8, further comprising one or more additional enzymes.

11. The composition of claim 8, **characterized by** being a detergent composition which, in addition to the polypeptide, comprises a surfactant.

12. The composition of claim 8, **characterized by** being a feed composition which in addition to the polypeptide comprises a cereal or grain product.

13. The composition of claim 8, **characterized by** being a food composition.

14. The composition of claim 8, further comprising a polysaccharide or a mixture of polysaccharides.

15. A method for preparing a composition of claim 8, comprising admixing the polypeptide of claims 1 to 6 with an excipient.

16. A polynucleotide having a nucleotide sequence which encodes for the polypeptide defined in any of claims 1 to 6.

17. A composition comprising the polynucleotide of claim 16

18. A nucleic acid construct comprising the nucleotide sequence defined in claim 16 operably linked to one or more control sequences that direct the production of the polypeptide in a host cell.

19. A recombinant expression vector comprising the nucleic acid construct of claim 18.

20. A recombinant host cell comprising the nucleic acid construct of claim 18.

21. A method for producing a polypeptide of claims 1 to 6 comprising:
(a) cultivating a recombinant host cell as defined in claim 20 under conditions conducive for production of the polypeptide; and
(b) recovering the polypeptide.

22. A process comprising employing a polypeptide of claims 1 to 6 or a polynucleotide of claim 16 in an industrial or household technical process.

## Patentansprüche

1. Isoliertes Polypeptid, umfassend eine Aminosäuresequenz, die mindestens 90% Identität mit SEQ ID NO: 27 aufweist, wobei das Polypeptid Glutamatpeptidaseaktivität aufweist.

2. Polypeptid nach Anspruch 1, wobei das das Polypeptid kodierende Polynukleotid aus der Region von SEQ ID NO: 2 besteht, die eine maturierte Glutamatpeptidase oder eine Sequenz, die sich davon aufgrund der Degeneriertheit des genetischen Codes unterscheidet, kodiert.

3. Polypeptid nach Anspruch 1, **dadurch gekennzeichnet, dass** es in der Peptidasestruktur frei von Disulfidbrücken ist.

4. Polypeptid nach Anspruch 1, erhältlich aus dem Stamm von Alicyclobacillus sp., der unter DSM Zugangsnummer 15716 hinterlegt ist.

5. Polypeptid nach Anspruch 1, umfassend oder bestehend aus der maturierten Glutamatpeptidase, die in SEQ ID NO: 27 umfasst ist.

6. Polypeptidenzym nach Anspruch 5, umfassend oder bestehend aus der Sequenz aus Position 1 bis 240 von SEQ ID NO: 27.

7. Bakterienstamm, hinterlegt unter Zugangsnummer DSM 15716.

8. Zusammensetzung, umfassend das Polypeptid gemäß Ansprüchen 1 bis 6.

9. Zusammensetzung nach Anspruch 8, des Weiteren umfassend mindestens zwei unterschiedliche Polypeptide, vorzugsweise mindestens 3, stärker bevorzugt mindestens 5, stärker bevorzugt mindestens 10, stärker bevorzugt mindestens 15, stärker bevorzugt mindestens 20 unterschiedliche Polypeptide, vorzugsweise alle Polypeptide sezerniert beim Fermentieren einer Probe von Alicyclobacillus sp. DSM 15716 oder einer Mutante davon, in der ein oder mehrere Gene deletiert oder hinzugefügt worden sind.

10. Zusammensetzung nach Anspruch 8, des Weiteren umfassend ein oder mehrere zusätzliche Enzyme.

11. Zusammensetzung nach Anspruch 8, **dadurch gekennzeichnet, dass** sie eine Detergenszusammensetzung ist, die zusätzlich zu dem Polypeptid ein oberflächenaktives Mittel umfasst.

12. Zusammensetzung nach Anspruch 8, **dadurch gekennzeichnet, dass** sie eine Futtermittelzusammensetzung ist, die zusätzlich zu dem Polypeptid ein Getreide- oder Kornprodukt enthält.

13. Zusammensetzung nach Anspruch 8, **dadurch gekennzeichnet, dass** sie eine Lebensmittelzusammensetzung ist.

14. Zusammensetzung nach Anspruch 8, des Weiteren umfassend ein Polysaccharid oder ein Gemisch von Polysacchariden.

15. Verfahren zum Herstellen einer Zusammensetzung nach Anspruch 8, umfassend das Beimischen des Polypeptids gemäß Ansprüchen 1 bis 6 zu einem Hilfsstoff.

16. Polynukleotid mit einer Nukleotidsequenz, die für das in einem beliebigen der Ansprüche 1 bis 6 definierte Polypeptid kodiert.

17. Zusammensetzung, umfassend das Polynukleotid gemäß Anspruch 16.

18. Nukleinsäurekonstrukt, umfassend die in Anspruch 16 definierte Nukleotidsequenz, funktionsfähig verknüpft mit einer oder mehreren Kontrollsequenzen, die die Herstellung des Polypeptids in einer Wirtszelle steuert/steuern.

19. Rekombinanter Expressionsvektor, umfassend das Nukleinsäurekonstrukt gemäß Anspruch 18.

20. Rekombinante Wirtszelle, umfassend das Nukleinsäurekonstrukt gemäß Anspruch 18.

21. Verfahren zum Herstellen eines Polypeptids gemäß Ansprüchen 1 bis 6, umfassend:
(a) Kultivieren einer wie in Anspruch 20 definierten rekombinanten Wirtszelle unter Bedingungen, die für die Herstellung des Polpeptids förderlich sind; und
(b) Gewinnen des Polypeptids.

22. Verfahren, umfassend das Einsetzen eines Polypeptids gemäß Ansprüchen 1 bis 6 oder eines Polynukleotids gemäß Anspruch 16 in einem technischen Industrie- oder Haushaltsverfahren.

## Revendications

1. Polypeptide isolé comprenant une séquence d'acides aminés présentant une identité d'au moins 90 % avec SEQ ID No : 27, dans lequel le polypeptide a une activité peptidase glutamique.

2. Polypeptide selon la revendication 1, dans lequel le polynucléotide codant pour le polypeptide est constitué par la région de SEQ ID No : 2 codant pour une peptidase glutamique mature ou pour une séquence différant de celle-ci en vertu de la dégénérescence du code génétique.

3. Polypeptide selon la revendication 1, **caractérisé par** l'absence de ponts disulfure dans la structure peptidase.

4. Polypeptide selon la revendication 1, pouvant être obtenu à partir de la souche d'*Alicyclobacillus sp.* déposée sous le No. d'accès DSM 15716.

5. Polypeptide selon la revendication 1, comprenant ou constitué par la peptidase glutamique mature représentée dans SEQ ID No : 27.

6. Enzyme polypeptidique selon la revendication 5, comprenant ou constituée par la séquence allant de la position 1 à 240 de SEQ ID No : 27.

7. Souche bactérienne déposée sous le numéro d'accès DSM 15716.

8. Composition comprenant le polypeptide selon les revendications 1 à 6.

9. Composition selon la revendication 8, comprenant en outre au moins deux polypeptides différents, de préférence au moins 3, plus préférablement au moins 5, plus préférablement au moins 10, plus préférablement au moins 15, plus préférablement au moins 20 polypeptides différents, tous les polypeptides étant de préférence sécrétés lors de la fermentation d'un échantillon d'*Alicyclobacillus sp.* DSM 15716 ou d'un mutant de celui-ci dans lequel un ou plusieurs gènes ont été délétés ou ajoutés.

10. Composition selon la revendication 8, comprenant en outre une ou plusieurs enzymes supplémentaires.

11. Composition selon la revendication 8, **caractérisée en ce qu'**il s'agit d'une composition détergente qui, en plus du polypeptide, comprend un tensioactif.

12. Composition selon la revendication 8, **caractérisée en ce qu'**il s'agit d'une composition de type aliment pour animaux qui, en plus du polypeptide, comprend une céréale ou un produit à base de grains.

13. Composition selon la revendication 8, **caractérisée en ce qu'**il s'agit d'une composition alimentaire.

14. Composition selon la revendication 8, comprenant en outre un polysaccharide ou un mélange de polysaccharides.

15. Méthode de préparation d'une composition selon la revendication 8, comprenant le mélange du polypeptide selon les revendications 1 à 6 avec un excipient.

16. Polynucléotide ayant une séquence de nucléotides qui code pour le polypeptide défini dans l'une quelconque des revendications 1 à 6.

17. Composition comprenant le polynucléotide selon la revendication 16.

18. Construit d'acide nucléique comprenant la séquence de nucléotides définie dans la revendication 16 fonctionnellement liée à une ou plusieurs séquences de contrôle qui dirigent la production du polypeptide dans une cellule hôte.

19. Vecteur d'expression recombinant comprenant le construit d'acide nucléique selon la revendication 18.

20. Cellule hôte recombinante comprenant le construit d'acide nucléique selon la revendication 18.

21. Méthode de production d'un polypeptide selon les revendications 1 à 6 comprenant :
(a) la culture d'une cellule hôte recombinante telle que définie dans la revendication 20 dans des conditions conduisant à la production du polypeptide ; et
(b) la récupération du polypeptide.

22. Procédé comprenant l'emploi d'un polypeptide selon les revendications 1 à 6 ou d'un polynucléotide selon la revendication 16 dans un procédé technique industriel ou domestique.
